# EUROPEAN PATENT APPLICATION

(11) **EP 4 285 887 A1**
(43) Date of publication of application: **06.12.2023**
(21) Application number: 22745729.8
(22) Date of filing: 21.01.2022
(51) Int. Cl.: A61J 3/00, G06T 7/00, G01N 21/85

(54) **DRUG IDENTIFICATION DEVICE, DRUG IDENTIFICATION METHOD AND PROGRAM, DRUG IDENTIFICATION SYSTEM, DRUG LOADING PLATFORM, ILLUMINATION DEVICE, IMAGING ASSISTANCE DEVICE, LEARNED MODEL, AND LEARNING DEVICE**

(30) Priority: 26.01.2021 JP 2021010289; 05.07.2021 JP 2021111559; 18.01.2022 JP 2022005745
(71) Applicant: FUJIFILM Toyama Chemical Co., Ltd., Chuo-ku Tokyo 104-0031 (JP)
(72) Inventor: HANEDA, Shinji, Tokyo 104-0031 (JP); TAKASHIMA, Masanobu, Tokyo 104-0031 (JP)
(74) Representative: Hughes, Andrea Michelle
(86) International application number: PCT/JP2022/002111
(87) International publication number: WO 2022/163508

(57) **Abstract**

To provide a drug identification device, a drug identification method and program, a drug identification system, a drug loading table, an illumination device, an imaging assistance device, a trained model, and a learning device, capable of achieving highly accurate, easy, and highly usable identification of a drug that is imparted with engraved mark and/or print. A region of a drug to be identified is detected from a captured image generated by imaging the drug to be identified that is imparted with engraved mark and/or print. The region of the drug to be identified in the captured image is processed to acquire an engraved mark and print extraction image that is an extracted image of the engraved mark and/or print of the drug to be identified. The engraved mark and print extraction image is input, and a drug type of the drug to be identified is inferred to acquire a candidate of the drug type of the drug to be identified.

## Description

### BACKGROUND OF THE INVENTION

### 1. Field of the Invention

The present invention relates to a drug identification device, a drug identification method and program, a drug identification system, a drug loading table, an illumination device, an imaging assistance device, a trained model, and a learning device. More particularly, the present invention relates to a technology of identifying drugs from captured images of the drugs.

### 2. Description of the Related Art

Drug identification devices that identify drugs from captured images of drugs are known (see Patent Literature 1 to Patent Literature 3). Non-Patent Literature 1 discloses a drug image recognition system using a smartphone.

### Citation List

Patent Literature 1: Japanese Patent Application Laid-Open No. 2015-535108
Patent Literature 2: Japanese Patent Application Laid-Open No. 2016-523405
Patent Literature 3: Japanese Patent Application Laid-Open No. 2015-523561

Non-Patent Literature 1: Mobile Deep Pill: A Small-Footprint Mobile Deep Learning System for Recognizing Unconstrained Pill Images, Xiao Zeng, K. Cao, Mi Zhang, Published 2017, "Computer Science, Proceedings of the 15th Annual International Conference on Mobile Systems, Applications, and Services"

### SUMMARY OF THE INVENTION

It is desired to implement a highly usable drug identification device that is operable under various light environments with a simplified imaging and illustration device or without the imaging and illustration device, using a mobile terminal having a camera, and that has improved usability for user to operate and use.

As of January 2021, the number of tablet-type and capsule-type pharmaceutical agents in Japan is almost 10,000. With some exceptions (such as plain tablets without an engraved mark or a print), individual drug are characterized by combinations of outer shape information (shapes such as round and oval, and sizes such as diameter, and major diameter/minor diameter), identification information based on engraved marks or prints imparted onto drugs, color information on drugs, and the likes. In other words, it is possible, in principle, to identify drug types by extracting such information from captured images of the drugs.

While color information on drugs is one source of information that characterizes the drugs, color reproducibility (color reproductivity) of identical drugs on individual captured images is unreliable, when application under various light environments is assumed. In terms of the outer shape information, the majority of drugs are in round or oval shapes, and almost all the drugs range in size from about 4 millimeters to about 3 centimeters, so that there are a large number of drugs in similar shapes. Furthermore, because users do not necessarily captures images of drugs directly from above, and because the drugs have thickness to have various shapes in a cross-sectional direction, the shape information on the drugs can be information of low reproducibility in the captured images. Therefore, it is difficult to say that the shape information is highly reliable.

On the other hand, the identification information imparted to the surface of the drugs includes abundant information (combinations of a plurality of letters and symbols, layouts, etc.) that characterizes the individual drugs. There are mainly two types of the identification information imparted to the surface of drugs: an engraved mark type; and a print type. It is relatively easy to extract information of a printed part from a captured image of a print-type drug because of a high contrast between a printed part and a non-printed part on the drug. On the other hand, in a case of an engraved mark-type drug, it may be difficult to secure a contrast between an engraved mark part and a non-engraved mark part on the drug from a captured image. This tendency is particularly noticeable when a dedicated illumination system is not used, or when the engraved mark is shallow, thin, small, etc.

In consideration of these circumstances, in order to achieve high-accuracy drug identification under various light environments, while using the engraved mark information or the print information as a main information source and using the color information or the size and shape information as a secondary information source, it is necessary to robustly extract identification information from the engraved mark-type drugs.

In the invention disclosed in Non-Patent Literature 1, in order to extract engraved mark information, the engraved mark information or print information on drugs are obtained from gradient information (edge information) in image processing. In this case, the engraved mark or print information co-exist with outer shape information on the drugs, in the gradient information. Although the outer shape information is also useful information in drug identification, in engraved mark-type drugs (in the case of shallow, small, or thin engraved mark in particular), the engraved mark information useful for identification has lowered reliability relative to the outer shape information, which leads to decrease in accuracy as described above.

The present invention has been made in view of such circumstances, and aims to provide a drug identification device, a drug identification method and program, a drug identification system, a drug loading table, an illumination device, an imaging assistance device, a trained model, and a learning device, capable of easily and highly accurately identify drugs on which engraved marks and/or prints are imparted, with high usability.

In order to accomplish the above object, one aspect of a drug identification device includes: an image acquisition unit configured to acquire a captured image generated by imaging at least one drug to be identified that is imparted with engraved mark and/or print; a drug detection unit configured to detect a region of the drug to be identified from the captured image; an engraved mark and print extraction unit configured to process at least the region of the drug to be identified in the captured image, to acquire an engraved mark and print extraction image that is an image of the engraved mark and/or print of the drug to be identified extracted from the region of the drug to be identified; and a first drug type recognition unit configured to receive input of the engraved mark and print extraction image, and infer a drug type of the drug to be identified to acquire at least one candidate of the drug type of the drug to be identified. According to the aspect, it is possible to identify a drug imparted with engraved mark and/or print, highly accurately and easily, with high usability.

It is preferable that the engraved mark and print extraction unit includes a first trained model configured to receive input of a first image of the drug that is imparted with the engraved mark and/or print and output a second image that is an image of the engraved mark and/or print of the drug extracted from the first image, and the first drug type recognition unit includes a second trained model configured to receive input of the second image and output the drug type of a drug corresponding to the engraved mark and/or print. By using the trained model, the engraved mark and print extraction image can be acquired highly accurately, and candidates of the drug type of the drug to be identified can be acquired in a short time.

It is preferable that the drug identification device includes a second drug type recognition unit configured to receive input of the at least the region of the drug to be identified in the captured image and infer the drug type of the drug to be identified, and the first drug type recognition unit integrates an inference result of the first drug type recognition unit with an inference result of the second drug type recognition unit to acquire the at least one candidate of the drug type of the drug to be identified, and the second drug type recognition unit includes a third trained model configured to receive input of the first image and output the drug type of the drug. Because the inference result of the first drug type recognition unit is integrated with the drug type of the drug to be identified that is inferred from the region of the drug to be identified, based on shape information, color information, or other information, the candidates of the drug type of the drug to be identified can be acquired more accurately.

It is preferable that the drug identification system includes a drug-annexed information acquisition unit configured to acquire drug-annexed information including at least one of shape, size, and color of a plurality of drugs, wherein the first drug type recognition unit integrates an inference result of the first drug type recognition unit with the drug-annexed information to acquire the at least one candidate of the drug type of the drug to be identified. Because the inference result of the first drug type recognition unit is integrated with the drug-annexed information, the candidate of the drug type of the drug to be identified can be acquired more accurately.

It is preferable that the image acquisition unit acquires the captured image generated by imaging the drug to be identified and at least one marker, and includes an image correction unit configured to standardize an imaging distance and an imaging viewpoint of the captured image based on the marker to acquire a standardized image, and the drug detection unit detects the region of the drug to be identified from the standardized image. By using the standardized image with a standardized imaging distance and imaging viewpoint, a region of the drug to be identified can be detected stably, regardless of the imaging environments. It also becomes possible to perform template matching with a master image that is acquired with the standardized imaging distance and imaging viewpoint.

It is preferable that the image acquisition unit acquires the captured image generated by imaging a plurality of ArUco markers, a plurality of circular markers, or a plurality of quadrangular markers. By using the ArUco markers, a standardized image can be acquired more appropriately and robustly. Using circular or quadrangular markers facilitates detection based on deep learning.

It is preferable that the circular markers include a concentric circle, and the quadrangular markers include a concentric quadrangle. This makes it easy to determine the coordinates of a center point of each marker, and makes it possible to acquire the standardized image that is robust against various types of noise.

It is preferable that the image acquisition unit acquires the captured image generated by imaging the drug to be identified and a reference gray color, and the image correction unit performs color tone correction on the captured image based on the reference gray color. By using the reference gray color, the color tone of the captured image can be corrected appropriately. In addition, it is possible to expect the effect to secure a contrast between an engraved mark part and a non-engraved mark part in the engraved mark-type drugs, in particular.

In order to accomplish the above object, one aspect of a drug identification system is a drug identification system including a mobile terminal and a server, which can communicate with each other. The mobile terminal includes an image acquisition unit configured to acquire a captured image generated by imaging at least one drug to be identified that is imparted with engraved mark and/or print, and the mobile terminal or the server includes a drug detection unit configured to detect a region of the drug to be identified from the captured image. The server includes: an engraved mark and print extraction unit configured to process at least the region of the drug to be identified in the captured image to acquire an engraved mark and print extraction image that is an image of the engraved mark and/or print of the drug to be identified extracted from the region of the drug to be identified; and a first drug type recognition unit configured to receive input of the engraved mark and print extraction image and infer a drug type of the drug to be identified, to acquire at least one candidate of the drug type of the drug to be identified. The mobile terminal further includes a display control unit configured to display the at least one candidate of the drug type of the drug to be identified. According to the aspect, it is possible to identify a drug that is imparted with engraved mark and/or print, highly accurately and easily, with high usability.

It is preferable that the engraved mark and print extraction unit includes a first trained model configured to receive input of a first image of a drug that is imparted with the engraved mark and/or print and output a second image that is an image of the engraved mark and/or print of the drug extracted from the first image, and the first drug type recognition unit includes a second trained model configured to receive input of the second image and output the drug type of a drug corresponding to the engraved mark and/or print. By using the trained model, the engraved mark and print extraction image can be acquired highly accurately, and candidates of the drug type of the drug to be identified can be acquired in a short time.

It is preferable that the server includes a second drug type recognition unit configured to receive input of at least the region of the drug to be identified in the captured image and infer the drug type of the drug to be identified, the first drug type recognition unit integrates an inference result of the first drug type recognition unit with an inference result of the second drug type recognition unit to acquire the at least one candidate of the drug type of the drug to be identified, and the second drug type recognition unit includes a third trained model configured to receive input of the first image and output the drug type of the drug. Because the inference result of the first drug type recognition unit is integrated with the drug type of the drug to be identified that is inferred from the region of the drug to be identified, the candidates of the drug type of the drug to be identified can be acquired more accurately.

It is preferable that the server includes a drug-annexed information acquisition unit configured to acquire drug-annexed information including at least one of shape, size, and color of a plurality of drugs, and the first drug type recognition unit integrates an inference result of the first drug type recognition unit with the drug-annexed information to acquire the at least one candidate of the drug type of the drug to be identified. Because integrating the inference result of the first drug type recognition unit with the drug-annexed information, the candidate of the drug type of the drug to be identified can be acquired more accurately.

It is preferable that the mobile terminal includes a camera, and a display, the image acquisition unit acquires the captured image generated by imaging the drug to be identified and at least one marker by the camera, the mobile terminal or the server includes an image correction unit configured to standardize an imaging distance and an imaging viewpoint of the captured image based on the marker to acquire a standardized image, and the drug detection unit detects the region of the drug to be identified from the standardized image. As a result, the server can recognize the candidate of the drug type of the drug to be identified that is imaged by the camera of the mobile terminal, and the mobile terminal can display the candidate on its display.

It is preferable that the drug to be identified is loaded on a loading surface having a gray color, and the mobile terminal includes an exposure correction unit configured to perform exposure correction of the camera based on the gray color. Because exposure correction is performed by using the reference gray color, the color of the drug can be acquired appropriately. In addition, it is possible to expect the effect to secure a contrast between an engraved mark part and a non-engraved mark part in the engraved mark-type drugs, in particular.

It is preferable that the image acquisition unit acquires the captured image that is imaged with a standard imaging distance and imaging viewpoint. By using the captured image that is imaged with the imaging distance and the imaging viewpoint, the shape information on the drug can be extracted with high reproducibility, and it is possible to expect improvement in identification accuracy.

It is preferable that the image acquisition unit acquires a captured image including a plurality of drugs to be identified, the drug detection unit detects respective regions of the plurality of drugs to be identified, the engraved mark and print extraction unit acquires a plurality of engraved mark and print extraction images respectively corresponding to the plurality of drugs to be identified, and the first drug type recognition unit acquires the candidates of the drug types respectively corresponding to the plurality of drugs to be identified. Even in the case of the captured image generated by concurrently imaging a plurality of drugs to be identified, it is possible to acquire the candidates of the drug type respectively corresponding to the plurality of drugs to be identified.

It is preferable that the first drug type recognition unit acquires a plurality of candidates of the drug type of the at least one drug to be identified, acquires master images of the respective candidates of the drug type, and performs template matching between the engraved mark and print extraction image and the master images. Using template matching, it is possible to narrow down the candidates of the drug type of the drug to be identified more accurately. Here, the template matching is desirably performed in a rotation direction and in a parallel movement direction.

It is preferable that the drug identification device or the drug identification system includes a display control unit configured to display, on a display, at least one of: an image of at least the region of the drug to be identified in the captured image; the engraved mark and print extraction image; and an image of the drug to be identified with the engraved mark and/or print being emphasized, wherein the display control unit further selectably displays, on the display, the at least one master image of the at least one candidate of the drug type of the at least one drug to be identified. Because the master images of the candidates of the drug type of the drug to be identified are selectably displayed on the display, a user can easily select the correct drug for the drug to be identified with high visibility.

It is preferable that the display control unit displays at least one of: the image of at least the region of the drug to be identified in the captured image; the engraved mark and print extraction image; and an image of the drug to be identified with the engraved mark and/or print being emphasized, and the at least one master image of the at least one candidate of the drug type of the at least one drug to be identified, in a state where directions of the engraved mark and/or print are arranged in an identical direction. Because the images are displayed in a state where the directions of the engraved marks and/or prints are arranged in the identical direction, a user can easily select the correct drug for the drug to be identified.

It is preferable that the display control unit displays on the display a search window into which a character string can be input, and the first drug type recognition unit specifies the drug type of the drug to be identified based on the character string input into the search window. Even when the drug candidates obtained by drug identification based on the captured image does not include a correct drug, it is possible to accurately identify the correct drug for the drug to be identified using the search window.

In order to accomplish the above object, one aspect of a drug loading table is a drug loading table for use in capturing a captured image in the drug identification device or the drug identification system. The drug loading table includes a loading surface on which the at least one drug to be identified is loaded, wherein the loading surface has a gray color, and a plurality of markers is arranged on the loading surface. According to the aspect, it is possible to appropriately acquire a standardized image, to appropriately correct the color tone of the captured image, and to secure the contrast between an engraved mark part and a non-engraved mark part in the engraved mark-type drugs.

It is preferable that the plurality of markers is, respectively, circular markers or quadrangular markers. This facilitates detection based on deep learning.

It is preferable that the circular markers include a concentric circle, and the quadrangular markers include a concentric quadrangle. This makes it easy to determine the coordinates of a center point of each marker, and makes it possible to acquire the standardized image that is robust against various types of noise.

It is preferable that the loading surface includes an indentation structure provided for loading the at least one drug to be identified. The indentation structure includes indentations, grooves, recesses, and holes. This makes it possible to place the drug to be identified in a stationary state. As a material of the drug loading table, paper, composite resin, fiber, rubber, or glass may be used. The drug loading table can be used for such purposes as identifying (discriminating) drugs brought in by a patient to be admitted to a hospital, drugs captured by a patient transported by an ambulance, and remaining drugs at home, or sorting drugs that are one-dose packaged but not used due to such reasons as change in prescriptions, or returned drugs in one-dose packaging.

In order to accomplish the above object, one aspect of an illumination device is an illumination device for use in imaging a captured image in the drug identification device or the drug identification system. The illumination device includes a plurality of light sources that emit illumination lights from directions different from each other, toward the drug to be identified. According to the aspect, it is possible to acquire a plurality of captured images by emitting illumination light from a plurality of directions so that a desired captured image can be acquired.

In order to accomplish the above object, one aspect of an imaging assistance device is an imaging assistance device for use in capturing a captured image in the drug identification device or the drug identification system. The imaging assistance device includes: a drug loading table having a loading surface on which the at least one drug to be identified is loaded; and an illumination device configured to irradiate the at least one drug to be identified that is loaded on the loading surface, with illumination light. The loading surface has a gray color, a plurality of markers is arranged on the loading surface, and the illumination device includes a plurality of light sources that emit illumination lights from directions different from each other, toward the drug to be identified. According to the aspect, it is possible to capture a desired captured image, to appropriately acquire a standardized image, to appropriately correct the color tone of the captured image, and to secure a contrast between an engraved mark part and a non-engraved mark part in the engraved mark-type drugs.

In order to accomplish the above object, one aspect of a drug identification method is a drug identification method including: an image acquisition step of acquiring a captured image generated by imaging at least one drug to be identified that is imparted with engraved mark and/or print; a drug detection step of detecting a region of the drug to be identified from the captured image; an engraved mark and print extraction step of processing at least the region of the drug to be identified in the captured image to acquire an engraved mark and print extraction image that is an image of the engraved mark and/or print of the drug to be identified extracted from the region of the drug to be identified; and a first drug type recognition step of receiving input of the engraved mark and print extraction image, and inferring a drug type of the drug to be identified to acquire at least one candidate of the drug type of the drug to be identified. According to the aspect, it is possible to identify a drug that is imparted with engraved mark and/or print, highly accurately and easily, with high usability.

It is preferable that in the engraved mark and print extraction step, the engraved mark and print extraction image is acquired by using a first trained model that receives input of one drug that is imparted with the engraved mark and/or print and outputs a second image that is an extracted image of the engraved mark and/or print of the drug, and in the first drug type recognition step, a drug type of the drug to be identified is inferred by using a second trained model that receives input of the second image and outputs a drug type of a drug corresponding to the engraved mark and/or print. By using the trained model, the engraved mark and print extraction image can be acquired highly accurately, and candidates of the drug type of the drug to be identified can be acquired in a short time.

In order to accomplish the above object, one aspect of a program is a program that makes a computer to execute the drug identification method. The aspect may also include a computer-readable, non-transitory storage medium that records the program. According to the aspect, it is possible to identify a drug that is imparted with engraved mark and/or print, highly accurately and easily, with high usability.

In order to accomplish the above object, one aspect of a trained model is a trained model that is machine-learned using a training data set including a second image that is an extracted image of engraved mark and/or print of a drug that is imparted with the engraved mark and/or print, and a drug type of a drug corresponding to the engraved mark and/or print, as a set. By outputting the drug type of the drug corresponding to the engraved mark and/or print from an extracted image of the engraved mark and/or print of the drug that is imparted with the engraved mark and/or print, the drug type of the drug can be recognized without being affected by the imaging environments.

It is preferable that noise is added to the second image. The added noise allows learning of the trained model that has acquired robustness against fluctuations in the imaging environments.

In order to accomplish the above object, one aspect of a learning device is a learning device including: a training data collection unit configured to collect a retraining data set including an engraved mark and print extraction image that is an extracted image of engraved mark and/or print of a drug to be identified, and information on a correct drug type of the drug to be identified, as a set; and a relearning unit configured to perform relearning of a second trained model by using the collected retraining data set, wherein the second trained model receives input of a second image that is an extracted image of the engraved mark and/or print of the drug and outputs a drug type of a drug corresponding to the engraved mark and/or print. By performing relearning of the second trained model, the drug type of the drug can be outputted with more accuracy.

In order to accomplish the above object, another aspect of the learning device is a learning device including: a training data collection unit configured to collect a retraining data set including: a captured image generated by imaging at least one drug to be identified that is imparted with engraved mark and/or print; and information on a drug type of the drug to be identified that is acquired by the drug identification device according to claim 3 or the drug identification system according to claim 11, as a set; and a relearning unit configured to perform relearning of the third trained model by using the collected retraining data set. By performing relearning of the third trained model, the drug type of the drug can be outputted with more accuracy.

In order to accomplish the above object, another aspect of the learning device is a learning device including: a training data collection unit configured to collect a retraining data set including: a captured image generated by imaging at least one drug to be identified that is imparted with engraved mark and/or print; and information on a drug type of the drug to be identified that is acquired by the drug identification device according to any one of claims 1 to 8, or 16 to 20, or the drug identification system according to any one of claims 9 to 20, as a set; and a learning unit configured to perform learning of a fourth trained model by using the collected retraining data set, wherein the fourth trained model receives input of a first image of a drug that is imparted with engraved mark and/or print and outputs a drug type of the drug. According to the aspect, it is possible to output the candidates of the drug type of the drug to be identified by using the fourth trained model that is newly learned instead of the third trained model.

In order to accomplish the above object, another aspect of the drug identification device is a drug identification device, including: an image acquisition unit configured to acquire a captured image generated by imaging at least one drug to be identified that is imparted with engraved mark and/or print; a drug detection unit configured to detect a region of the drug to be identified from the captured image; an engraved mark and print extraction unit configured to process at least the region of the drug to be identified in the captured image to acquire an engraved mark and print extraction image that is an image of the engraved mark and/or print of the drug to be identified extracted from the region of the drug to be identified; and a display control unit configured to display, on a display, at least one of: an image of at least the region of the drug to be identified in the captured image; the engraved mark and print extraction image; and an image of the drug to be identified with the engraved mark and/or print being emphasized, in a state where a direction of the engraved mark and/or print is made upright. According to the aspect, because the direction of the engraved mark and/or print is made upright, it is possible to display the images related to the drug to be identified in an easily viewable state for a user.

It is preferable that the engraved mark and print extraction unit includes a first trained model configured to receive input of a first image of the drug that is imparted with the engraved mark and/or print, and output a second image that is an image of the engraved mark and/or print of the drug extracted from the first image. By using the trained model, the engraved mark and print extraction image can be acquired highly accurately.

It is preferable that the display control unit acquires a master image of the drug to be identified in which the direction of the engraved mark and/or print is made upright, and collates a rotation direction by template matching between at least one of: the image of at least the region of the drug to be identified in the captured image; the engraved mark and print extraction image; and an image of the drug to be identified with the engraved mark and/or print being emphasized, and the master image. By using collation in the rotation direction by template matching, the direction of the engraved mark and/or print can be made upright appropriately.

It is preferable that the display control unit include a fifth trained model configured to receive input of a first image of a drug that is imparted with the engraved mark and/or print, and output a third image in which a direction of the engraved mark and/or print is made upright. By using the trained model, the direction of the engraved mark and/or print can be made upright appropriately.

It is preferable that the display control unit displays on the display a search window into which character information can be input, and the drug identification device comprises: a search unit configured to retrieve at least one candidate of a drug type of the drug to be identified based on the character information input into the search window; and a first drug type recognition unit configured to receive input of the engraved mark and print extraction image, and infer the drug type of the drug to be identified to acquire the at least one candidate of the drug type of the drug to be identified, and the display control unit automatically inputs into the search window the character information indicating a most promising candidate of the drug type of the drug to be identified that is acquired in the first drug type recognition unit. Because character information indicating the most promising candidate of the drug type of the drug to be identified is automatically input in the search window, the user can quickly search the drug to be identified.

It is preferable that the display control unit displays the candidate of the drug type of the drug to be identified that is searched by the search unit. This allows the user to visibly recognize the search result.

In order to accomplish the above object, another aspect of the drug identification system is a drug identification system including a mobile terminal and a server which can communicate with each other, wherein the mobile terminal includes an image acquisition unit configured to acquire a captured image generated by imaging at least one drug to be identified that is imparted with engraved mark and/or print, the mobile terminal or the server includes a drug detection unit configured to detect a region of the drug to be identified from the captured image, the server includes an engraved mark and print extraction unit configured to processes at least the region of the drug to be identified in the captured image to acquire an engraved mark and print extraction image that is an image of the engraved mark and/or print of the drug to be identified extracted from the region of the drug to be identified, and the mobile terminal further includes a display control unit configured to display, on a display, at least one of: an image of at least the region of the drug to be identified in the captured image; the engraved mark and print extraction image; and an image of the drug to be identified with the engraved mark and/or print being emphasized, in a state where a direction of the engraved mark and/or print is made upright. According to the aspect, Because the direction of the engraved mark and/or print is made upright, it is possible to display the images related to the drug to be identified in an easily viewable state for a user.

In order to accomplish the above object, another aspect of the drug identification method is a drug identification method, including: an image acquisition step of acquiring a captured image generated by imaging at least one drug to be identified that is imparted with engraved mark and/or print; a drug detection step of detecting a region of the drug to be identified from the captured image; an engraved mark and print extraction step of processing at least the region of the drug to be identified in the captured image to acquire an engraved mark and print extraction image that is an image of the engraved mark and/or print of the drug to be identified extracted from the region of the drug to be identified; and a display control step of displaying, on a display, at least one of: an image of at least the region of the drug to be identified in the captured image; the engraved mark and print extraction image; and an image of the drug to be identified with the engraved mark and/or print being emphasized, in a state where a direction of the engraved mark and/or print is made upright. According to the aspect, Because the direction of the engraved mark and/or print is made upright, it is possible to display the images related to the drug to be identified in an easily viewable state for a user.

In order to accomplish the above object, one aspect of an output object is an output object for use in capturing a captured image in the drug identification device or the above drug identification system. The output object includes: a personal information display region on which information that identifies an individual is displayed; and a loading region on which at least one drug to be identified of the individual is loaded. According to the aspect, it is possible to specify an individual and to identify the drug of the individual.

It is preferable that information that identifies the individual includes a barcode. By reading the barcode with a reader, the individual can be specified automatically.

It is preferable that the loading region has a gray color, and a plurality of markers is arranged in the loading region. Because an image of the drug loaded in the loading region is captured, it is possible to appropriately acquire a standardized image, to appropriately correct the color tone of the captured image, and to secure the contrast between an engraved mark part and a non-engraved mark part in the engraved mark-type drugs.

In order to accomplish the above object, one aspect of a production method of the output object is a production method of an output object, including: a step of acquiring the information that identifies the individual; and a step of arranging and printing the personal information display region and the loading region on a printing medium. According to the aspect, it is possible to specify an individual and to print the output object that can identify the drug of the individual on a printing medium.

According to the present invention, it is possible to identify a drug that is imparted with engraved mark and/or print, highly accurately and easily, with highly usability.

### BRIEF DESCRIPTION OF THE DRAWINGS

Figure 1 is a front perspective view of a smartphone.
Figure 2 is a rear perspective view of the smartphone.
Figure 3 is a block diagram showing an electrical configuration of the smartphone.
Figure 4 is a block diagram showing an internal configuration of an in-camera.
Figure 5 is a block diagram showing a functional configuration of a drug identification device.
Figure 6 is a flowchart showing steps of a drug identification method using the drug identification device.
Figure 7 is a view showing a process in the steps of the drug identification method.
Figure 8 is a block diagram showing a functional configuration of a drug identification device.
Figure 9 is a flowchart showing steps of a drug identification method using the drug identification device.
Figure 10 shows a process in the steps of the drug identification method.
Figure 11 is a block diagram showing a functional configuration of a drug identification device.
Figure 12 shows an example of master images of a drug stored in a master image storage unit.
Figure 13 is a flowchart showing steps of a drug identification method using the drug identification device.
Figure 14 shows a screen display on a touch panel display.
Figure 15 shows a screen display on the touch panel display.
Figure 16 shows a screen display on the touch panel display.
Figure 17 shows a screen display on the touch panel display.
Figure 18 shows a screen display on the touch panel display.
Figure 19 shows a screen display on the display of a tablet-type computer terminal.
Figure 20 shows a screen display on the display of the tablet-type computer terminal.
Figure 21 shows a screen display on the display of the tablet-type computer terminal.
Figure 22 shows a screen display on the display of the tablet-type computer terminal.
Figure 23 is a top view of an imaging assistance device.
Figure 24 is a cross-sectional view along a 24-24 line in Figure 23.
Figure 25 shows an example of data sets of ArUco markers each made of four dots in longitudinal and lateral directions.
Figure 26 is a top view of the imaging assistance device in a state where an auxiliary light source is removed.
Figure 27 is a top view of an imaging assistance device.
Figure 28 is a cross-sectional view along a 28-28 line in Figure 27.
Figure 29 is a top view of the imaging assistance device with the auxiliary light sources being removed.
Figure 30 shows the configuration of a drug identification system.
Figure 31 is a block diagram showing a functional configuration of the drug identification system.
Figure 32 is a flowchart showing steps of a drug identification method using the drug identification system.
Figure 33 is a block diagram showing an electrical configuration of an image processing device.
Figure 34 shows an example of training data sets for generating a first trained model.
Figure 35 is a block diagram showing a functional configuration of an image learning device.
Figure 36 shows an example of training data sets for generating a second trained model.
Figure 37 is a block diagram showing a functional configuration of the image learning device.
Figure 38 shows an example of training data sets for generating a third trained model.
Figure 39 is a block diagram showing a functional configuration of a drug identification device.
Figure 40 is a flowchart showing steps of a drug identification method.
Figure 41 is a view showing a screen display on the touch panel display of the smartphone.
Figure 42 shows a screen display on the touch panel display of the smartphone.
Figure 43 shows a screen display on the touch panel display of the smartphone.
Figure 44 shows a screen display on the touch panel display of the smartphone.
Figure 45 shows a screen display on the touch panel display of the smartphone.
Figure 46 is a block diagram showing a configuration of a drug identification system.
Figure 47 is a flowchart showing steps of a drug identification method.
Figure 48 is a view showing an example of a brought-in drug discrimination request sheet.
Figure 49 is a view showing a case where one dose of the brought-in drugs of a patient is loaded onto a loading region of the brought-in drug discrimination request sheet and imaged.
Figure 50 shows views of bounding boxes for drugs in a captured image and their ground truth data.
Figure 51 illustrates detection of reference markers when the reference markers are quadrangular.
Figure 52 illustrates detection of reference markers when the reference markers are circular.
Figure 53 shows specific examples of the circular reference markers.
Figure 54 shows top views of a drug loading table using the circular markers as reference markers.
Figure 55 shows top views of the drug loading table using circular markers according to a modification.
Figure 56 shows specific examples of quadrangular reference markers.
Figure 57 is a block diagram showing the functional configuration of the drug identification device implemented by the smartphone.
Figure 58 shows an example of images included in a first training data set.
Figure 59 shows an example of an image included in a second training data set.
Figure 60 is a flowchart showing an example of a learning method of a sixth trained model.
Figure 61 is a flowchart showing an example of an inference method using the sixth trained model.
Figure 62 shows a drug loading table having an indentation structure.
Figure 63 shows a drug loading table having an indentation structure for capsules.
Figure 64 shows a drug loading table having an indentation structure for oval tablets.

### DESCRIPTION OF THE PREFERRED EMBODIMENTS

Hereinafter, preferred embodiments of the present invention are described in detail with reference to accompanying drawings.

### <First Embodiment>

A drug identification device according to the present embodiment is a device that identifies the drug type of a drug to be identified on which an engraved mark and/or a print are imparted, to specify a correct drug. Note that the "engraved mark and/or print" refers to "one of the engraved mark and the print" or "both the engraved mark and the print".

Here, "engraved mark imparted on a drug" means that identification information is formed by making grooves that are recessed regions on the surface of the drug. The grooves are not limited to those made by digging the surface, and may be those formed by pressing the surface. The engraved mark may also include those without an identification function, such as sectioning lines.

In addition, "print imparted on a drug" means that identification information is formed by applying edible ink, or the like, to the surface of the drug in a contact or noncontact manner. Here, "print imparted on a drug" is synonymous with "imparted by printing".

For an example, the drug identification device is mounted on a mobile terminal device. The mobile terminal device includes at least one of a portable telephone, a personal handy phone system (PHS), a smartphone, a personal digital assistant (PDA), a tablet-type computer terminal, a notebook personal computer terminal, and a portable game machine. Hereinafter, detailed description is given with reference to the drawings by taking a drug identification device formed using a smartphone, for an example.

### [Appearance of Smartphone]

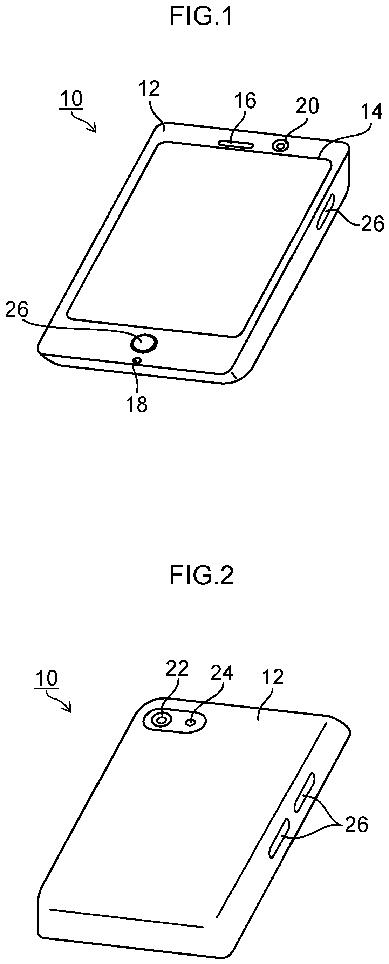

Figure 1 is a perspective front view of a smartphone 10 that is a portable terminal device with a camera, according to the present embodiment. As shown in Figure 1, the smartphone 10 has a plate-shaped casing 12. The smartphone 10 includes a touch panel display 14, a speaker 16, a microphone 18, and an in-camera 20 on the front surface of the casing 12.

The touch panel display 14 includes: a display unit that displays images, or the like; and a touch panel unit that is located on the front surface of the display unit and accepts touch input. The display unit is, for example, a color liquid crystal display (LCD) panel.

The touch panel unit is, for example, a capacitance touch panel that is provided on an optically transparent substrate body and has a planar shape. The capacitance touch panel includes an optically transparent position detection electrode and an insulating layer provided on the position detection electrode. The touch panel unit generates and outputs two-dimensional position coordinate information corresponding to the touch operation of a user. The touch operation includes tapping, double-tapping, flicking, swiping, dragging, pinch-in, and pinch-out operations.

The speaker 16 is a voice output unit that outputs voice when talking on the telephone and reproducing moving images. The microphone 18 is a voice input unit that receives input of voice, when talking on the telephone and capturing moving images. The in-camera 20 is an imaging device that captures moving images and still images.

Figure 2 is a perspective rear view of the smartphone 10. As shown in Figure 2, the smartphone 10 includes an out-camera 22 and a lighting 24 on the rear surface of the casing 12. The out-camera 22 is an imaging device that captures moving images and still images. The lighting 24, which is a light source that emits illumination light at the time of capturing images with the out-camera 22. The light 24 includes a light emitting diode (LED), for example.

As shown in Figures 1 and 2, the smartphone 10 further includes switches 26 each on the front surface and a side surface of the casing 12. The switches 26 are input members that receive instructions from the user. The switches 26 are push-button switches that are turned on when pressed by a finger or the like and are turned off due to resilient force of a spring, or the like, when the finger is released.

The configuration of the casing 12 is not limited to this configuration, and a configuration with folding structure or a sliding mechanism may be adopted.

### [Electrical Configuration of Smartphone]

The smartphone 10 includes, as a main function, a wireless communication function for performing mobile wireless communication through a base station device and a mobile communication network.

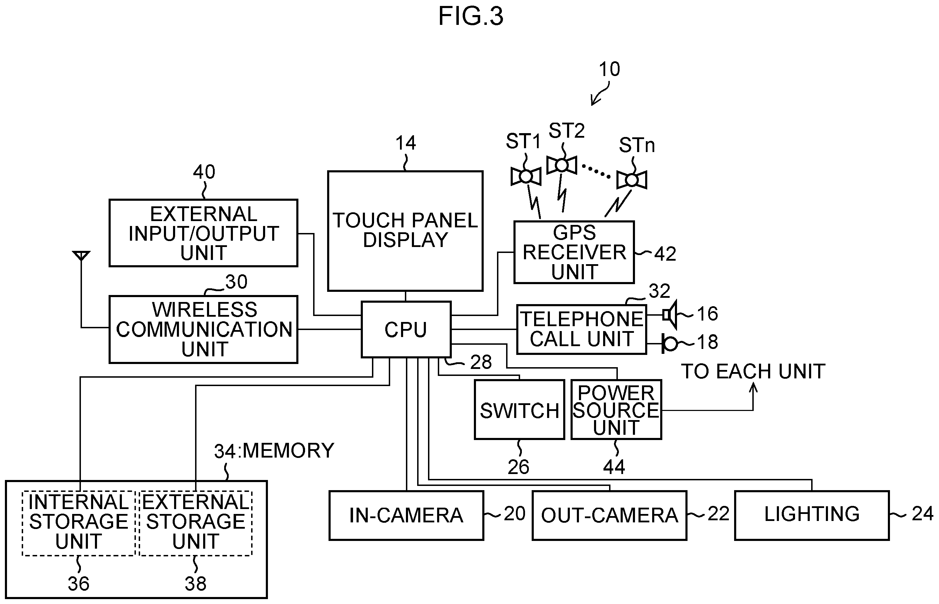

Figure 3 is a block diagram showing an electrical configuration of the smartphone 10. As shown in Figure 3, the smartphone 10 includes the touch panel display 14, the speaker 16, the microphone 18, the in-camera 20, the out-camera 22, the lighting 24, and the switches 26 as described above. The smartphone 10 also includes a central processing unit (CPU) 28, a wireless communication unit 30, a telecommunication unit 32, a memory 34, an external input/output unit 40, a GPS receiver unit 42, and a power source unit 44.

The CPU 28 is an example of a processor that executes commands stored in the memory 34. The CPU 28 operates according to a control program and control data stored in the memory 34. The CPU 28 comprehensively controls each part of the smartphone 10. The CPU 28 includes a mobile communication control function for controlling each unit of the communication system and an application processing function, in order to perform voice communication and data communication through the wireless communication unit 30.

The CPU 28 also includes an image processing function for displaying moving images, still images, and text, or the like, on the touch panel display 14. The image processing function visually transmits information, such as still images, moving images, and text, to the user. The CPU 28 also acquires two-dimensional position coordinate information corresponding to the touch operation of the user, from the touch panel unit of the touch panel display 14. The CPU 28 further acquires input signals from the switches 26.

The hardware structure of the CPU 28 is various processors as shown below. The various processors include: a central processing unit (CPU) that is a general-purpose processor that functions as various function units by executing software (programs); a graphic processing unit (GPU) that is a processor dedicated to image processing, a programmable logic device (PLD) capable of changing circuit configuration after manufacturing, such as field programmable gate arrays (FPGAs); and an exclusive electrical circuit that is a processor having circuit configuration exclusively designed for execution of specific processing, such as application specific integrated circuits (ASICs).

One processing unit may be formed by one of the various processors, or may be formed by two or more processors of the same kind or different kinds (for example, a combination of FPGAs, or a combination of a CPU and an FPGA, or a combination of a CPU and a GPU). One processor may form a plurality of function units. As an example of one processor forming the function units, firstly, there is a configuration, as represented by a computer such as a client or a serve, where a combination of one or more CPUs and software constitutes one processor, and the one processor functions as the processing units. Secondary, there is a configuration of using a processor that implements the function of the entire system, including the function units, by a single integrated circuit (IC) chip, as represented by a system on chip (SoC) or the like. In this way, various function units are formed by using one or more of the various processors in terms of the hardware structure.

Furthermore, the hardware structures of the various kinds of processors correspond to electrical circuits (circuitries) formed by combining circuit elements such as semiconductor elements to be more specific.

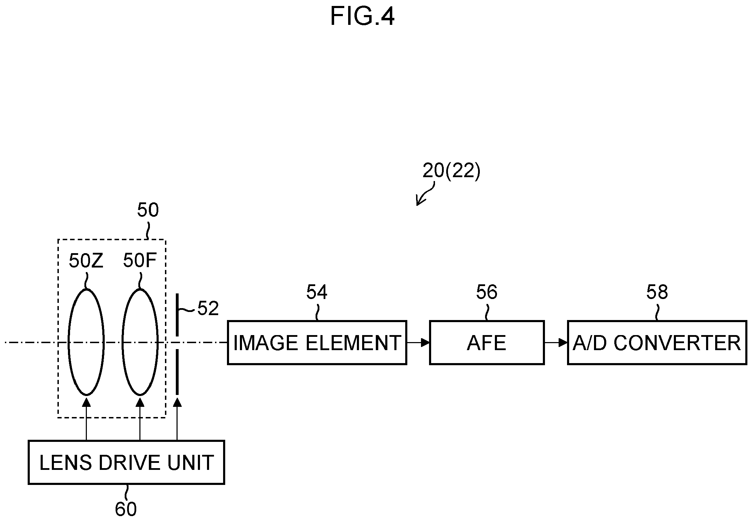

The in-camera 20 and the out-camera 22 capture moving images and still images according to an instruction of the CPU 28. Figure 4 is a block diagram showing an internal configuration of the in-camera 20. Here, the internal configuration of the out-camera 22 is the same as the in-camera 20. As shown in Figure 4, the in-camera 20 includes a photographic lens 50, a diaphragm 52, an image element 54, an analog front end (AFE) 56, an analog to digital (A/D) converter 58, and a lens drive unit 60.

The photographic lens 50 includes a zoom lens 50Z and a focus lens 50F. The lens drive unit 60 drives the zoom lens 50Z and the focus lens 50F in a forward and backward directions to perform optical zoom adjustment and focus adjustment according to the commands from the CPU 28. The lens drive unit 60 also controls the diaphragm 52 and adjusts exposure according to the command from the CPU 28. The lens drive unit 60 corresponds to an exposure correction unit that performs exposure correction of the camera based on a gray color described later. Information, such as the position of the zoom lens 50Z and the focus lens 50F, and an opening degree of the diaphragm 52, is input into the CPU 28.

The image element 54 includes a light-receiving surface on which a large number of light-receiving elements are arranged in a matrix. Subject light which has passed through the zoom lens 50Z, the focus lens 50F, and the diaphragm 52 forms an image on a light-receiving surface of the image element 54. On the light-receiving surface of the image element 54, color filters of red (R), green (G), and blue (B) are provided. The light-receiving elements of the image element 54 each convert the subject light that forms an image on the light-receiving surface into electrical signals based on signals of respective colors of R, G, and B. Thus, the image element 54 acquires a color image of a subject. As the image element 54, photoelectric conversion elements such as complementary metal-oxide semiconductors (CMOSs) or charge coupled devices (CCDs) may be used.

The AFE 56 removes noise from an analog image signal that is output from the image element 54 and performs amplification or other operations. The A/D converter 58 converts the analog image signal from the AFE 56 into a digital image signal with a gradation width. An electronic shutter is used as a shutter that controls the exposure time of incident light to the image element 54. In the case of the electronic shutter, the exposure time (the shutter speed) can be adjusted by the CPU 28 controlling a charge accumulation period of the image element 54.

The in-camera 20 may convert the image data of the captured moving image and the still image into compressed image data, such as moving picture experts group (MPEG) data or joint photographic experts group (JPEG) data.

Back to the description in Figure 3, the CPU 28 stores the moving image and the still image captured by the in-camera 20 and the out-camera 22 in the memory 34. The CPU 28 may also output the moving image and the still image captured by the in-camera 20 and the out-camera 22 to the outside of the smartphone 10 through the wireless communication unit 30 or the external input/output unit 40.

The CPU 28 further displays the moving image and the still image captured by the in-camera 20 and the out-camera 22 on the touch panel display 14. The CPU 28 may use the moving image and the still image captured by the in-camera 20 and the out-camera 22 within the application software.

Note that at the time of imaging with the out-camera 22, the CPU 28 may irradiate a subject with imaging auxiliary light by turning on the lighting 24. Turning on and off the lighting 24 may be controlled by the touch operation on the touch panel display 14 or by the operation of the switches 26 by the user.

The wireless communication unit 30 performs wireless communication with a base station device that is incorporated in a mobile communication network, according to an instruction of the CPU 28. The smartphone 10 uses the wireless communication to transmit and receive various file data such as voice data and image data, and e-mail data, and to receive Web (which stands for World Wide Web) data, streaming data, and the like.

The telecommunication unit 32 is connected to the speaker 16 and the microphone 18. The telecommunication unit 32 decodes voice data received by the wireless communication unit 30, and outputs the data through the speaker 16. The telecommunication unit 32 converts the voice of the user that is input through the microphone 18 into voice data that can be processed by the CPU 28, and outputs the data to the CPU 28.

The memory 34 stores commands to be executed by the CPU 28. The memory 34 includes an internal storage unit 36 incorporated into the smartphone 10 and an external storage unit 38 that can be attached to and detached from the smartphone 10. The internal storage unit 36 and the external storage unit 38 are implemented by using well-known storage media.

The memory 34 stores data such as a control program, control data, and application software for the CPU 28, address data that is associated with names, telephone numbers, or the like, of communication partners, transmitted and received e-mail data, Web data downloaded through Web browsing, and downloaded content data. The memory 34 may also temporarily store streaming data, or the like.

The external input/output unit 40 serves as an interface with external devices that are coupled to the smartphone 10. The smartphone 10 is directly or indirectly connected to other external devices through communication or the like, via the external input/output unit 40. The external input/output unit 40 transmits the data received from the external devices to each internal component member inside the smartphone 10 and transmits data inside the smartphone 10 to the external devices.

Examples of means for communication, or the like, include universal serial bus (USB), institute of electrical and electronics engineers (IEEE) 1394, the Internet, local area network (LAN), Bluetooth (registered trademark), radio frequency identification (RFID), and infrared communication. Examples of the external devices include headsets, external chargers, data ports, audio equipment, video equipment, smartphones, PDAs, personal computers, and earphones.

The GPS receiver unit 42 detects the position of the smartphone 10 based on positioning information from GPS satellites ST1, ST2, ..., STn.

The power source unit 44 is an electric power supply source that supplies electric power to each unit of the smartphone 10 via a power source circuit that is not shown. The power source unit 44 includes a lithium-ion secondary battery. The power source unit 44 may include an A/D converter that generates DC voltage from an external AC power source.

The smartphone 10 configured in this way can be set to an imaging mode in response to an instruction input from the user using the touch panel display 14 or the like, so that a moving image and a still image can be captured with the in-camera 20 and the out-camera 22.

When the smartphone 10 is set to the imaging mode, the smartphone 10 switches into an imaging standby state, a moving image is captured by the in-camera 20 or the out-camera 22. The captured moving image is displayed as a live view image (captured moving image is displayed in real time) on the touch panel display 14.

The user can visually recognize the live view image displayed on the touch panel display 14 to determine its composition, confirm a subject that the user desires to image, or set imaging conditions.

When imaging is instructed by an instruction input from the user using the touch panel display 14 or the like in the imaging standby state, the smartphone 10 performs auto focus (AF) control and auto exposure (AE) control to capture and store a moving image and a still image.

### [Functional Configuration of Drug Identification Device]

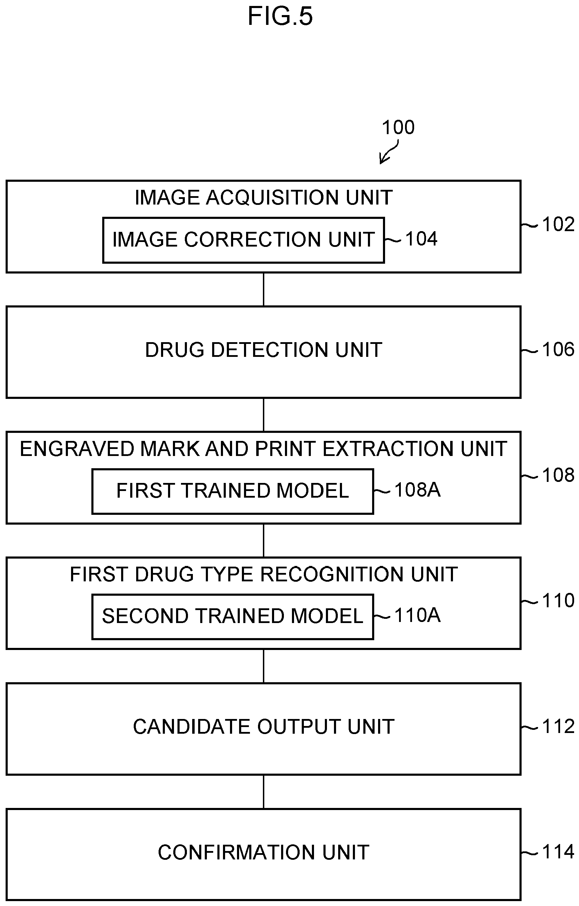

Figure 5 is a block diagram showing the functional configuration of a drug identification device 100 implemented by the smartphone. The functions of the drug identification device 100 are each implemented when the CPU 28 executes programs stored in the memory 34. As shown in Figure 5, the drug identification device 100 includes an image acquisition unit 102, a drug detection unit 106, and an engraved mark and print extraction unit 108, a first drug type recognition unit 110, a candidate output unit 112, and a confirmation unit 114.

The image acquisition unit 102 acquires a captured image generated by imaging a drug to be identified that is imparted with engraved mark and/or print. The captured image is, for example, an image captured by the in-camera 20 or the out-camera 22. The captured image may be an image acquired from another device via the wireless communication unit 30, the external storage unit 38, or the external input/output unit 40.

The captured image may be an image generated by imaging a drug to be identified and a marker. There may be one or more markers, and the marker may be an ArUco marker, a circular marker, or a quadrangular marker. The captured image may be an image generated by imaging a drug to be identified and a reference gray color.

The captured image may be an image captured with a standard imaging distance and imaging viewpoint. The imaging distance can be expressed by a distance between the drug to be identified and the photographic lens 50 and by a focal length of the photographic lens 50. In addition, the imaging viewpoint can be expressed by an angle formed between a marker print surface and an optical axis of the photographic lens 50.

The captured image may include more than one drug to be identified. Without being limited to the drugs to be identified of an identical drug type, the drugs to be identified may be of different drug types from each other.

The image acquisition unit 102 includes an image correction unit 104. When the captured image includes a marker (markers), the image correction unit 104 standardizes the imaging distance and imaging viewpoint of the captured image based on the marker, and acquires a standardized image. When the captured image includes a region having a gray color as a reference (reference gray-colored region), the image correction unit 104 performs color tone correction of the captured image based on the reference gray color.

The drug detection unit 106 detects a region of the drug to be identified from the captured image that is acquired by the image acquisition unit 102. In a case where the image correction unit 104 acquires the standardized image, the drug detection unit 106 detects the region of the drug to be identified from the standardized image. In a case where the captured image includes more than one drugs to be identified, the drug detection unit 106 detects respective regions of the drugs to be identified.

The engraved mark and print extraction unit 108 processes at least the region of the drug to be identified in the captured image to remove outer edge information on the drug to be identified, and acquires an engraved mark and print extraction image that is an extracted image of the engraved mark and/or print. Here, the engraved mark and print extraction image is an image in which engraved mark and/or print is emphasized by expressing an engraved mark portion or a print portion with relatively higher in luminance than portions other than the engraved mark portion or the print portion.

In a case where the drug detection unit 106 detects the respective regions of the drugs to be identified, the engraved mark and print extraction unit 108 acquires the engraved mark and print extraction images corresponding to the respective drugs to be identified.

The engraved mark and print extraction unit 108 includes a first trained model 108A. The first trained model 108A is a trained model that outputs, upon receiving input of a first image of the drug that is imparted with the engraved mark and/or print, a second image that is an extracted image of the engraved mark and/or print of the drug. The first trained model 108A is machine-learned based on training data sets of different drugs that are imparted with engraved mark and/or print. The training data sets include images of the drugs imparted with engraved mark and/or print, and extracted images of the engraved mark and/or print of the drugs, as sets for learning. To the first trained model 108A, a convolutional neural network (CNN) can be applied.

Here, since the image correction unit 104 standardizes the imaging distance and the imaging viewpoint, and color tone correction of the captured image, it is possible to expect stable operation of the first trained model 108A.

The first drug type recognition unit 110 receives input of the engraved mark and print extraction image and infers a drug type of the drug to be identified to acquire a candidate (candidates) of the drug type of the drug to be identified. The candidate of the drug type includes drug identification information including a drug name, a commodity name, an abbreviated name, or a combination of these. In a case where the engraved mark and print extraction images respectively corresponding to a plurality of drugs to be identified are input, the first drug type recognition unit 110 acquires candidates of the drug types corresponding to the respective drugs to be identified.

The first drug type recognition unit 110 includes a second trained model 110A. The second trained model 110A is a trained model that outputs, upon receiving input of a second image that is an extracted image of the engraved mark and/or print of the drug, the drug type of a drug corresponding to the engraved mark and/or print of the drug. The second trained model 110A is machine-learned based on training data sets of different drugs that are imparted with engraved mark and/or print. The training data sets include extracted images of the engraved mark and/or print, and the drug types of the drugs corresponding to the engraved mark and/or print of the drugs, as sets for learning. To the second trained model 110A, similarly to the first trained model 108A, the CNN may be applied.

Thus, since the first drug type recognition unit 110 performs recognition based on the engraved mark and/or print information without using color information, the first drug type recognition unit 110 is robust to the influence of the imaging environments.

The candidate output unit 112 outputs a candidate (candidates) of the drug type of the drug to be identified that is acquired by the first drug type recognition unit 110. The candidate output unit 112 (one example of the display control unit) selectably displays, for example, more than one candidate of the drug type of the drug to be identified on the touch panel display 14.

The confirmation unit 114 confirms the correct drug for the drug to be identified, out of the candidates of the drug type of the drug to be identified. The confirmation unit 114 confirms, as the correct drug, the candidate of the drug selected by the user, out of the candidates of the drug type of the drug to be identified that are displayed on the touch panel display 14, for example. In a case where the candidate output unit 112 outputs only one candidate of the drug type of the drug to be identified, the confirmation unit 114 may confirm the candidate of the drug type, as the correct drug.

### [Drug Identification Method]

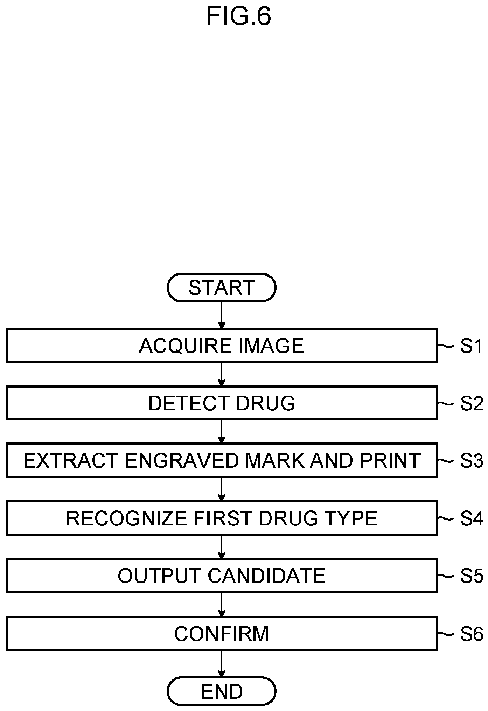

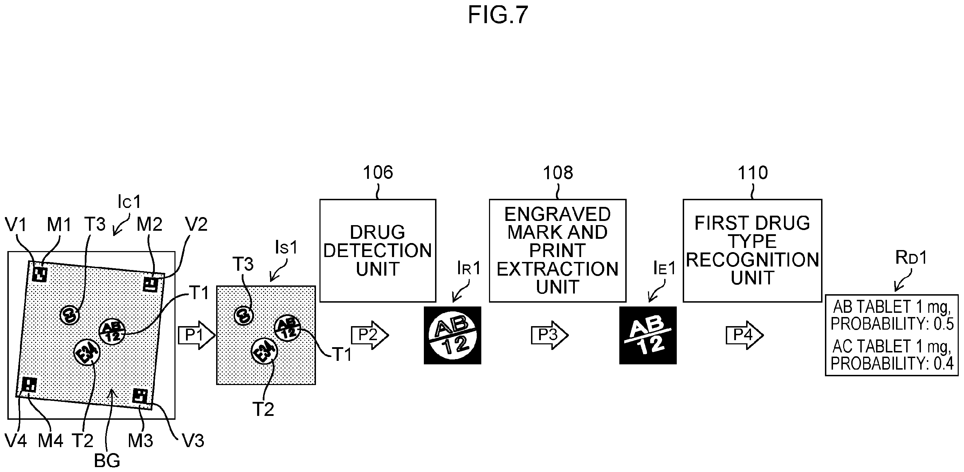

Figure 6 is a flowchart showing the steps of a drug identification method using the drug identification device. Figure 7 shows the process in the steps of the drug identification method. The drug identification method is implemented when the CPU 28 reads a drug identification program from the memory 34 and executes the program. The drug identification program may be provided via the wireless communication unit 30 or the external input/output unit 40.

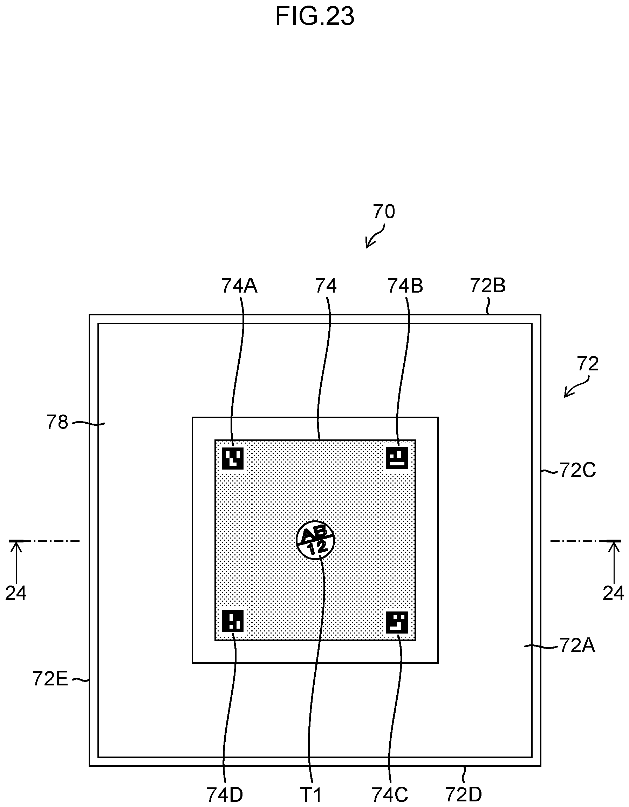

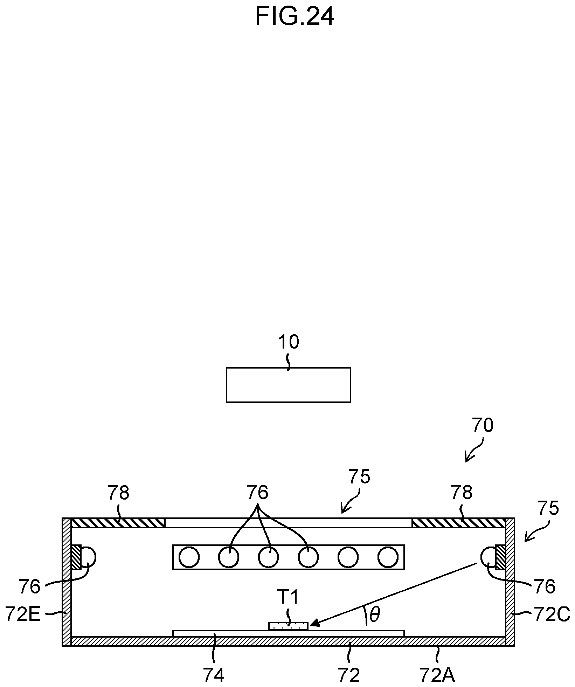

In step S1 that is an image acquisition step, the image acquisition unit 102 acquires the captured image generated by imaging the drug to be identified that is imparted with engraved mark and/or print. Here, the image acquisition unit 102 acquires the captured image I_{C}1 captured by the out-camera 22. The captured image I_{C}1 may be an image of the drug to be identified that is irradiated with illumination light by the lighting 24, or may be a total illumination image or a partial illumination image captured by using an imaging assistance device 70 (see Figures 23 and 24) or the like, as described later.

Figure 7 shows an example of the captured image I_{C}1 in which tablets T1, T2, and T3, which are drugs to be identified, are photographed. Tablets are solid drugs molded into fixed shapes by compression molding. The tablets T1, T2, and T3 are each imparted with identification information by engraved mark and/or print.

The captured image I_{C}1 shows a background BG, which is a region having the reference gray color. The captured image I_{C}1 further shows four markers M1, M2, M3, and M4. The markers M1 to M4 are each ArUco markers. The three tablets T1, T2, and T3 are shown at positions surrounded with the markers M1 to M4.

Thus, the captured image I_{C}1 is an image generated by concurrently imaging the drug to be identified, the markers M1 to M4 and the region having the reference gray color. The image correction unit 104 performs standardization processing of the imaging distance and the imaging viewpoint of the captured image I_{C}1 based on the markers M1 to M4, and performs color tone correction on the captured image I_{C}1 based on the gray color of the background BG so as to acquire a standardized image I_{S}1 (process P1).

Here, the standardization processing of the imaging distance and the imaging viewpoint is performed as follows.

First, the image correction unit 104 acquires in-image coordinates of four vertexes V1, V2, V3, and V4 of the respective four markers M1, M2, M3, and M4 in the captured image. The image correction unit 104 then specifies the coordinates of the four vertexes that are used for position adjustment. Here, the image correction unit 104 specifies the coordinates of an upper-left vertex V1 of the marker M1 arranged on the upper left side, an upper-right vertex V2 of the marker M2 arranged on the upper right side, a lower-right vertex V3 of the marker M3 arranged on the lower right side, and a lower-left vertex V4 of the marker M4 arranged on the lower left side.

After specifying the coordinates of the four vertexes V1 to V4, the image correction unit 104 further designates coordinates of the four vertexes V1 to V4 after standardization of the imaging distance and the imaging viewpoint. The image correction unit 104 obtains a perspective transformation matrix that converts the four vertexes V1 to V4 with their coordinates transformed into the designated coordinate positions. Such a perspective transformation matrix is uniquely defined, in a case where there are four points. For example, in a case where there is a correspondence relation between four points, the transformation matrix can be obtained by a getPerspectiveTransform function of OpenCV.

The image correction unit 104 performs perspective transformation of the entire original captured image I_{C}1 by using the obtained perspective transformation matrix, and acquires an image after the transformation. Such perspective transformation can be executed using warpPerspective function of OpenCV. The image after transformation is the standardized image I_{S}1 in which the imaging distance and the imaging viewpoint are standardized.

In step S2 that is a drug detection step, the drug detection unit 106 detects the respective regions of the tablets T1 to T3 from the standardized image I_{S}1. The drug detection unit 106 may detect each region of the tablets T1 to T3 using a trained model for drug detection. Figure 7 shows an example where the drug detection unit 106 detects the region of the tablet T1 and acquires a region image I_{R}1 (process P2).

Here, in the case where the standardized image I_{S}1 is not acquired because of the reasons such as the markers and the gray region are not shown in the captured image I_{C}1, the drug detection unit 106 may detect each region of the tablets T1 to T3 from the captured image I_{C}1.

In step S3 that is an engraved mark and print extraction step, the engraved mark and print extraction unit 108 processes the respective regions of the tablets T1 to T3 that are detected in step S2, and acquires engraved mark and print extraction images that are extracted images of the respective engraved mark and/or print of the tablets T1 to T3. The engraved mark and print extraction unit 108 may acquire the engraved mark and print extraction images by the first trained model 108A. Figure 7 shows an example where the engraved mark and print extraction unit 108 processes the region image I_{R}1 to acquire an engraved mark and print extraction image I_{E}1 that is an extracted image of the engraved mark of the tablet T1 (process P3). The engraved mark and print extraction image I_{E}1 is an image in which an engraved mark portion or a print portion is relatively higher in luminance than portions other than the engraved mark portion or the print portion.

In step S4 that is a first drug type recognition step, the first drug type recognition unit 110 infers the respective drug types of the tablets T1 to T3 from the respective engraved mark and print extraction images of the tablets T1 to T3 that are acquired in step S3, and acquires candidates of the drug types of the tablets T1 to T3. The first drug type recognition unit 110 may acquire candidates R_{D}1 of the drug type of the tablet T1 by the second trained model 110A. Figure 7 shows an example where the first drug type recognition unit 110 acquires the candidates R_{D}1 of the drug type of the tablet T1 from the engraved mark and print extraction image I_{E}1 of the tablet T1 (process P4). In the example shown in Figure 7, the candidates R_{D}1 of the drug type of the tablet T1 includes two drugs: "AB Tablet 1 mg"; and "AC Tablet 1 mg". The two drugs of "AB Tablet 1 mg" and "AC Tablet 1 mg", respectively have their probabilities, that are score values, of "0.5" and "0.4".

In step S5 that is a candidate output step, the candidate output unit 112 outputs the candidates R_{D}1 of the drug type of the tablet T1 acquired in step S4. Here, the candidate output unit 112 selectably displays the candidates R_{D}1 of the drug type of the tablet T1 that are acquired in step S4 on the touch panel display 14. This allows the user to recognize that the candidates of the drug type of the tablet T1 are "AB Tablet 1 mg" and "AC Tablet 1 mg".

The candidate output unit 112 may collectively display the candidates of the respective drug types of the tablets T1 to T3 on the touch panel display 14.

In step S6 that is a confirmation step, the confirmation unit 114 confirms a correct drug for the tablet T1, out of the candidates R_{D}1 of the drug type displayed in step S5. Here, the confirmation unit 114 confirms the drug selected by the user, out of "AB tablet 1 mg" and "AC tablet 1 mg", through the touch panel display 14, as the correct drug for the tablet T1.

Since the drug identification device 100 according to the first embodiment can extract drug-specific identification information without outer shape information, it is possible to recognize drugs with high accuracy based on only the identification information that is imparted by the engraved mark and/or print. Therefore, the drug type of the drug can be recognized without the influence of the imaging environments. Moreover, since the engraved mark and print extraction image can be acquired in one imaging, the effects of less labor in imaging and high usability are demonstrated.

### <Second Embodiment>

### [Functional Configuration of Drug Identification Device]

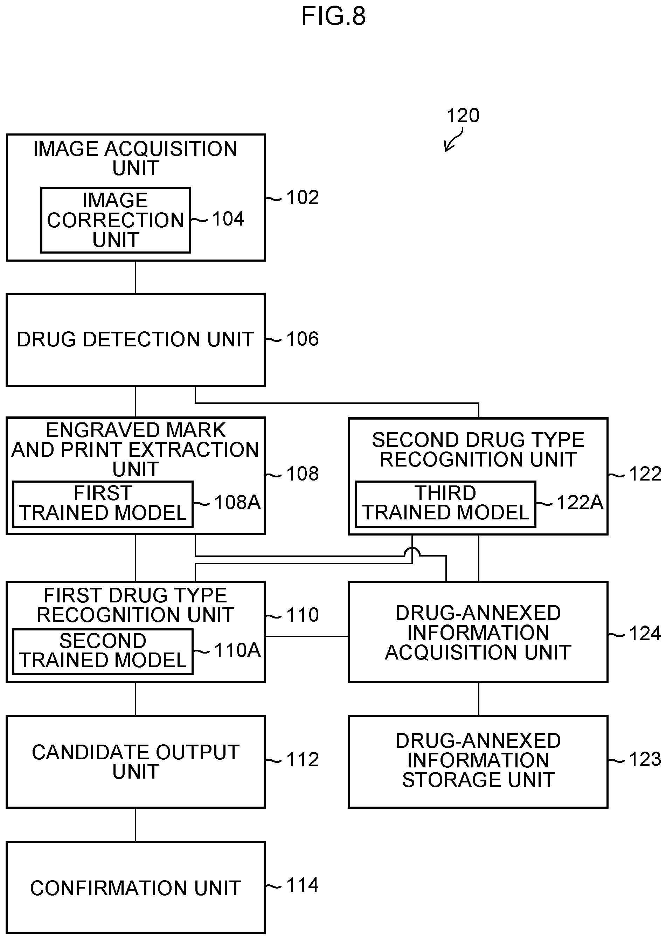

Figure 8 is a block diagram showing the functional configuration of a drug identification device 120 implemented by the smartphone 10. Here, component members in common with those of Figure 5 are designated by identical reference numerals to omit a detailed description thereof. As shown in Figure 8, the drug identification device 120 includes a second drug type recognition unit 122, a drug-annexed information storage unit 123, and a drug-annexed information acquisition unit 124.

The second drug type recognition unit 122 receives input of at least the region of the drug to be identified in the captured image, and infers the drug type of the drug to be identified. When the regions corresponding to the respective drugs to be identified are input, the second drug type recognition unit 122 infers the drug types corresponding to the respective drugs to be identified. The image input into the second drug type recognition unit 122 may be, instead of the image of the region of the drug to be identified, an image (composite extraction image) generated by composing the image of the region of the drug to be identified with an image that is generated by inverting the luminance of the engraved mark and print extraction image that is generated by the engraved mark and print extraction unit 108.

The second drug type recognition unit 122 includes a third trained model 122A. The third trained model 122A is a trained model that outputs the drug type of the drug that is imparted with engraved mark and/or print, upon receiving input of a first image of the drug. The third trained model 122A is machine-learned based on training data sets of different drugs that are imparted with engraved mark and/or print. The training data sets include images of the drugs imparted with engraved mark and/or print, and the drug types of the drugs corresponding to the engraved mark and/or print of the drugs, as sets for learning. To the third trained model 122A, similarly to the first trained model 108A and the second trained model 110A, the CNN can be applied.

Because the second drug type recognition unit 122 uses at least regions of the drugs to be identified in the captured image as an input, the second drug type recognition unit 122 recognizes the drugs not only using the identification information based on engraved mark and/or print of the drugs to be identified, but also using at least one of color, size and shape of the drugs.

The drug-annexed information storage unit 123 stores drug-annexed information on the drugs. The drug-annexed information includes at least one of shape, size, and color of the drugs, in addition to name and identification symbol of the drugs. The drug-annexed information may include information on drugs similar in engraved mark and/or print.

The drug-annexed information acquisition unit 124 acquires the drug-annexed information on a predetermined drug from the drug-annexed information storage unit 123.

### [Drug Identification Method]

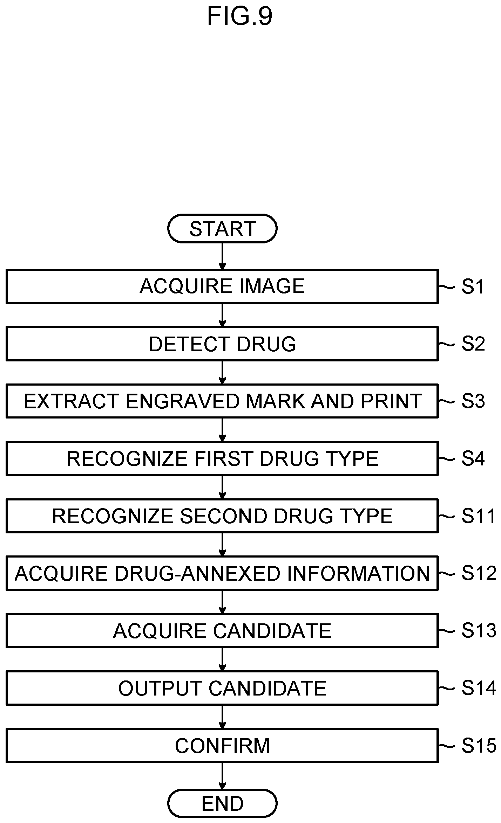

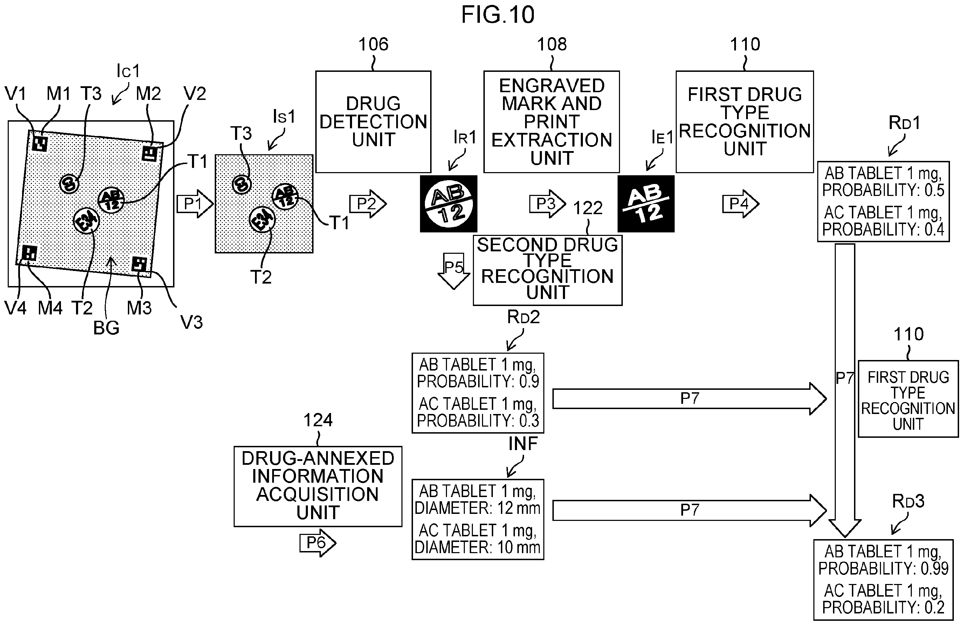

Figure 9 is a flowchart showing the steps of a drug identification method using the drug identification device 120. Figure 10 shows the process in the steps of the drug identification method. Here, component members in common with those of the Figures 6 and 7 are designated by identical reference numerals to omit a detailed description thereof.

The processing in steps S1 to S4 and in processes P1 to P4 is similar to the processing in the first embodiment.

In step S11 that is a second drug type recognition step, the second drug type recognition unit 122 processes the respective regions of the tablets T1 to T3 detected in step S2, and infers the respective drug types of the tablets T1 to T3. The second drug type recognition unit 122 may infer the respective drug types of the tablets T1 to T3 by the third trained model 122A. Figure 10 shows an example where the second drug type recognition unit 122 acquires candidates R_{D}2 of the drug type of the tablet T1 from the region image I_{R}1 of the tablet T1 (process P5).

In the example shown in Figure 10, the candidates R_{D}2 of the drug type of the tablet T1 are two drugs: "AB tablet 1 mg"; and "AC tablet 1 mg". The two drugs of "AB tablet 1 mg" and "AC tablet 1 mg", respectively have their probabilities, that are score values, of "0.9" and "0.3".

Here, the order of the first drug type recognition step and the second drug type recognition step is not particularly limited, and either process may be performed first.

In step S12 that is a drug-annexed information acquisition step, the drug-annexed information acquisition unit 124 acquires drug-annexed information on drugs of the relevant drug type from the drug-annexed information storage unit 123, based on the inference result of the first drug type recognition unit 110 that is acquired in step S4, and on the inference result of the second drug type recognition unit 122 that is acquired in step S5. Figure 10 shows an example where the drug-annexed information acquisition unit 124 acquires drug-annexed information INF for "AB Tablet 1 mg" and "AC Tablet 1 mg", which are the candidate R_{D}1 and the candidate R_{D}2 for the drug type of the tablet T1 (process P6).

In the example shown in Figure 10, the acquired drug-annexed information INF is size information. The size information on "AB tablet 1 mg" and "AC tablet 1 mg" are "diameter 12mm" and "diameter 10mm", respectively.

In step S13 that is a candidate acquisition step, the first drug type recognition unit 110 integrates the inference result of the second drug type recognition unit 122 that is acquired in step S11 and the drug-annexed information that is acquired in step S12, into the inference result of the first drug type recognition unit 110 that is acquired in step S4, and thereby acquires candidates of each drug type of the tablets T1 to T3.

The method of integrating the inference result of the first drug type recognition unit 110 with the inference result of the second drug type recognition unit 122 may be the method to obtain the identification result by weighting both the inference results. The weighting of both the inference results may be based on empirical rules or may be based on machine learning for each drug.

The method of integrating the inference result of the first drug type recognition unit 110 with the inference result of the second drug type recognition unit 122 may be a method of obtaining the sum of the score values in the first drug type recognition unit 110 and the score values in the second drug type recognition unit 122, and then arranging the drug types in descending order, or may also be a method of multiplying the score values of the first drug type recognition unit 110 and the score values of the second drug type recognition unit 122 by a coefficient common to all drug types, obtaining the sum of each of the resultant values, and then arranging them in descending order. The method of integrating the inference result of the first drug type recognition unit 110 with the inference result of the second drug type recognition unit 122 may also be a method of multiplying the score values of the first drug type recognition unit 110 and the score values of the second drug type recognition unit by a coefficient specific to each drug type, obtaining the sum of each of the resultant values, and then arranging them in descending order, or may also be a method of multiplying the score values of the first drug type recognition unit 110 and the score values of the second drug type recognition unit 122 by a coefficient specific to each drug type that is learned so as to maximize drug type identification performance, obtaining the sum of each of the resultant values, and then arranging them in descending order.

Figure 10 shows an example where the first drug type recognition unit 110 integrates the candidate R_{D}2 and the drug-annexed information INF into the candidates R_{D}1 to acquire candidates R_{D}3 (process P7). In the example shown in Figure 10, the candidates R_{D}3 for the drug type of the tablet T1 are "AB tablet 1 mg" and "AC tablet 1 mg", and "AB tablet 1 mg" and "AC tablet 1 mg" respectively have their probabilities, that are score values, of "0.99" and "0.2".

The processing in subsequent step S14 and step S15 is similar to the processing of step S5 and step S6 in the first embodiment.

Since the first drug type recognition unit 110 recognizes the drug type based on the engraved mark and/or print information without using color information, drugs that are identical or similar in engraved mark and print information and that are different in color, may be identified less accurately. On the other hand, since the second drug type recognition unit 122 uses the color information, it may be affected by the imaging environments. In the drug identification device 100 according to the second embodiment, the inference result of the first drug type recognition unit 110, the inference result of the second drug type recognition unit 122, and the drug identification information are integrated, so that the first and second drug type recognition units can compensate mutual disadvantages, and acquire more accurate candidates of the drug type. The drug identification device 100 may integrate at least the inference result of the second drug type recognition unit 122 with the inference result of the first drug type recognition unit 110.

### <Third Embodiment>

### [Functional Configuration of Drug Identification Device]

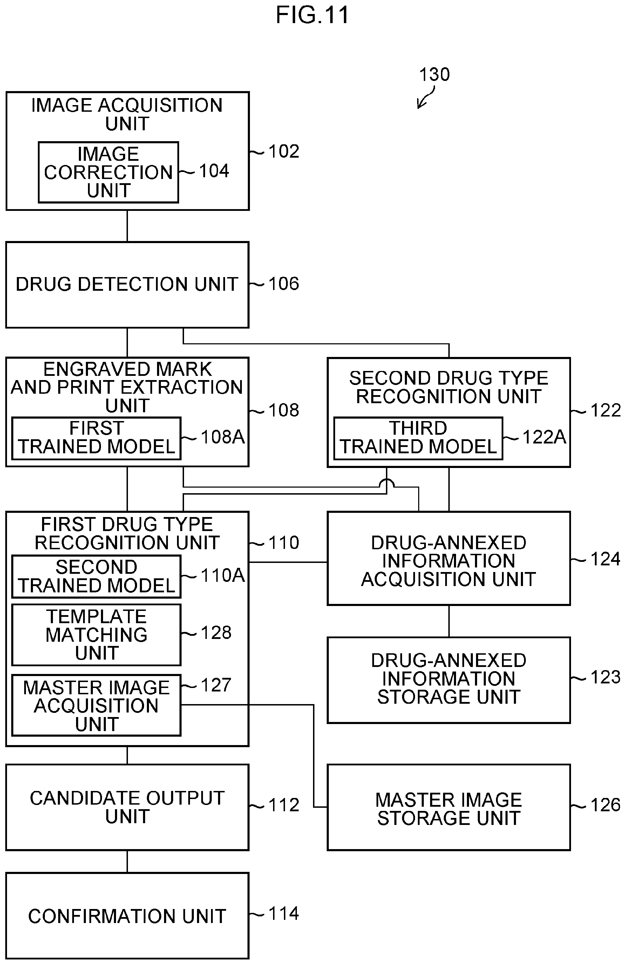

Figure 11 is a block diagram showing the functional configuration of a drug identification device 130 implemented by the smartphone 10. Here, component members in common with those of the Figure 8 are designated by identical reference numerals to omit a detailed description thereof. As shown in Figure 11, the drug identification device 130 includes a master image storage unit 126. The first drug type recognition unit 110 in the drug identification device 130 includes a master image acquisition unit 127 and a template matching unit 128.

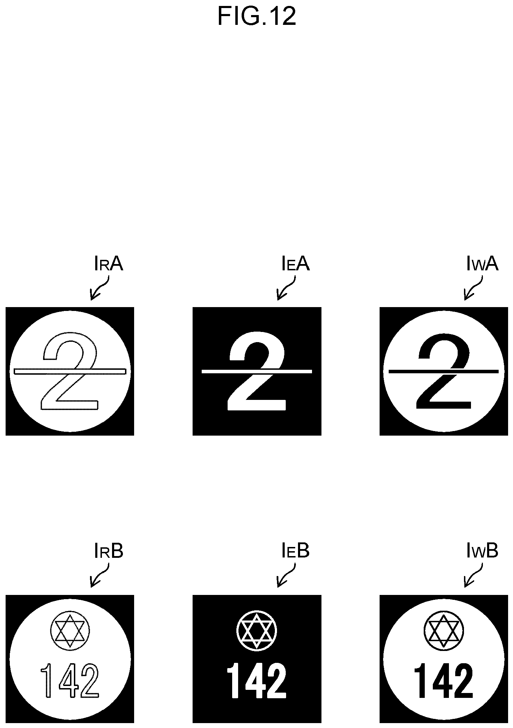

The master image storage unit 126 stores the master images of the drugs. Figure 12 shows examples of the master images of a drug stored in the master image storage unit 126. The master image storage unit 126 stores six master images for one drug: a front-surface region image I_{R}A; a front-surface engraved mark and print extraction image I_{E}A; a front-surface composite extraction image I_{W}A; a rear-surface region image I_{R}B; a rear-surface engraved mark and print extraction image I_{E}B; and a rear-surface composite extraction image IwB.

The front-surface region image I_{R}A and the rear-surface region image I_{R}B are images of the region of the drug that are detected from the captured image of the front surface of the drug and the captured image of the rear surface of the drug, respectively. Original captured images are images captured in a controlled environment with known imaging distance and imaging viewpoint.

The front-surface engraved mark and print extraction image I_{E}A, and the rear-surface engraved mark and print extraction image I_{E}B are images obtained by performing engraved mark and print extraction processing on the respective front-surface region image I_{R}A and the rear-surface region image I_{R}B. The engraved mark and print extraction processing is processing for expressing an engraved mark portion or a print portion with relatively higher in luminance than portions other than the engraved mark portion and the print portion. This processing may be performed by image processing or performed manually by the user. The engraved mark and print extraction image I_{E}A and the rear-surface engraved mark and print extraction image I_{E}B are images used as a reference of each drug when template matching of drugs is performed in the present embodiment, and may also be called an engraved mark master image or engraved mark master images.

The front-surface composite extraction image IwA is an image generated by inverting the luminance of the front-surface engraved mark and print extraction image I_{E}A, and superimposing the inverted image on the front-surface region image I_{R}A. Similarly, the rear-surface composite extraction image I_{W}B is an image generated by inverting the luminance of the rear-surface engraved mark and print extraction image I_{E}B, and superimposing the inverted image on the rear-surface region image I_{R}B.

The master image storage unit 126 retains six images even for images having rotational symmetry such as capsules for convenience. However, as for the engraved mark and print extraction image, a blank file is retained. Here, the capsules are drugs in which powders or granules are filled in each capsule base.

Back to the description of Figure 11, the master image acquisition unit 127 acquires the engraved mark master image of a predetermined drug type from the master image storage unit 126.

The template matching unit 128 performs template matching between the engraved mark and print extraction images of the drugs that are drug type candidates, and the engraved mark master images acquired by the master image acquisition unit 127. The template matching is image processing that compares the engraved mark and print extraction images of the drugs, and the engraved mark master images to search for (retrieve) the engraved mark master images that match the engraved mark and print extraction images, and specifies which drug type the drugs in the engraved mark and print extraction images belong to. In the present embodiment, the region images are acquired after the standardized image is generated by the image correction unit 104. Therefore, it is possible to perform template matching with the engraved mark master images that are generated from captured image with known imaging distance and imaging viewpoint.

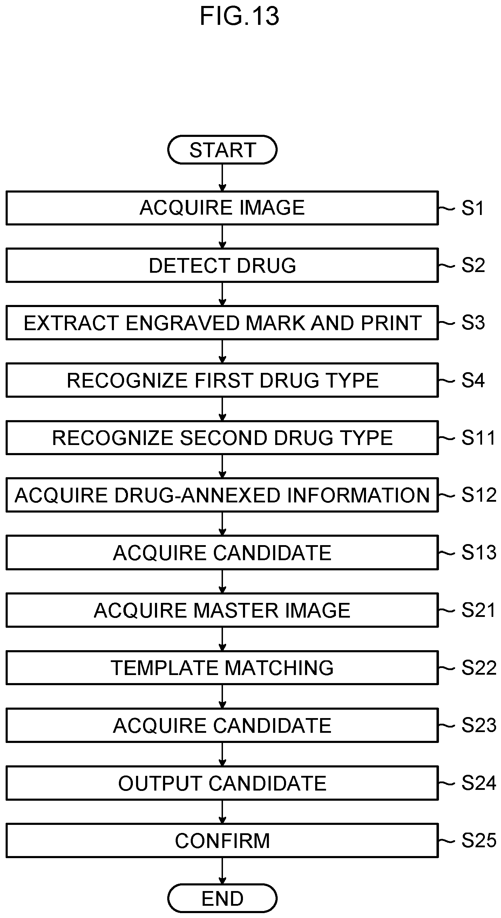

Figure 13 is a flowchart showing the steps of a drug identification method using the drug identification device 130. Here, component members in common with those of Figure 9 are designated by identical reference numerals to omit a detailed description thereof.

The processing in steps S1 to S4 and steps S11 to S13 is similar to those in the second embodiment.

In step S21 that is a master image acquisition step, the master image acquisition unit 127 acquires the engraved mark master images of the respective drug type candidates of the tablets T1 to T3 that are acquired in step S13 from the master image storage unit 126.

For example, in the example described in the second embodiment, the candidates R_{D}3 for the drug type of the tablet T1 are "AB tablet 1 mg" and "AC tablet 1 mg". Therefore, the master image acquisition unit 127 acquires the engraved mark master images of "AB Tablet 1 mg" and the engraved mark master images of "AC Tablet 1 mg", for the tablet T1. When there are a large number of drug type candidates, the engraved mark master images of N (for example, N = 10) number of drug type candidates, that are top N number of candidates highest in score, may be acquired.

In step S22 that is a template matching step, the template matching unit 128 performs template matching between the respective engraved mark and print extraction images of the tablets T1 to T3 and the engraved mark master images acquired in step S21.

For example, the template matching unit 128 performs, for the tablet T1, template matching between the engraved mark and print extraction image I_{E}1 and the engraved mark master image of "AB Tablet 1 mg", and template matching between the engraved mark and print extraction image I_{E}1 and the engraved mark master image of "AC Tablet 1 mg" in order to specify whether the drug type of the tablet T1 is "AB Tablet 1 mg" or "AC Tablet 1 mg".

Here, the template matching unit 128 compares the engraved mark and print extraction image I_{E}1 with the respective engraved mark master images by rotating and moving the engraved mark master images in parallel with the engraved mark and print extraction image I_{E}1, and specifies the drug of the engraved mark master image higher in score indicating a matching degree. In the engraved mark and print extraction image I_{E}1, the direction of the engraved mark and/or print is arbitrary. Therefore, in order to cope with arbitrary rotation of the drug, the template matching unit 128 performs template matching by rotating the engraved mark master images in increments of 1°, for example.

In addition to the drugs that are acquired as the candidates of the drug type in step S13, the master image acquisition unit 127 may also add drugs similar in engraved mark and/or print to the drugs that are acquired in step S13, to the candidates of the drug type, and may acquire the engraved mark master images of the drugs similar in engraved mark and/or print. For example, round-robin template matching is performed for the engraved mark master images, and information on the drugs that are similar in engraved mark and/or print to each other is stored in the engraved mark master images, in advance. Thus, when acquiring the engraved mark master images of drugs, the master image acquisition unit 127 can acquire the engraved mark master images of the drugs similar in engraved mark and/or print based on the information. In the template matching unit 128, template matching is performed on the drug candidates higher in score and also on the candidates similar to these drug candidates. Therefore, the drugs can be extracted without omission so that the accuracy of drug recognition can be enhanced.

In step S23 that is a secondary candidate acquisition step, the first drug type recognition unit 110 uses the template matching results of step S22 to acquire the drug candidates for the tablets T1 to T3 that are further narrowed down.

The processing in subsequent steps S24 and S5 is similar to the processing in steps S5 and S6 in the first embodiment. The drugs specified by template matching in step S22 may be confirmed as the correct drugs for the tablets T1 to T3.

When the engraved mark information can be extracted, it is possible to perform highly accurate collation by the template matching with the engraved mark master images. However, the template matching has such disadvantages that all the drug types are to be searched (search targets) and it takes a very long time (several tens to several hundreds of seconds) in a case where the rotation angle is arbitrary. On the other hand, when collation is performed by the trained model, identification results can be obtained in a very short time (several tens of milliseconds), though an accuracy rate is less than that of the template matching with the engraved mark master images. In the drug identification device 130 according to the third embodiment, because template matching is performed on the top drug candidates acquired by the first drug type recognition unit 110, it becomes possible to acquire the candidates for the drug type of the drugs to be identified while satisfying both accuracy and speed.

### [Handling of Capsules]

There are capsules of the same color, same type, and same size, and it is necessary to recognize an identification symbol on the capsules in order to identify the drug type. However, there are a huge number of patterns in the images of the capsules due to the arbitrariness in rotation and occlusion of the images of the capsules. Therefore, it is difficult to recognize the identification symbol based on both the machine learning and the template matching.

In view of these circumstances, the drug identification device 100 (120, 130) performs first stage identification for the capsules based on color, size, and shape in order to narrow down the candidates of the drug type. The candidates of the drug type are capsules having scores of top N-th levels (for example, N = 5), or the capsules having a certain score or higher.

The drug identification device 100 (120, 130) presents the user with the master image, the identification symbol and the drug name of these few candidates, side by side. In addition, the drug identification device 100 (120, 130) enlarges and displays the captured image, or concurrently displays the extracted image of the engraved mark and/or print with the captured image, to allow the user to easily select the correct drug.

### [Graphical User Interface (GUI) of Mobile Terminal device]

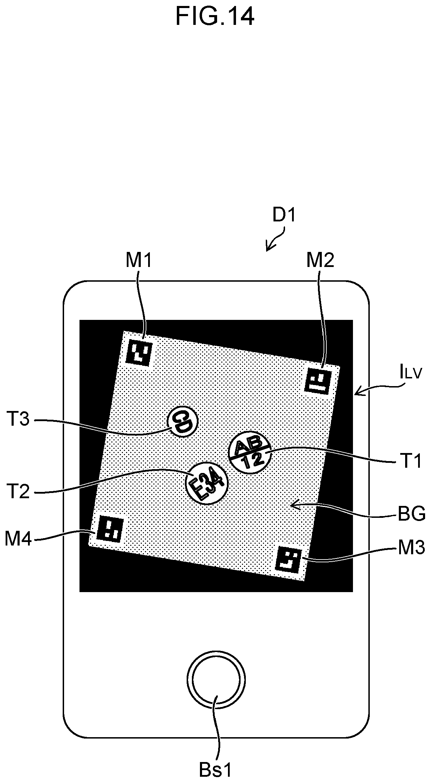

Figures 14 to 18 show screen displays D1 to D5 on the touch panel display 14 of the smartphone 10 in each step in the respective drug identification methods.

Figure 14 shows the screen display D1 in the image acquisition step. The screen display D1 displays a live view image I_{LV} of objects to be imaged and a shooting button B_{S}1.

The live view image I_{LV} is a moving image of a region captured by the out-camera 22 and displayed in real time, simultaneously with capturing. The live view image I_{LV} includes the tablets T1 to T3, the markers M1 to M4, and the background BG in the reference gray color.

In a case where the user taps the shooting button B_{S}1, actual shooting (actual image capturing) by the out-camera 22 is performed, and the screen display D1 shifts to the screen display D2. The captured image that is a still image generated by actual shooting is stored in the memory 34.

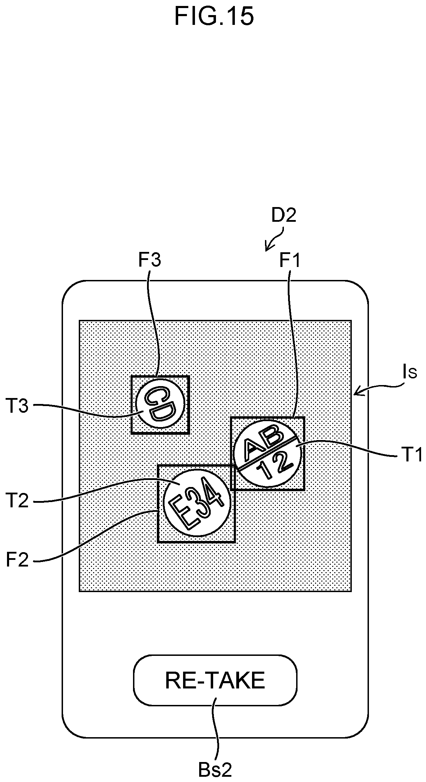

Figure 15 shows the screen display D2 in the drug detection step. The screen display D2 displays a standardized image Is and re-take button (re-shooting button) B_{S}2. The standardized image I_{S} is an image generated by standardizing the captured image and then cutting out a region of the captured image enclosed with a straight line that connects the markers M1 to M4. Displayed here are frames F1, F2, and F3 that enclose the respective regions of the tablets T1, T2 and T3 that are detected from the standardized image I_{S}.

In the screen display D2, the standardized image Is is enlarged and displayed in response to pinching by the user on the touch panel display 14.

In a case where one of the regions of the tablets T1 to T3 (one of the regions enclosed with the frames F1 to F3) is tapped on the screen display D2, the screen display D2 shifts to the screen display D3. In a case where the re-take button B_{S}2 is tapped, the screen display D2 shifts to the screen display D1.

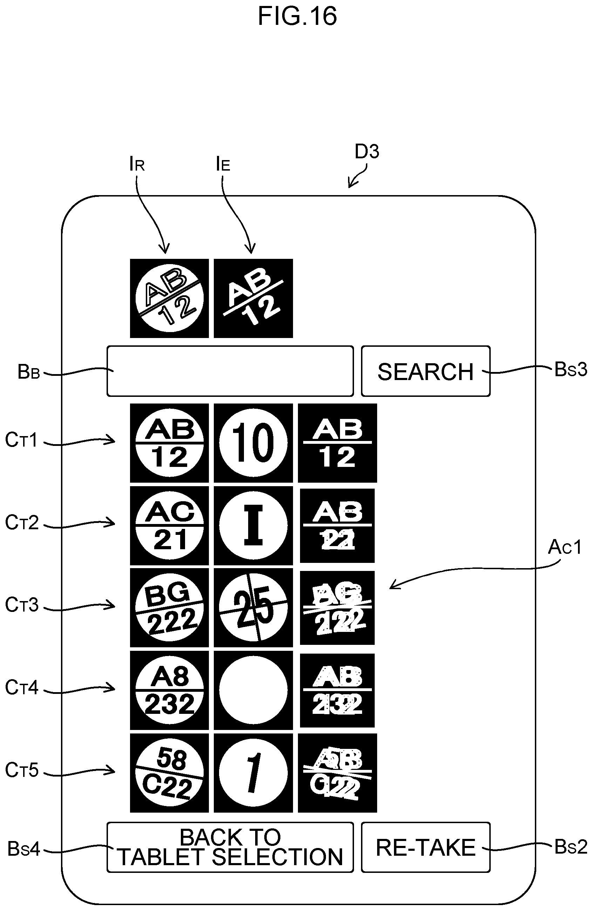

Figure 16 shows the screen display D3 in the candidate output step. Shown here is a case where the candidates for the drug type of the tablet T1 are acquired in response to the tapping of the region of the tablet T1 (the region enclosed with the frame F1) on the screen display D2.

In the screen display D3, the region image I_{R} of the tablet T1, which is a drug to be identified and is tapped in the screen display D2, and the engraved mark and print extraction image I_{E} of the region image I_{R} are displayed side by side. A composite extraction image of the tablet T1 may further be displayed side-by-side.

The screen display D3 also displays a text box B_{B}, a search button B_{S}3, a back button B_{S}4, and the re-take button B_{S}2.

In a case where the back button B_{S}4 is tapped, the screen display D3 shifts to the screen display D2. In a case where the re-take button B_{S}2 is tapped, the screen display D3 shifts to the screen display D1.

In addition, the screen display D3 includes a candidate drug display region A_{C}1. In the candidate drug display region A_{C}1, candidate drugs that are recognized as the candidates for the drug type of the drug that is the tablet T1, are selectably displayed from top to bottom in the order of higher scores. In the candidate drug display region Ac2, the candidate drugs with lower scores are displayed in response to swiping from the lower side toward the upper side of the screen by the user. In the candidate drug display region A_{C}1, the candidate drugs with higher scores are displayed in response to subsequent swiping from the upper side toward the lower side of the screen by the user.

For each of the candidate drugs C_{T}1 to C_{T}5 of the tablet T1, the candidate drug display region A_{C}1 displays the front-surface composite extraction image and the rear-surface composite extraction image, which are the master images, and overlap image generated by superimposing the engraved mark and print extraction image of the tablet T1 and the engraved mark and print extraction image of the target surface in the master images. The rotation angle and the parallel movement position of the master image when the maximum score is obtained in template matching, is determined as a reference direction of the master image. The overlap image is displayed according to the reference direction of the master image. The composite extraction image is displayed in such a manner that the direction of the engraved mark and/or print in the composite extraction image is aligned with the direction of the engraved mark and/or print in the overlap image. In this case, regarding the candidate drug C_{T}1 displayed at the top, the engraved mark and print extraction image of the tablet T1 is matched with the engraved mark and print extraction image of the master image, and therefore the overlap image is clearly displayed.

In this way, in addition to the region image I_{R} and the engraved mark and print extraction image I_{E} of the tablet T1, the screen display D3 displays the front-surface the composite extraction images and the rear-surface composite extraction images, which are master images of the candidate drugs C_{T}1 to C_{T}5 for the tablet T1, and the overlap images based on the rotation angle and the parallel movement position of the master image when the highest score is obtained in template matching. Therefore, the user can determine whether the recognition result is correct or wrong with high visibility.

In a case where an arbitrary candidate drug is tapped in the candidate drug display region A_{C}1 in the screen display D3, the screen display D3 shifts to the screen display D4.

The text box B_{B} is a search window into which the user can enter a character string and which is also an interface for specifying the drug type of the tablet T1 by the character string input by the user. In a case where there is no correct drug in the candidate drug display region A_{C}1, the user may input the identification symbol of the tablet T1, as text input, into the text box B_{B} while referring to the region image I_{R} and the engraved mark and print extraction image I_{E} of the tablet T1 on the screen display D3, and then, tap the search button Bs3. As a result of this operation by the user, it is possible to acquire a candidate (candidates) of the drug type related to the input character string. The text to be input may be the identification symbol such as "AB 12" or may be the name of the drug. In a case where the text box B_{B} is selected, the smartphone 10 may displays a keyboard on the lower side of the screen such that the user can input text into the text box B_{B}, or the smartphone 10 may receive text input using so-called voice input.

In a case where text is input into the text box B_{B} on the screen display D3 and the search button B_{S}3 is tapped, the screen display D3 shifts to screen display D5.

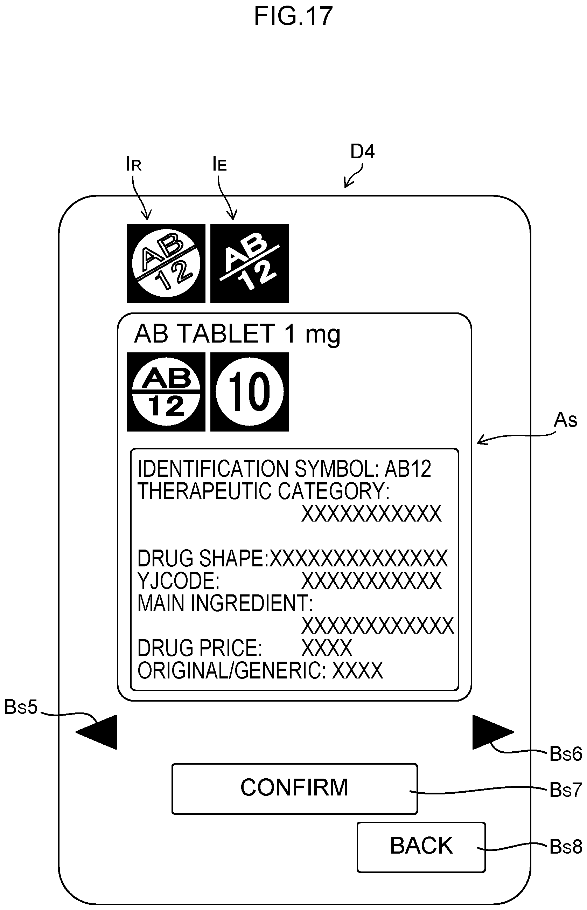

Figure 17 shows the screen display D4 shifted from the screen display D3. Here, the case where the candidate drug C_{T}1 is tapped in the screen display D3 is shown.

The screen display D4 displays the region image I_{R} and the engraved mark and print extraction image I_{E} of the tablet T1 that is a drug to be identified, as well as an upper shift button B_{S}5, a lower shift button B_{S}6, a confirmation button B_{S}7 and a back button B_{S}8.

In a case where the back button B_{S}8 is tapped, the screen display D4 shifts to the screen display D3.

The screen display D4 also includes a selected drug display region A_{S}. The selected drug display region A_{S} displays the information on the drug that is tapped in the screen display D3. Here, the selected drug display region A_{S} displays the name of the candidate drug C_{T}1, composite extraction images, identification symbol, therapeutic category, dosage form, YJ code (individual pharmaceutical code), principal ingredient, drug price, and information related to the candidate drug C_{T}1 such as original/generic. Additional information related to the candidate drug C T1, such as side effect information and interaction information, may further be displayed.

In a case where the upper shift button Bs5 is tapped, the selected drug display region A_{S} displays a candidate drug whose score ranking is higher by one, than the candidate drug currently displayed. In a case where the lower shift button B_{S}6 is tapped, the selected drug display region A_{S} displays a candidate drug whose score ranking is lower by one, than the candidate drug currently displayed. In a case where the confirmation button B_{S}7 is tapped, the drug displayed in the selected drug display region As is confirmed as the correct drug for the tablet T1 that is a drug to be identified.

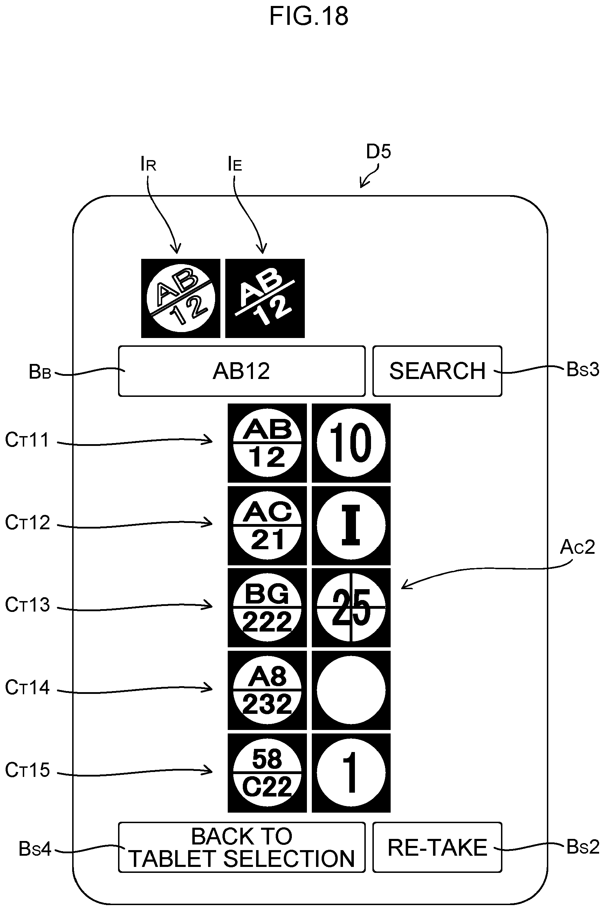

Figure 18 shows the screen display D5 shifted from the screen display D3. When the search button B_{S}3 is tapped in the screen display D3, the first drug type recognition unit 110 searches the drug-annexed information storage unit 123 using the input text, to acquire the candidates of the drug type corresponding to the input text. Described here in the case where the search button B_{S}3 is tapped after "AB12" is input in the text box B_{B} in the screen display D3.

The screen display D5 displays the region image I_{R} and the engraved mark and print extraction image I_{E} of the tablet T1 that is a drug to be identified, as well as the text box B_{B}, the search button B_{S}3, the back button B_{S}4, and the re-take button B_{S}2.

In a case where the back button B_{S}4 is tapped, the screen display D5 shifts to the screen display D2. In a case where the re- take button B_{S}2 is tapped, the screen display D5 shifts to the screen display D1.

The screen display D5 includes the candidate drug display region A_{C}2. In the candidate drug display region Ac2, the master images of the candidates of the drug types corresponding to the text that is input in the text box B_{B} in the screen display D3, are selectably displayed from top to bottom in the order of a higher degree of matching with the text.

Here, the candidate drug display region Ac2 displays the front-surface composite extracted image and the rear-surface composite extracted image of the master images, for each of the candidate drugs C_{T}11 to C_{T}15.

In the candidate drug display region Ac2, the candidate drugs relatively lower in matching degree in the text search result are displayed, in response to swiping from the lower side toward the upper side of the screen by the user. In the candidate drug display region A_{C}2, the candidate drugs relatively higher in matching degree in the text search result are displayed, in response to swiping from the upper side toward the lower side of the screen by the user.

In a case where an arbitrary candidate drug is tapped in the candidate drug display region A_{C}2 in the screen display D5, the screen display D5 shifts to the screen display D4.

In this way, even in a case where any appropriate candidates of the drug type cannot be acquired, using the text box B_{B} enables the user to acquire the candidate drugs by text search while referring to the region image I_{R} and the engraved mark and print extraction image I_{E} of the tablet T1 that is a drug to be identified.

In a case where there is no correct drug in the candidate drugs display region Ac2, the user may input different text into the text box B_{B} and tap the search button Bs3, to perform re-search for the correct drug.

As described before, the drug identification device may include a tablet-type computer terminal. Although a detailed description of the tablet-type computer terminal is omitted, the tablet-type computer terminal includes an unshown display with a relatively larger area than the smartphone 10 and an unshown camera.

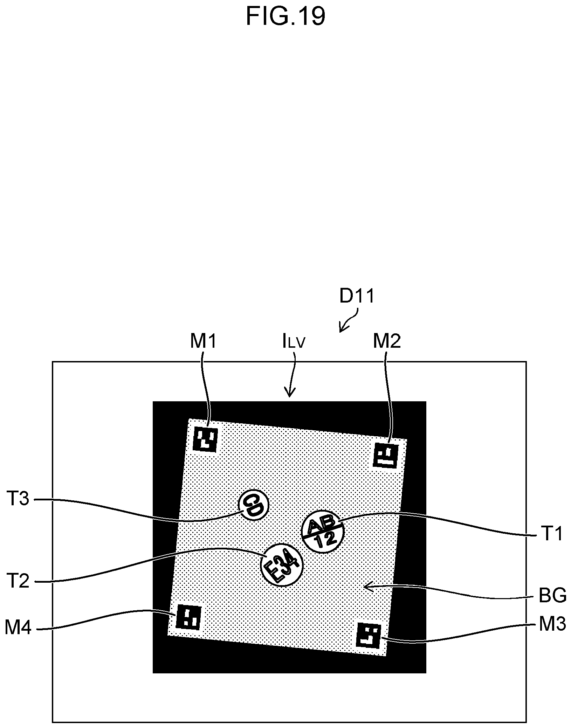

Figures 19 to 22 show screen displays D11 to D14 on the display of the tablet-type computer terminal in each step in the respective drug identification methods. Here, component members in common with those of the Figures 14 to 18 are designated by identical reference numerals to omit a detailed description thereof.

Figure 19 shows the screen display D11 in the image acquisition step. The screen display D11 displays a live view image I_{LV} of objects to be imaged. In a case where the user taps the shooting button which is not shown, the actual shooting is performed, and the screen display D11 shifts to the screen display D12

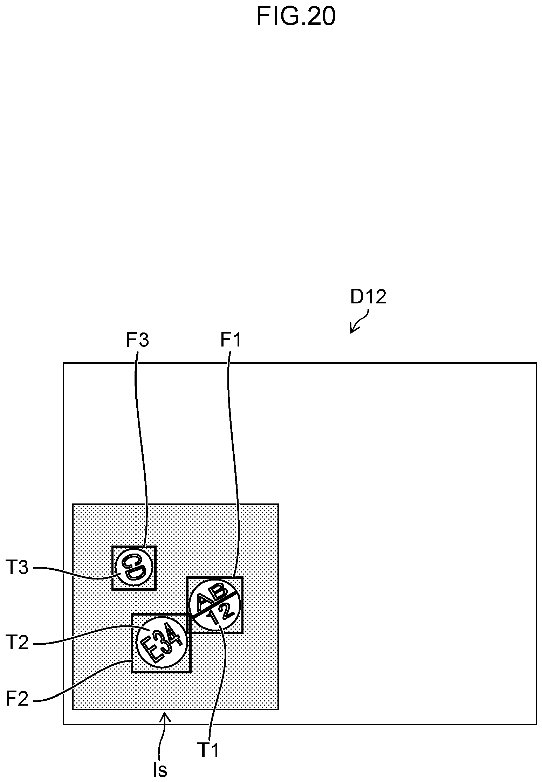

Figure 20 shows the screen display D12 in the drug detection step. The screen display D12 displays a standardized image I_{S}. In a case where the user performs an operation to select an arbitrary drug in the screen display D12, the screen display D12 shifts to the screen display D13.

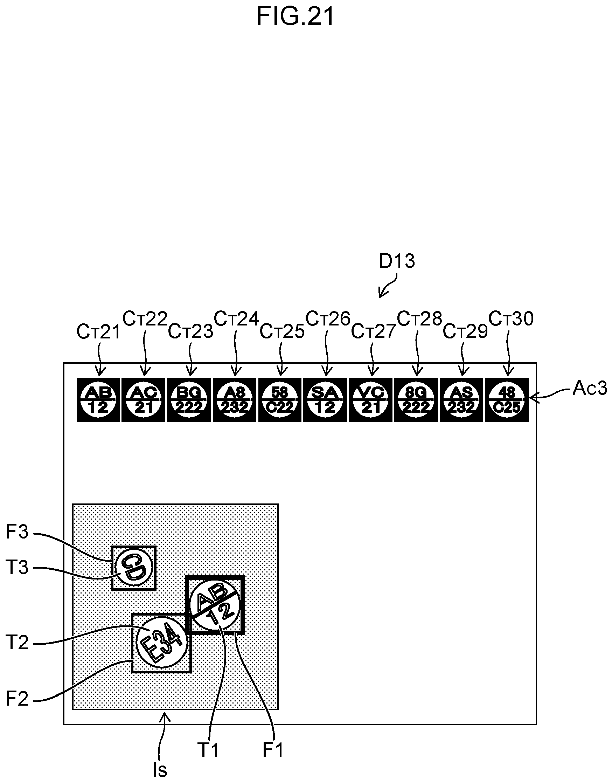

Figure 21 shows the screen display D13 in the candidate output step. Shown here is the case where the candidates of the drug type of the tablet T1 are acquired, after the operation to select the tablet T1 is performed in the screen display D12.

In the standardized image I_{S} on the screen display D13, the frame F1 of the tablet T1, which is a drug to be identified and is selected in the screen display D12, is displayed with a relatively thicker line than the frames F2 and F3. The frame of the drug to be identified may be displayed in a different color from the lines of the frames of the other drugs.

The screen display D13 includes a candidate drug display region A_{C}3. In the candidate drug display region Ac3, the candidate drugs, recognized as the candidates for the drug type of the drug that is the tablet T1, are displayed from left to right in the order of higher scores. Here, the candidate drug display region Ac3 displays the master images of the candidate drugs C_{T}21 to C_{T}30 of the tablet T1, with the engraved mark or print on the surface being emphasized. In the candidate drug display region Ac3, the candidate drugs with lower scores may be displayed in response to swiping from the right to the left side of the screen by the user.

In a case where one of the candidate drugs C_{T}21 to C_{T}30 is selected in screen display D13, the selected drug is confirmed as the correct drug of the tablet T1 that is a drug to be identified.

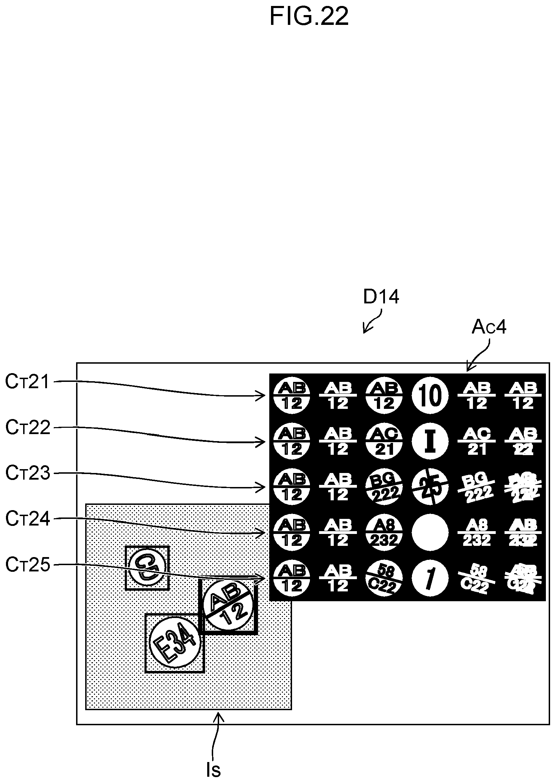

Figure 22 shows the screen display D14 in the secondary candidate acquisition step. The screen display D14 includes a candidate drug display region A_{C}4. In the candidate drug display region A_{C}4, the master images of the candidate drugs C_{T}21 to C_{T}25 are displayed side by side with the captured image and the engraved mark and print extraction image of the tablet T1. The candidate drugs C_{T}21 to C_{T}25 are top five candidate drugs having top scores out of ten candidate drugs of the tablet T1, and the master images of the candidate drugs C_{T}21 to C_{T}25 are displayed in the candidate drug display region Ac4 from top to bottom in the order of higher scores in template matching.

The master images of each of the candidate drugs, which are displayed side by side with the captured image and the engraved mark and print extraction image of the tablet T1, are: the front-surface composite extraction image; the rear-surface composite extraction image; and the engraved mark and print extraction image of the target surface (here, the front surface) in order from the left. In addition, side by side with the master images of each of the candidate drugs, the overlap image generated by superimposing the engraved mark and print extraction image of the tablet T1 and the engraved mark and print extraction images of the target surface of each master image, is displayed. Similarly to the candidate drug display region A_{C}1 in the screen display D3, a reference direction of the master image is determined based on the rotation angle and the parallel movement position of the master image when the maximum score is obtained in template matching, and these images are displayed according to the reference direction of the master image. In Figure 22, in the case of the candidate drug C_{T}21 displayed at the top, the engraved mark and print extraction image of the tablet T1 is matched with the engraved mark and print extraction image of the master image. Therefore, the overlap image of the candidate drug C_{T}21 is clearly displayed.

In a case where one of the candidate drugs C_{T}21 to C_{T}25 is selected in the screen display D14, the selected drug is confirmed as the correct drug for the tablet T1 that is a drug to be identified.

### [Imaging Assistance Device]

Figure 23 shows a top view of the imaging assistance device 70 for capturing images to be input into the drug identification devices 100, 120, and 130. Figure 24 is a cross-sectional view along a 24-24 line in Figure 23. Figure 24 also shows the smartphone 10 that captures an image of a drug using the imaging assistance device 70.

As shown in Figures 23 and 24, the imaging assistance device 70 includes a casing 72, a drug loading table 74, a main light source 75, and an auxiliary light source 78.

The casing 72 includes a square bottom surface plate 72A that is supported horizontally, four rectangular side surface plates 72B, 72C, 72D, and 72E that are vertically fixed at end parts of the respective sides of the bottom surface plate 72A.

The drug loading table 74 is fixed to the top surface of the bottom surface plate 72A of the casing 72. The drug loading table 74 is a member having a surface on which a drug (drugs) is loaded. Here, the drug loading table 74 is a thin sheet-like member made of plastic or paper, and has a square shape in a top view. The loading surface on which the drug (drugs) to be identified is to be loaded, has the reference gray color. The reference gray color, if expressed by 256 gradation values from 0 (black) to 255 (white), is a gradation value in the range of 130 to 220, and more preferably a gradation value in the range of 150 to 190, for example.

In general, in a case where a drug (drugs) is imaged with the smartphone 10 on a white or black background, color skip may occur due to an automatic exposure adjustment function, and sufficient engraved mark information may not be obtained. According to the drug loading table 74, the loading surface is in gray color so that the details of the engraved mark may be captured while suppressing the color skip. In addition, when pixel value of the gray color in the captured image are acquired and the acquired pixel value is corrected based on a true gradation value of the gray color, the color correction or exposure correction of the captured image can be implemented.

Reference markers 74A, 74B, 74C, and 74D, which are each formed in black color and white color, are arranged at the four corners of the loading surface of the drug loading table 74 by pasting or print. As the reference markers 74A, 74B, 74C, and 74D, any marker may be used. Here, simple ArUco markers robust in detection are used. The ArUco markers are square two-dimensional markers having a code portion made of 4 to 8 dots in longitudinal and lateral directions. A data set of four dots in longitudinal and lateral directions is preferably used as it is simple and highly robust in detection. It is preferable that each dot includes a plurality of pixels.

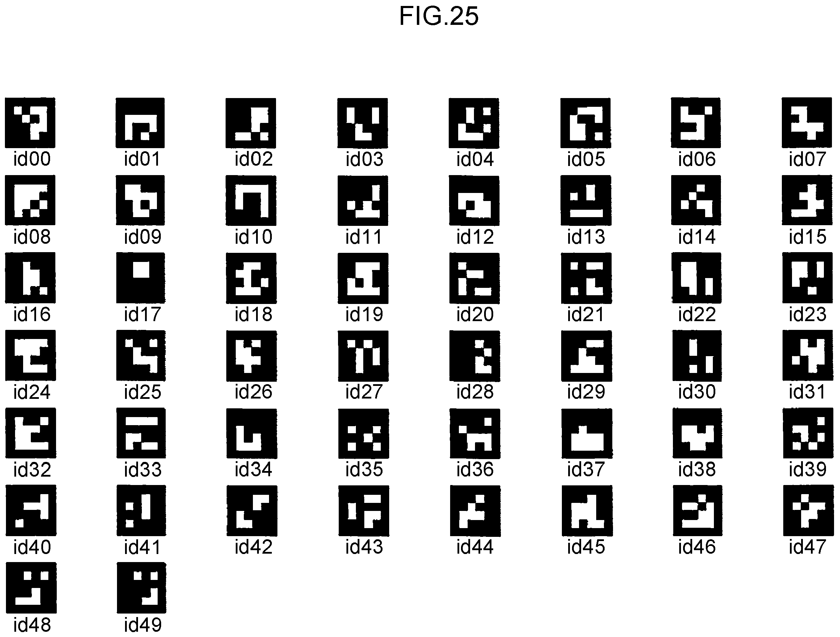

Figure 25 shows an example of a data set of ArUco markers each made of four dots in longitudinal and lateral directions. Figure 25 shows ArUco markers of 50 types from id0 to id49. In the present embodiment, the reference markers 74A, 74B, 74C and 74D are ArUco markers of id3, id13, id48, and id30, respectively. By arranging these four ArUco markers in the respective positions, higher detection robustness can be achieved.

Here, the size of the reference markers 74A, 74B, 74C and 74D is preferably 3 to 30 mm in the longitudinal and lateral directions, and more preferably 5 to 15 mm.

Also, a distance between the reference marker 74A and the reference marker 74B and a distance between the reference marker 74A and the reference marker 74D are each preferably 20 to 100 mm, and more preferably 20 to 60 mm.

Here, the bottom surface plate 72A of the casing 72 may also serve as the drug loading table 74.

The main light source 75 and the auxiliary light source 78 constitute the illumination device used to capture an image of the drug (drugs) to be identified. The main light source 75 is used to extract the engraved mark on the drug to be identified. The auxiliary light source 78 is used to accurately obtain the color and shape of the drug to be identified. The imaging assistance device 70 may or may not be provided with the auxiliary light source 78.

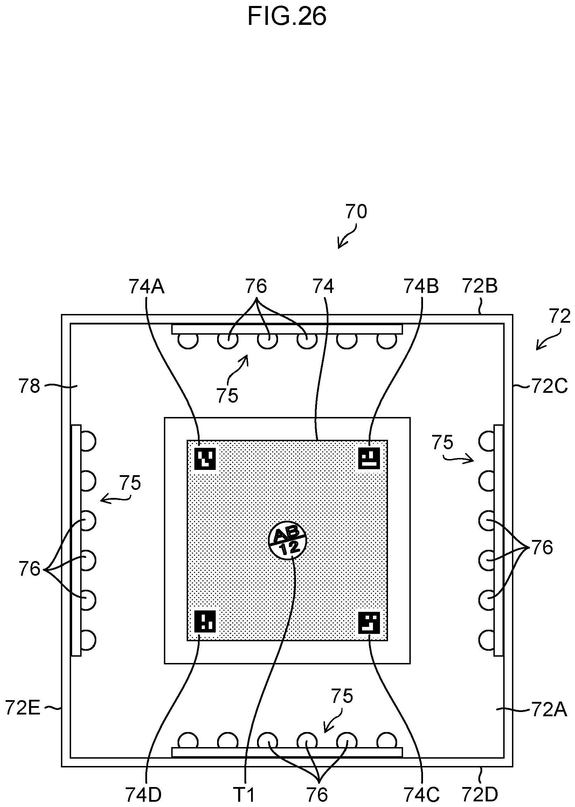

Figure 26 is a top view of the imaging assistance device 70 in a state where the auxiliary light source 78 is removed.

The main light source 75 includes LEDs 76. The LEDs 76 each have a light emitting unit that is a white light source with a diameter of 10 mm or less. Here, six LEDs 76 are arranged on each of four rectangular side surface plates 72B, 72C, 72D and 72E, so as to be aligned in a horizontal direction at a fixed height. As a result, the main light source 75 irradiates the drug to be identified with illumination light from at least four directions. Note that the main light source 75 may irradiate the drug to be identified with illumination light from at least two directions.

An angle θ formed between irradiation light emitted by the LEDs 76 and the top surface (horizontal plane) of the drug to be identified is preferably in the range of 0° to 45° to extract the engraved mark. The main light source 75 may include bar-shaped light sources which have a width of 10 mm or less, and are arranged horizontally on the respective four rectangular side surface plates 72B, 72C, 72D and 72E .

The main light source 75 may constantly be turned on. This enables the imaging assistance device 70 to irradiate the drug to be identified with illumination light from all directions. An image captured in a state where all the LEDs 76 are turned on, is called a total illumination image. According to the total illumination image, it is easy to extract the print of the drug to be identified imparted with the print.

In the main light source 75, the turning on and off of the LEDs 76 may be switched according to timing, or may be switched with a switch not shown. This enables the imaging assistance device 70 to irradiate the drug to be identified with illumination light from different directions by the main light source 75.

For example, an image captured in the state where only six LEDs 76 that are provided on the side surface plate 72B are turned on, is called a partial illumination image. Similarly, a partial illumination image is captured in the state where only six LEDs 76 provided on the side surface plate 72C are turned on, a partial illumination image is captured in the state where only six LEDs 76 provided on the side surface plate 72D are turned on, and a partial illumination image is captured in the state where only six LEDs 76 provided on the side surface plate 72E are turned on. Therefore, it is possible to acquire four partial illumination images captured by irradiating the illumination light from the respective different directions. Using the partial illumination images captured by irradiating the illumination light from the respective different directions, it becomes easy to extract the engraved mark of the drug to be identified that is imparted with the engraved mark.

The auxiliary light source 78 is a plate-shaped planar white light source, and has a square outer shape and a square opening in its center. The auxiliary light source 78 may be an achromatic reflector that diffuses and reflects the irradiation light from the main light source 75. The auxiliary light source 78 is arranged between the smartphone 10 and the drug loading table 74 so that the drug to be identified is uniformly irradiated with irradiation light from an imaging direction (the direction of an optical axis of the camera). The illuminance of the irradiation light from the auxiliary light source 78 that irradiates the drug to be identified is relatively lower than the illuminance of the irradiation light from the main light source 75 that irradiates the drug to be identified.

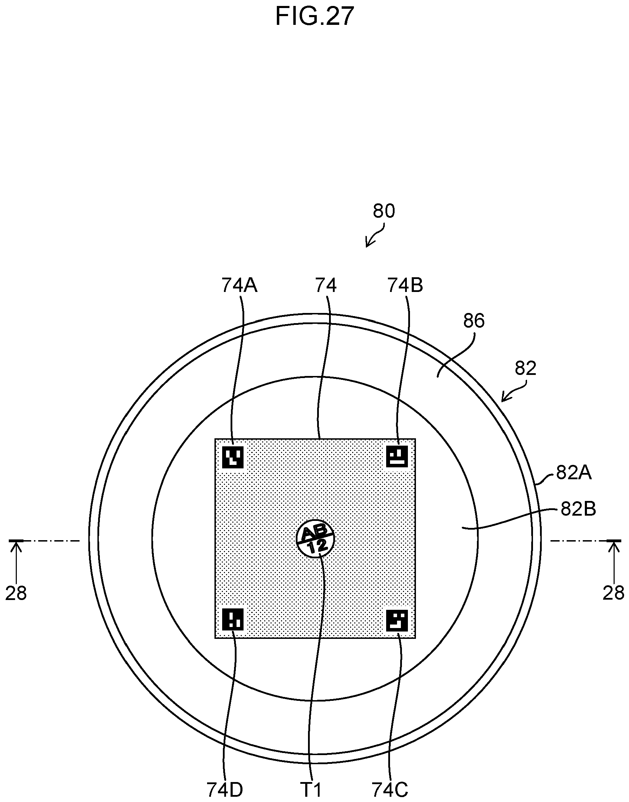

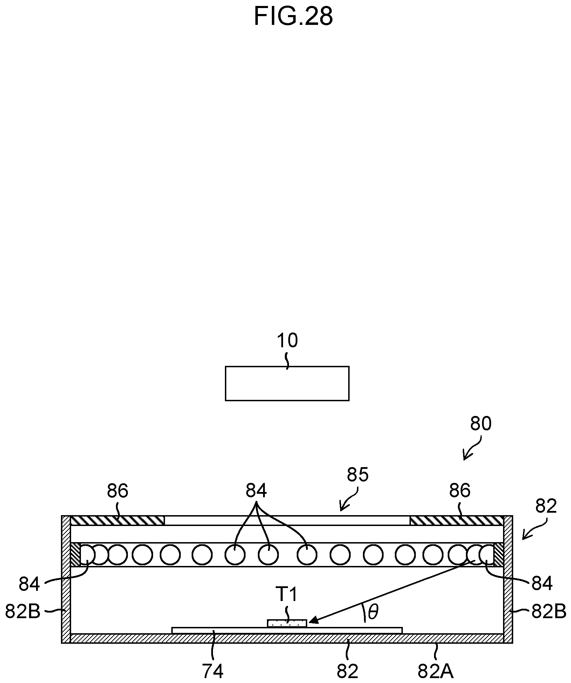

Figure 27 shows the top view of an imaging assistance device 80 according to another embodiment. Figure 28 is a cross-sectional view along a 27-27 line in Figure. 27. Figure 28 also shows the smartphone 10 that captures an image of a drug using the imaging assistance device 80. Here, component members in common with those of the Figures 23 and 24 are designated by identical reference numerals to omit a detailed description thereof. As shown in Figures 27 and 28, the imaging assistance device 80 includes a casing 82, a main light source 84, and an auxiliary light source 86. The imaging assistance device 80 may not include the auxiliary light source 78.

The casing 82 has a cylindrical shape, and includes a circular bottom surface plate 82A supported horizontally and a side surface plate 82B fixed vertically to the bottom surface plate 82A. On the top surface of the bottom surface plate 82A, the drug loading table 74 is fixed.

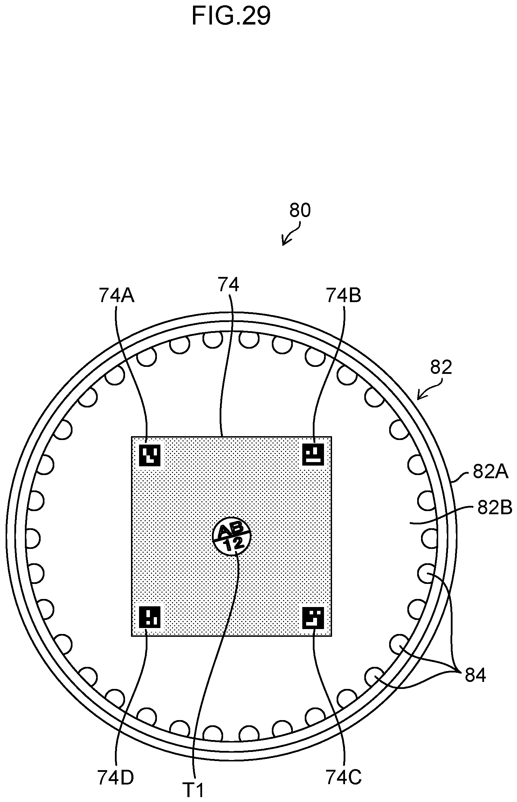

The main light source 84 and the auxiliary light source 86 constitute the illumination device used to capture captured images of the drug to be identified. Figure 29 is a top view of the imaging assistance device 80 in a state where the auxiliary light source 86 is removed.

The main light source 84 includes 24 LEDs 85 that are arranged on the side surface plate 82B so as to form a ring shape at a fixed height and at fixed intervals in the horizontal direction. The main light source 84 may constantly be turned on. Or, turning on and off of the LEDs 85 may be switched.

The auxiliary light source 86 is a plate-shaped planar white light source and has a circular outer shape and a circular opening in its center. The auxiliary light source 86 may be an achromatic reflector that diffuses and reflects the irradiation light of the main light source 84. The illuminance of the irradiation light from the auxiliary light source 86 that irradiates the drug to be identified is relatively lower than the illuminance of the irradiation light from the main light source 84 that irradiates the drug to be identified.

The imaging assistance device 70 and the imaging assistance device 80 may include an unshown fixing mechanism which fixes the smartphone 10 that images the drug to be identified, at a position with a reference imaging distance and imaging viewpoint. The fixing mechanism may be configured such that the distance between the drug to be identified and the camera can be changed according to a focal length of the photographic lens 50 of the smartphone 10.

### [Drug Identification System]

### [Configuration of Drug Identification System]

Description has been given of the example where a mobile terminal device alone constitutes the drug identification device 100 that specifies the correct drug for a drug to be identified that is imparted with engraved mark and/or print. However, the drug identification device may include a mobile terminal device and a server that can communicate with the mobile terminal device, or may include a server alone. Description is now given of a drug identification system that is implemented by a mobile terminal device and a server, which can communicate with each other.

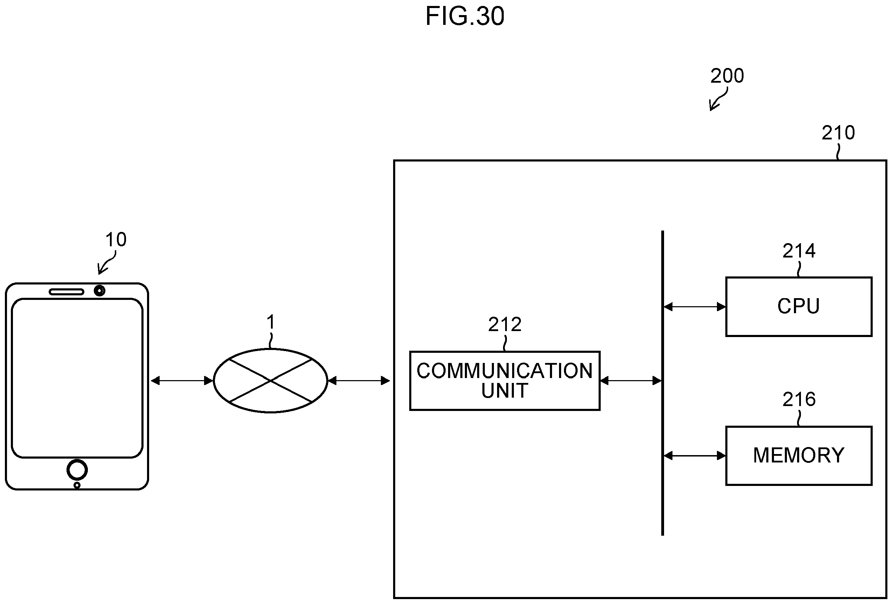

Figure 30 shows the configuration of a drug identification system 200. As shown in Figure 30, the drug identification system 200 includes a smartphone 10, and a server 210. The smartphone 10 and the server 210 are connected to each other via a network 1, such as the Internet and a local area network (LAN) so as to allow data communication. Although only one smartphone 10 is shown in Figure 30, the drug identification system 200 may include more than one smartphone 10.

The server 210 includes a communication unit 212, a CPU 214, and a memory 216.

The communication unit 212 communicates with the smartphone 10 via the network 1. The CPU 214 is a processor that executes commands stored in the memory 216, and has a hardware structure similar to the CPU 28. The memory 216 stores commands to be executed by the CPU 214. The memory 216 also stores data necessary for drug identification.

### [Functional Configuration of Drug Identification System]

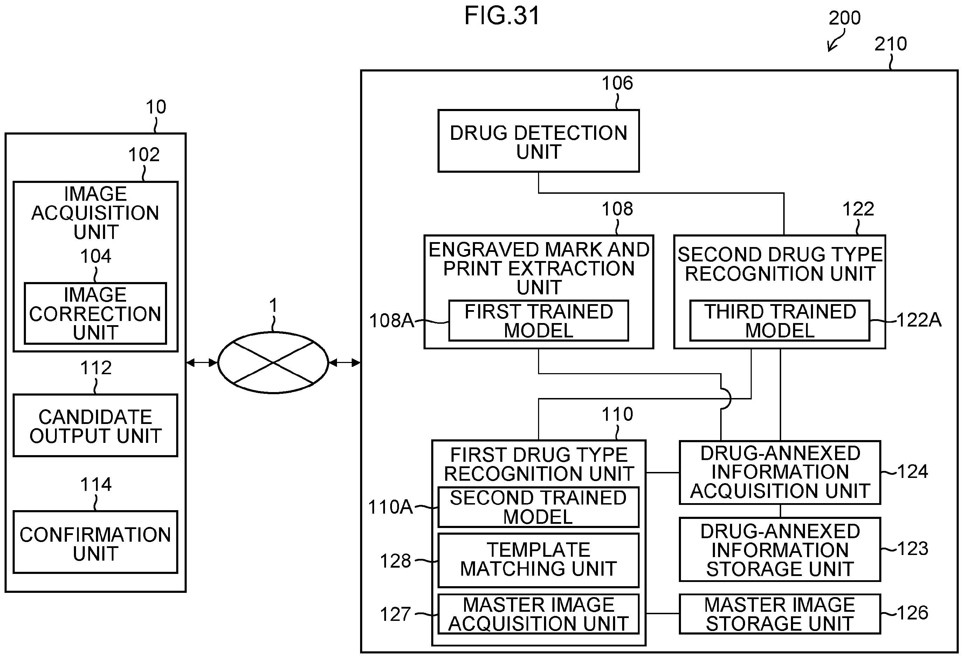

Figure 31 is a block diagram showing the functional configuration of the drug identification system 200. Here, component members in common with those of Figure 11 are designated by identical reference numerals to omit a detailed description thereof. Each function of the drug identification system 200 is implemented when the CPU 28 executes programs stored in the memory 34 in the smartphone 10, and the CPU 214 executes programs stored in the memory 216 in the server 210.

As shown in Figure 31, the drug identification system 200 includes the image acquisition unit 102, the candidate output unit 112, and the confirmation unit 114 in the smartphone 10. The drug identification system 200 also includes the drug detection unit 106, the engraved mark and print extraction unit 108, the first drug type recognition unit 110, the second drug type recognition unit 122, the drug-annexed information storage unit 123, the drug-annexed information acquisition unit 124, and the master image storage unit 126.

### [Drug Identification Method]

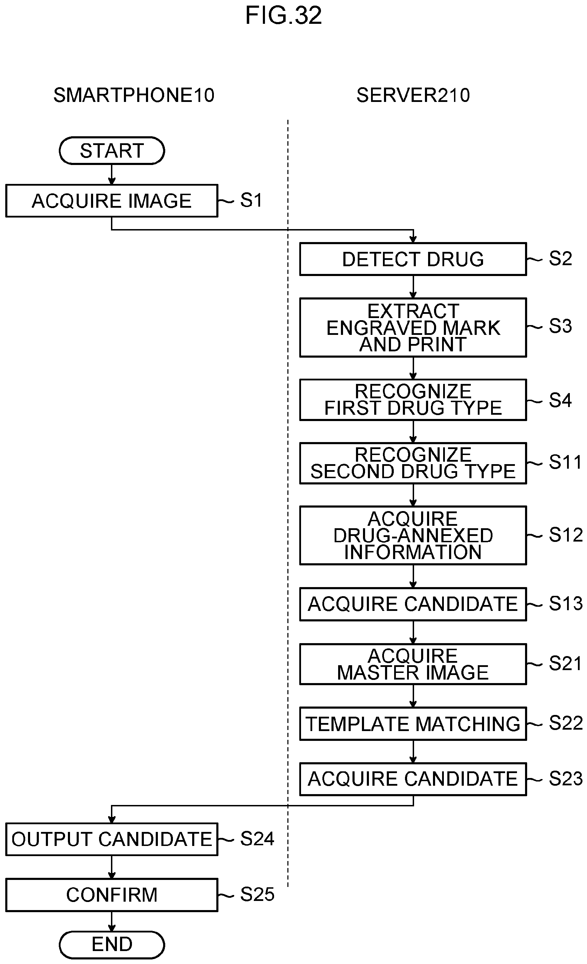

Figure 32 is a flowchart showing the steps of a drug identification method using the drug identification system 200. Here, component members in common with those of Figure 13 are designated by identical reference numerals to omit a detailed description thereof.

In step S1, the image acquisition unit 102 in the smartphone 10 acquires a captured image generated by imaging a drug to be identified that is imparted with engraved mark and/or print. The image correction unit 104 acquires a standardized image from the captured image. The smartphone 10 transmits the standardized image to the server 210. The image correction unit 104 may be in the server 210, and the standardization processing may be performed in the server 210.

In step S2, the drug detection unit 106 in the server 210 detects a region of a drug (regions of drugs) from the standardized image that is received from the smartphone 10. The server 210 performs processing of steps S3 to S23 in the same way as in the third embodiment. The server 210 transmits candidates of the drug type that are acquired in step S23 to the smartphone 10.

In step S24, the candidate output unit 112 in the smartphone 10 displays the candidates of the drug type received from the server 210 on the touch panel display 14. In step S25, the confirmation unit 114 in the smartphone 10 confirms the drug selected by the user via the touch panel display 14 as the correct drug for the tablet T1.

As described in the foregoing, the drug identification system 200 can achieve highly accurate, easy, and highly usable identification of the drug that is imparted with engraved mark and/or print through communication with the server 210 using the smartphone 10.

Here, the drug detection unit 106 is provided in the server 210, though the drug detection unit 106 may be provided in the smartphone 10. In this case, the smartphone 10 may detect the drug from the captured image, and transmits the image of a region of the detected drug (regions of drugs) to the server 210. Because only the image of the region of the drug is transmitted in this way, the amount of communication can be reduced compared to a case where the entire image is transmitted.

Although the description is omitted, the drug identification system 200 can implement all the functions of the drug identification device 100.

### [Learning Device]

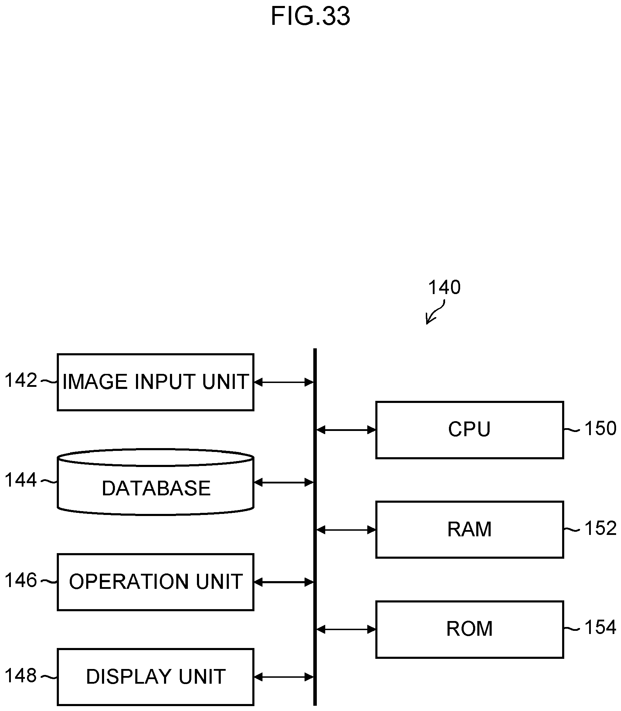

Figure 33 is a block diagram showing an electrical configuration of an image processing device 140 including a learning device. As the image processing device 140, a personal computer or a workstation may be used.

As shown in Figure 33, the image processing device 140 includes an image input unit 142, a database 144, an operation unit 146, a display unit 148, a CPU 150, a random access memory (RAM) 152, and a read only memory (ROM) 154.

The image input unit 142 is an input interface used to receive input of training data sets to be stored in the database 144, and includes both wired and wireless communication interfaces.

The database 144 is a storage unit that stores training data sets and includes a large-capacity storage device.

The operation unit 146 is a user interface for the user to control the image processing device 140, and includes a keyboard and a pointing device.

The display unit 148 is an output interface that visually displays the state of the image processing device 140 and includes a display panel.

The CPU 150 is a processor that executes commands stored in the RAM 152 and the ROM 154, and has a hardware structure similar to the CPU 28. The RAM 152 is a memory device that temporarily stores data used by the CPU 150 for various computations, and includes a semiconductor memory. The ROM 154 is a memory device that stores programs for the CPU 150 to execute, and includes a hard disk.

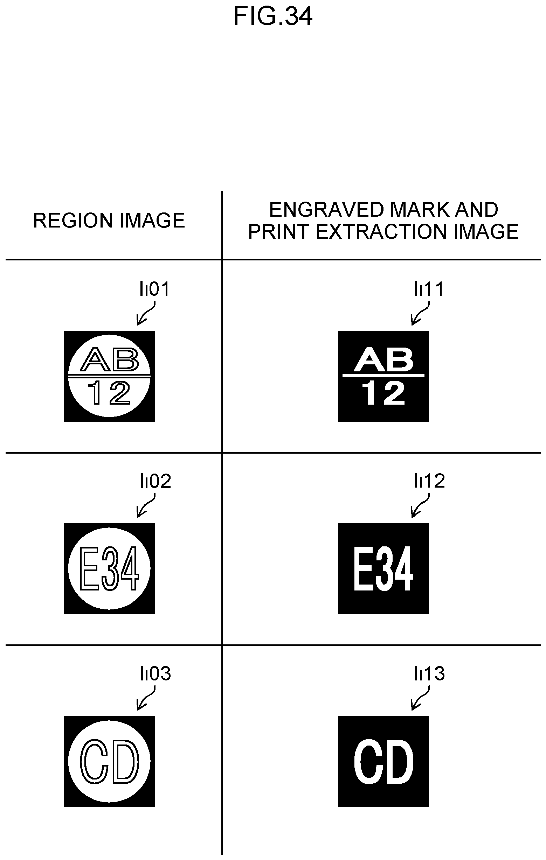

Figure 34 shows an example of training data sets for generating a first trained model. The training data sets are sets including: region images of the drugs different in type from each other; and the engraved mark and print extraction images as the ground truth data. In the example shown in Figure 34, a region image I_{I}01 and an engraved mark and print extraction image I_{I}11 that is an extracted image of the engraved mark in the region image I_{I}01 constitute one training data set. Similarly, a region image I_{I}02 and an engraved mark and print extraction image I_{I}12 that is an image of the engraved mark extracted from the region image I_{I}02 constitutes one training data set, and a region image I_{I}03 and an engraved mark and print extraction image I_{I}13 that is an image of the engraved mark extracted from the region image I_{I}03 constitutes one training data set.

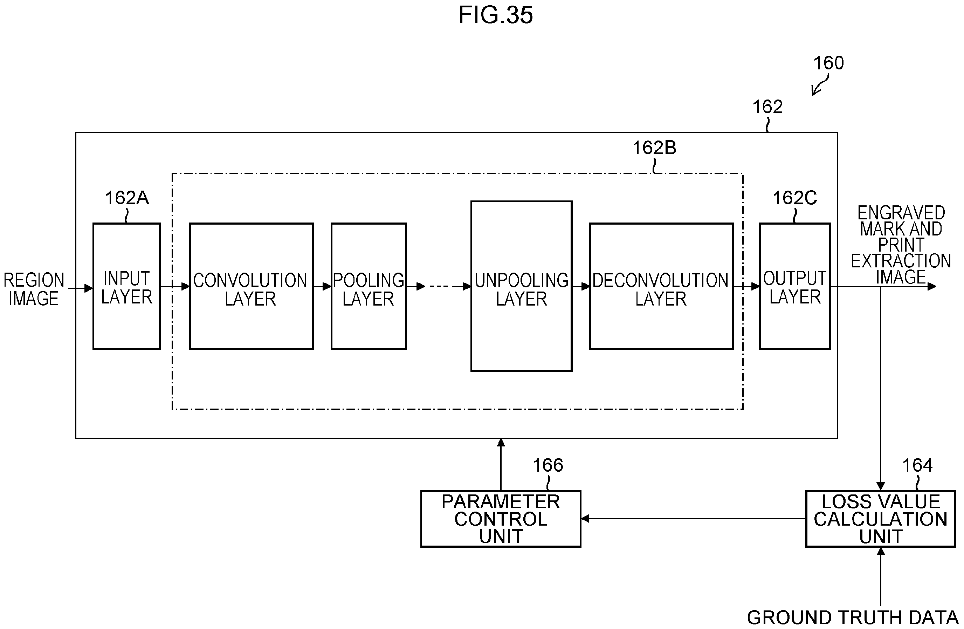

Figure 35 is a block diagram showing a functional configuration of a learning device 160 that is implemented by the image processing device 140. The learning device 160 is a device for generating the first trained model. As shown in Figure 35, the learning device 160 includes a recognizer 162, a loss value calculation unit 164, and a parameter control unit 166.

To the recognizer 162, a CNN model is applied. When the parameters of the recognizer 162 are updated from initial values to optimal values, the recognizer 162 may be changed from untrained model to a trained model. The initial values of the parameters of the recognizer 162 may be arbitrary values. Or, the parameters of an existing trained model may be applied to the initial values of the parameters of the recognizer 162, for example.

The recognizer 162 includes an input layer 162A, an intermediate layer 162B, and an output layer 162C. Each layer is structured so that nodes are connected by edges.

In a learning phase, the input layer 162A receives input of a region image of the training data set.

The first half of the intermediate layer 162B is a layer that extracts features from the region image input from the input layer 162A. The first half of the intermediate layer 162B includes multiple sets, each set including a convolution layer and a pooling layer. The convolution layer performs convolution operation using a filter on nodes in the vicinity of a previous layer to acquire a feature map. The pooling layer reduces the feature map that is output from the convolution layer to obtain a new feature map. The convolution layer plays the role of feature extraction, such as edge extraction, from an image, and the pooling layer plays the role of providing robustness so that the extracted features are not affected by parallel movement or the like. Furthermore, without being limited to the sets of the convolution layer and the pooling layer, the first half of the intermediate layer 162B may include a series of convolution layers, or may include a normalization layer.

The latter half of the intermediate layer 162B is a layer that restores the region image by enhancing the resolution of the extracted feature map. The latter half of the intermediate layer 162B includes multiple sets, each set including an unpooling layer and a deconvolution layer. The unpooling layer enlarges the feature map to obtain a new feature map. The deconvolution layer performs a deconvolution operation to restore the amount of features (feature amount) included in the feature map. The deconvolution layer at the last stage outputs an engraved mark and print extraction image with the same size as the region image. Here, the latter half of the intermediate layer 162B includes the unpooling layer and the deconvolution layer, as one set. However, without being limited to this example, the latter half of the intermediate layer 162B may include a series of deconvolution layers, or may include a normalization layer.

The output layer 162C is a layer that outputs the engraved mark and print extraction image that is a recognition result of the recognizer 162.

The loss value calculation unit 164 acquires the engraved mark and print extraction image output from the output layer 162C in the recognizer 162, and the engraved mark and print extraction image that is a training data set of the region image input into the input layer 162A, and calculates a loss value between the two images. The loss value may be calculated by, for example, using Jacquard coefficients or dice coefficients.

Based on the loss value calculated by the loss value calculation unit 164, the parameter control unit 166 adjusts the parameters (coefficients of filters in the convolution layer and the deconvolution layer, etc.) of the recognizer 162 by using error reverse propagation so as to minimize the distance in feature space between the engraved mark and print extraction image output from the output layer 162C of the recognizer 162 and the engraved mark and print extraction image of the ground truth data, or to maximize the similarity.

The adjustment processing of the parameters is repeated, and learning is repeatedly performed until the loss value calculated by the loss value calculation unit 164 converges.

The training data sets stored in the database 144 in this way are used to generate the learned recognizer 162 with optimized parameters as the first trained model. Here, learning may be performed by artificially adding moderate noise to the region image and the engraved mark and print extraction image of the drug. This makes it possible to generate the recognizer 162 that has acquired robustness to fluctuations in the imaging environments.

In a recognition phase, the recognizer 162 that is the first trained model outputs, from the region image of any drug to be input, the engraved mark and print extraction image of the drug.

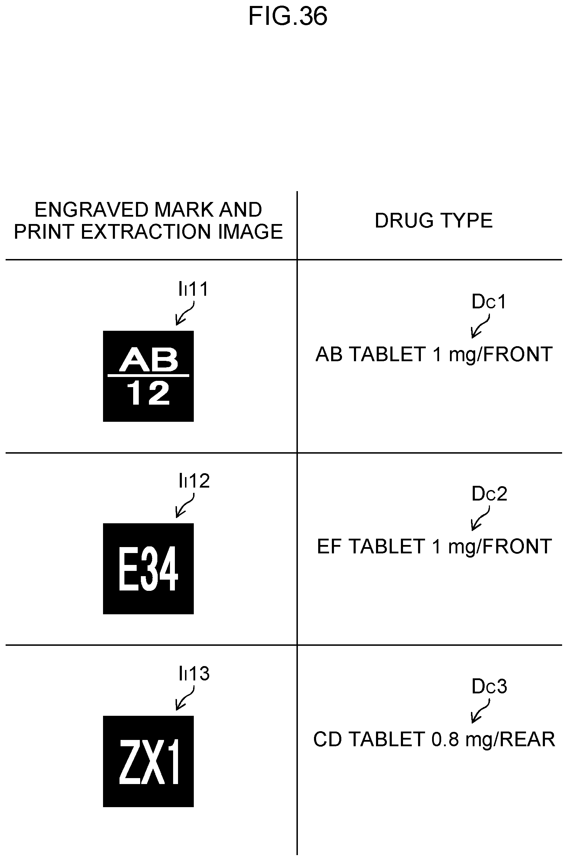

Figure 36 shows an example of training data sets for generating a second trained model. The training data sets include: the engraved mark and print extraction images of the drugs different in type from each other; and ground truth data of the types of the drugs and information on whether the engraved mark and print extraction images are of the front surface or the rear surfaces, as sets. In the example shown in Figure 36, an engraved mark and print extraction image I_{I}11, a drug type D_{C}1 of the drug of the engraved mark and print extraction image I_{I}11, and information indicating that the an engraved mark and print extraction image I_{I}11 is the image of the front surface, constitute one training data set. Similarly, an engraved mark and print extraction image I_{I}12, a drug type Dc2 of the drug of the engraved mark and print extraction image I_{I}12, and information indicating that the engraved mark and print extraction image I_{I}12 is the image of the front surface, constitute one training data set. An engraved mark and print extraction image I_{I}13, a drug type D_{C}3 of the drug of the engraved mark and print extraction image I_{I}13, and information indicating that the engraved mark and print extraction image I_{I}13 is the image of the rear surface, constitute one training data set.

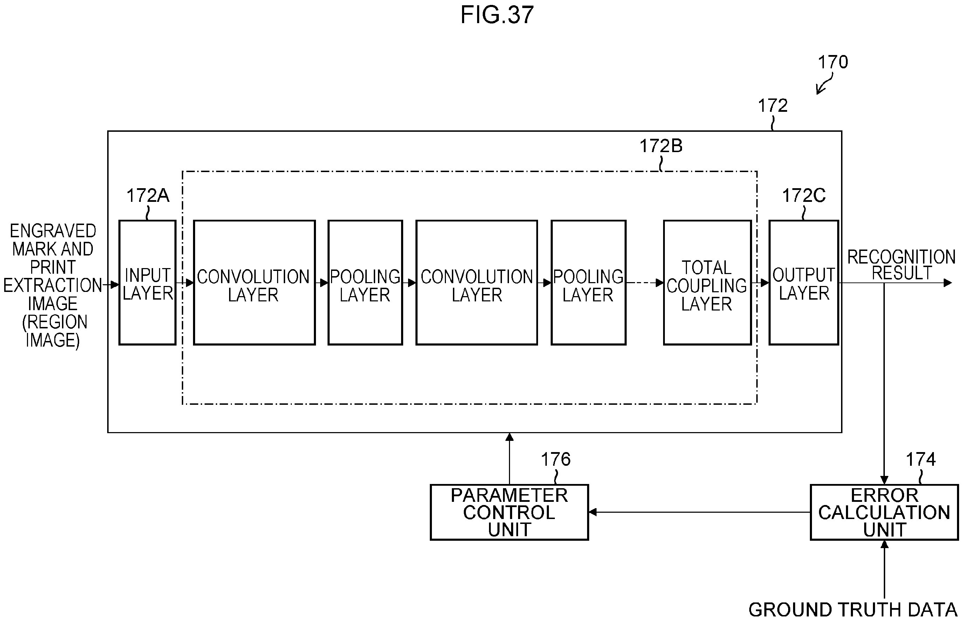

Figure 37 is a block diagram showing a functional configuration of a learning device 170 implemented by the image processing device 140. The learning device 170 is a device for generating the second trained model. As shown in Figure 37, the learning device 170 includes a recognizer 172, an error calculation unit 174, and a parameter control unit 176.

To the recognizer 172, a CNN model is applied. When the parameters of the recognizer 172 are updated from initial values to optimal values, the recognizer 172 can be changed from untrained model to a trained model. The initial values of the parameters of the recognizer 172 may be arbitrary values. Or, the parameters of an existing trained model may be applied to the initial values of the parameters of the recognizer 172, for example.

The recognizer 172 includes an input layer 172A, an intermediate layer 172B, and an output layer 172C. Each layer is structured so that nodes are connected by edges.

In a learning phase, the input layer 172A receives input of the engraved mark and print extraction image of a training data set.

The intermediate layer 172B is a layer that extracts features from the engraved mark and print extraction image input from the input layer 172A. The intermediate layer 172B includes: multiple sets, each set including a convolution layer and a pooling layer; and a total coupling layer. The total coupling layer couples all the nodes in the previous layer (in this case, the pooling layer).

The output layer 172C is a layer that outputs the drug type of the drug that is a recognition result of the recognizer 172.

The error calculation unit 174 acquires the recognition result output from the output layer 172C in the recognizer 172, and the drug type of the drug that is the training data set of the engraved mark and print extraction image input into the input layer 172A, and calculates error therebetween. The error may be calculated by, for example, softmax cross entropy or mean squared error (MSE).

The parameter control unit 176 adjusts the parameters (coefficients of the filters of the convolution layer and the deconvolution layer, etc.) of the recognizer 172 by the error back propagation based on the error calculated by the error calculation unit 174.

The adjustment processing of the parameters is repeated, and learning is performed repeatedly until the difference between the output of the recognizer 172 and the ground truth data becomes small.

The training data sets stored in the database 144 in this way are used to generate the learned recognizer 172 with optimized parameters as the second trained model. Here, learning may be performed by artificially adding moderate noise to the engraved mark and print extraction image of the drug. This makes it possible to generate the second trained model that has acquired robustness to fluctuations in the imaging environments.

Here, the engraved mark and print extraction image on one surface (one side) of the drug is used as an input image. Instead, a set of the engraved mark and print extraction images of both the surfaces may be used as the input images. In this case, since the amount of information is larger than in the case of only one surface, an enhanced accuracy can be expected, though the usability may be lowered due to the necessity of continuously imaging both the surfaces.

In a recognition phase, the recognizer 172 that is the second trained model outputs, from the region image of any drug to be input, the engraved mark and print extraction image of the drug.

The image input unit 142 (an example of the training data collection unit) may collect retraining data sets in the database 144, the retraining data sets, using the engraved mark and print extraction images acquired in the first embodiment to the third embodiment as input images and using the correct drugs confirmed in the first embodiment to the third embodiment as ground truth data. In other words, when the recognition by the first drug type recognition unit 110 is proper, the proper correct drug becomes ground truth data, and when the recognition by the first drug type recognition unit 110 is not proper, the correct drug confirmed afterwards becomes ground truth data. The learning device 170 (an example of the relearning unit) may perform relearning of the second trained model 110A by using the collected training data sets.

The image input unit 142 may also collect retraining data sets of manually generated engraved mark and print extraction images and candidates of the drug types of the drugs in the database 144. The learning device 170 may also perform relearning of the second trained model 110A by using the manually generated training data sets.

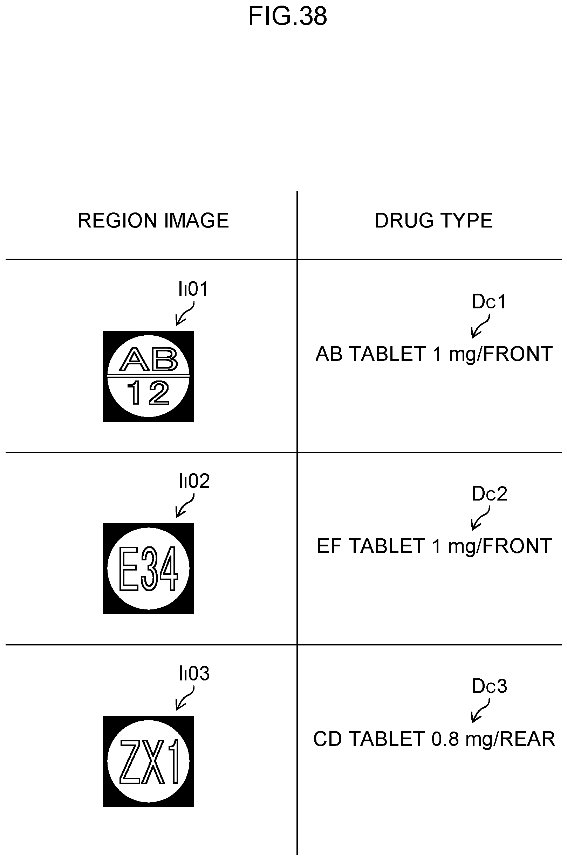

Figure 38 shows an example of training data sets for generating a third trained model. The training data sets are sets of the region images of the drugs different in type from each other, the drug types of the drugs as ground truth data, and information on the front or rear surfaces of the region images. In the example shown in Figure 38, a region image I_{I}01, a drug type D_{C}1 of the region image I_{I}01, and information indicating that a region image I_{I}01 is the image of the front surface, constitute one training data set. Similarly, a region image I_{I}02, a drug type D_{C}2 of the region image I_{I}02, and information indicating that region image I_{I}02 is the image of the front surface, constitute one training data set, and a region image I_{I}03, a drug type D_{C}3 of the region image I_{I}03, and information indicating that region image I_{I}03 is the image of the rear surface, constitute one training data set.

The learning device 170 performs learning of the training data sets to generate the third trained model 122A. Here, learning may be performed by artificially adding moderate noise to the region image of the drug. This makes it possible to generate the third trained model 122A that has acquired robustness to fluctuations in the imaging environments.

The training data sets for learning of the first trained model 108A, the second trained model 110A, the third trained model 122A may be acquired from an existing one-dose packaging audit support system (unit-dose packaging audit support system), for example.

In the one-dose packaging audit support system, imaging with fixed distance and viewpoint is performed in order to perform highly accurate drug recognition. The partial illumination images that are irradiated with light in different ways and total illumination images are image-processed and composed, and then engraved mark and print extraction processing is performed. For this reason, a large number of engraved mark and print extraction images suitable as the training data are accumulated.

On the other hand, in the present embodiment, a standardized image is generated by the image correction unit 104 and then the region image and the engraved mark and print extraction image are generated. Therefore, the images derived from the one-dose packaging audit support system may be used as they are by applying magnification and reduction conversion so as to match actual sizes on the standardized image and the actual sizes on the image acquired from the one-dose packaging audit support system.

Thus, appropriate learning may be performed by using the total illumination images, the engraved mark and print extraction images, the composite extraction images, and corresponding drug identification information, which are collected through the one-dose packaging audit support system.

The image input unit 142 (an example of the training data collection unit) may collect retraining data sets in the database 144, the retraining data sets using the region images that are acquired in the first embodiment to the third embodiment as input images, and using the correct drugs confirmed in the first embodiment to the third embodiment as ground truth data. The learning device 170 (an example of the relearning unit) may perform relearning of the third trained model 122A by using the collected training data sets.

The image input unit 142 may also collect retraining data sets of manually generated region images and candidates of the drug types of the drugs in the database 144. The learning device 170 may also perform relearning of the third trained model 122A by using the manually generated training data sets.

Furthermore, the image input unit 142 may collect retraining data sets in the database 144, the retraining data sets using the region images acquired in the first embodiment to the third embodiment as input images, and using the correct drugs confirmed in the first embodiment to the third embodiment as ground truth data. The learning device 170 (an example of the relearning unit) may perform learning of a new fourth trained model by using the collected training data sets. The fourth trained model learned in this way may be used in the second drug type recognition unit 122 in place of the third trained model 122A.

### <Fourth Embodiment>

In the case of a drug search based on an actual drug to be identified, generally, the user visually reads identification characters and identification symbols written on the print and/or engraved mark of the drug, inputs text information into a search system, and makes an inquiry to an identification character and symbol database. An actual drug is small and hard to see by visual observation. In addition, it is difficult to distinguish identification characters on engraved mark-type drugs in particular. When the search system is a personal computer, input is performed using a keyboard while viewing the screen of the personal computer. This causes movement of the eyes among the actual drug, the screen of the personal computer, and the keyboard or the like, and movement of the hands or the like, so that usability is compromised. Therefore, in the fourth embodiment, a drug search with improved usability is implemented by using a mobile terminal device.

A mobile terminal device, such as a smartphone, typically includes a camera, and can easily enlarge and display an image by zooming at the time of imaging. In a case where drugs to be identified are engraved mark-type drugs, enlarged display alone may not be enough to solve the difficulty of reading, and therefore the image is subjected to engraved mark emphasis processing using AI (Artificial Intelligence) and is presented to the user. The AI includes an engraved mark emphasis AI that estimates an engraved mark emphasized image from a total illumination captured image, and an engraved mark identification AI that performs drug identification information from the engraved mark emphasized image alone. These AIs may be AIs that have acquired robustness to fluctuations in the imaging environments by receiving moderate noise that is artificially added to the AIs during learning. Since the engraved mark emphasis AI learns from teacher images obtained under certain conditions, it is desirable to infer under the conditions as close as possible to the certain conditions. This can be achieved by standardizing the viewpoint and the distance using ArUco markers, etc., and bringing the conditions closer to the conditions under which the teacher images have been obtained. Moreover, by arranging four ArUco markers on a mount and placing drugs inside the markers, it is possible to stabilize the processing to specify and cut out a drug placement range as the image processing.

Hereinafter, the details of a drug identification device according to the fourth embodiment will be described.

### [Functional Configuration of Drug Identification Device]

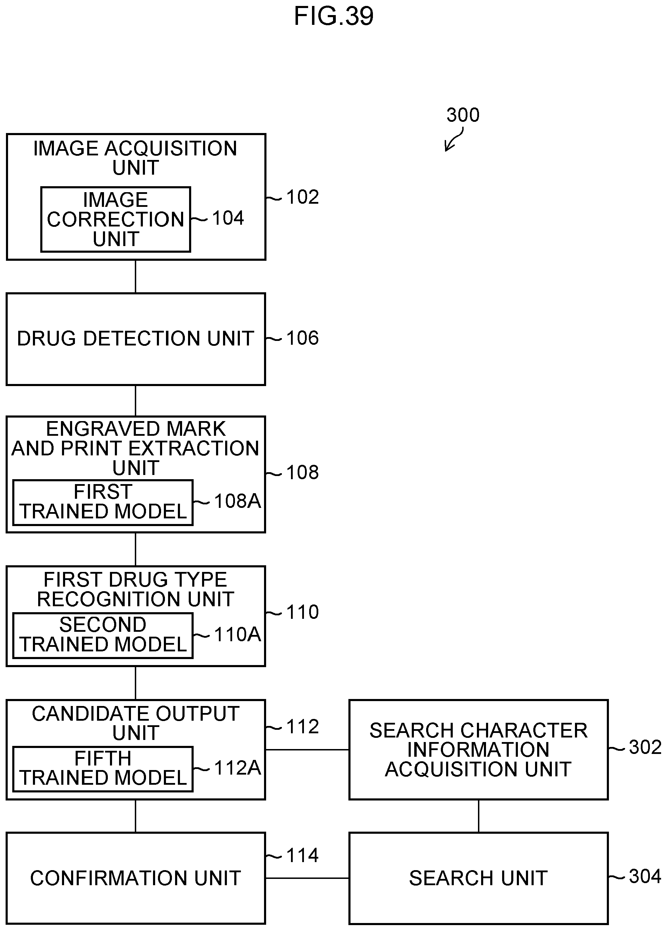

Figure 39 is a block diagram showing the functional configuration of a drug identification device 300 implemented by the smartphone 10 (see Figure 10). Here, component members in common with those of Figure 5 are designated by identical reference numerals to omit a detailed description thereof. As shown in Figure 39, the drug identification device 300 includes a search character information acquisition unit 302 and a search unit 304.

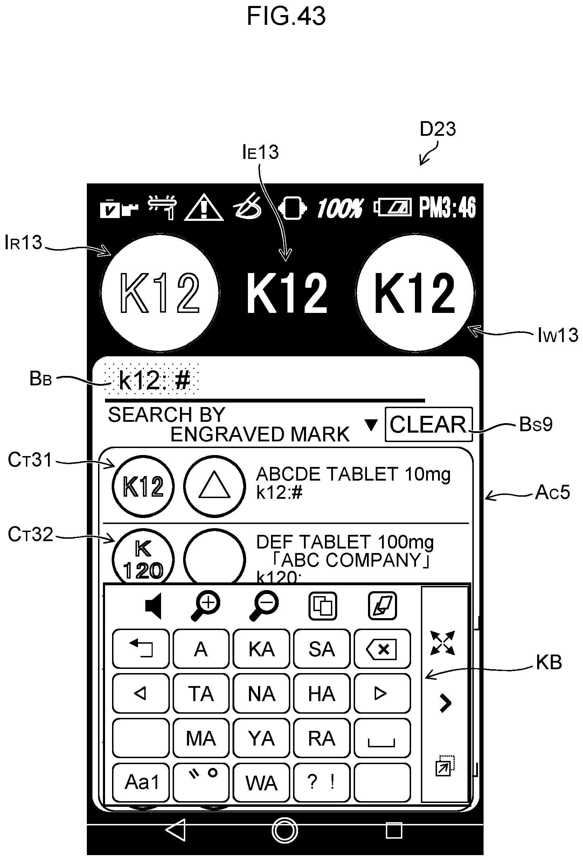

The search character information acquisition unit 302 acquires character information of the drug type of the most promising candidate, out of the candidates of the drug type of the drug to be identified that are output from the candidate output unit 112. The most promising candidate out of the candidates of the drug type of the drug to be identified is, for example, the drug type of the candidate with a highest score among the candidates of the drug type of the drug to be identified. The search character information acquisition unit 302 specifies the drug type of the most promising candidate by using a drug recognition AI such as the third trained model 122A, or by combining the drug recognition AI with template matching, and acquires the engraved mark and print information of the specified drug type as character information from an unshown engraved mark and print database. The candidate output unit 112 inputs the character information of the drug type of the most promising candidate, which is acquired by the search character information acquisition unit 302, into the text box B_{B} (see Figure 43) and displays.

Based on the character information input into the text box B_{B}, the search unit 304 retrieves candidates of the drug type of the drug to be identified from an unshown drug database. This unshown drug database needs to be a database having the same drug identification keys as the unshown engraved mark and print database described above. The engraved mark and print database and the drug database may be a single database. The candidates of the drug type of the drug to be identified that are searched by the search unit 304 are selectably displayed on the touch panel display 14 (see Figure 1) in the candidate output unit 112.

The candidate output unit 112 also makes the direction of the engraved mark and/or print upright (aligns an up-down direction) in the image of the drug that is displayed on the touch panel display 14. The candidate output unit 112 includes a fifth trained model 112A. The fifth trained model 112A is a trained model that outputs, upon receiving input of a first image of the drug that is imparted with the engraved mark and/or print, a third image with the direction of the engraved mark and/or print of the drug being made upright. The fifth trained model 112A is machine-learned based on training data sets of different drugs that are imparted with engraved mark and/or print, the training data sets including: images of the drugs imparted with the engraved mark and/or print in an arbitrary direction; and images of the drugs with the direction of the engraved mark and/or print of the drugs being made upright, as sets for learning. To the fifth trained model 112A, as in the case of the first trained model 108A, the CNN may be applied.

The fifth trained model 112A may be a trained model that outputs, upon receiving input of a first image of the drug that is imparted with the engraved mark and/or print, an actual value of a rotation angle used for further rotating the drug from a current rotation angle in order to make the direction of the engraved mark and/or print of the drug upright. In this case, the fifth trained model 112A is machine-learned using training data sets of different drugs that are imparted with engraved mark and/or print. Here, the training data sets include: images of the drugs imparted with the engraved mark and/or print in an arbitrary direction; and rotation angles necessary for making the direction of the engraved mark and/or print of the drugs upright from the current direction, as sets for learning. To the fifth trained model 112A, as in the case of the first trained model 108A, the CNN may be applied.

The candidate output unit 112 may make the direction of the engraved mark and/or print of the image of the drug to be displayed on the touch panel display 14 upright by collating the rotation direction through template matching with master images.

### [Drug Identification Method]

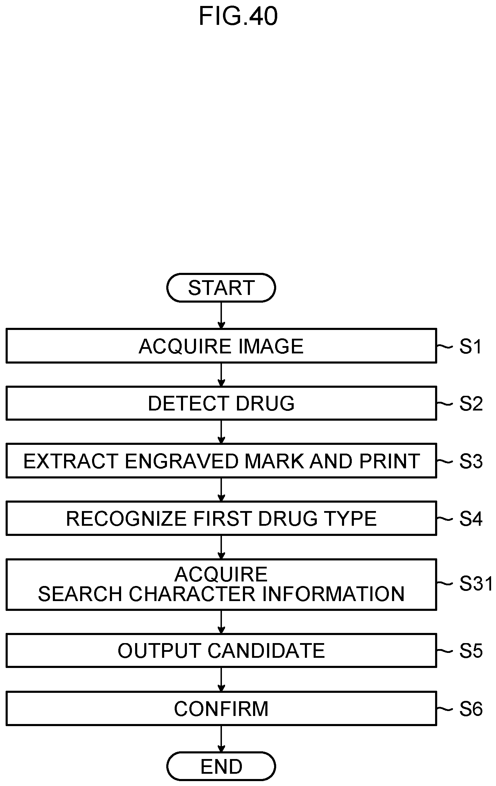

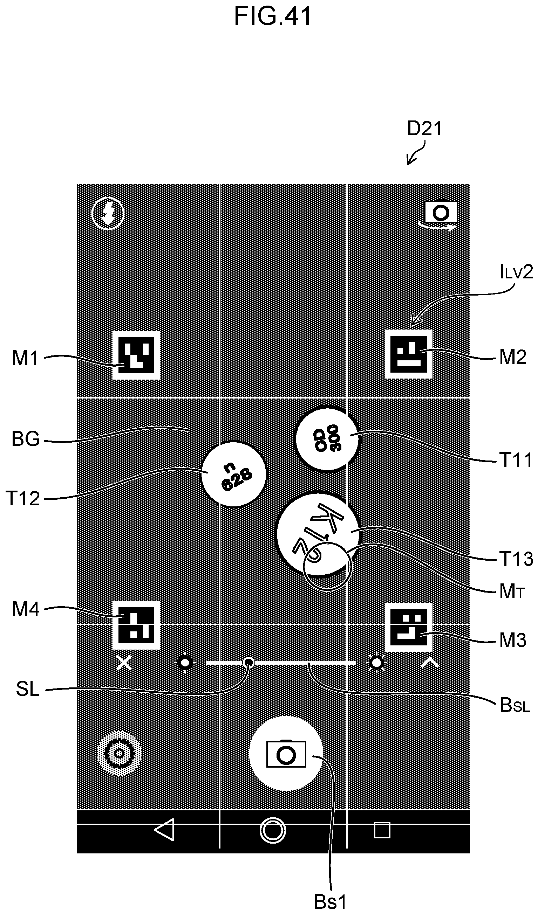

Figure 40 is a flowchart showing the steps of a drug identification method using the drug identification device 300. Here, component members in common with those of the Figure 6 are designated by identical reference numerals to omit a detailed description thereof. Figures 41 to 45 respectively show screen displays D21 to D25 on the touch panel display 14 of the smartphone 10 in each step in the drug identification method.

In step S1, the image acquisition unit 102 acquires a captured image generated by imaging drugs to be identified that are imparted with engraved mark and/or print. Figure 41 shows the screen display D21 in a case where drugs to be identified are imaged in step S1. The screen display D21 displays a live view image I_{LV}2 of objects to be imaged, a tap position marker M_{T}, a slider bar B_{SL} for brightness correction, and a shooting button B_{S}1.

The live view image I_{L}V2 displays a moving image captured by the out-camera 22 in real time. The live view image I_{LV}2 includes tablets T11, T12, and T13, markers M1 to M4, and a background BG in the reference gray color.

In a case where a tablet portion in the live view image I_{LV}2 is tapped, the focal length of the camera is adjusted to focus on that portion. The tap position marker M_{T} indicates the position on the touch panel display 14 when tapping is performed to focus on the tablet portion. The slider bar B_{SL} for brightness correction, which is not displayed until then, is displayed as tapping as a trigger. The slider bar B_{SL} for brightness correction adjusts the brightness of the captured image. The slider bar B_{SL} for brightness correction includes a slider SL that can be moved by dragging on the touch panel display 14 by the user. In a case where the slider SL is moved to the left in Figure 41, the captured image is corrected to be darker, whereas in a case where the slider SL is moved to the right, the image is corrected to be brighter. By changing the position of the slider SL, the user may adjust the brightness of the captured image to the brightness that allows easy identification of the engraved mark and/or print on the drug to be identified.

In a case where the shooting button BS1 is tapped by the user on the screen display D21, actual shooting is performed by the out-camera 22, and the processing shifts to step S2. The captured image that is a still image generated by the actual shooting is stored in the memory 34 (see Figure 3).

In step S2, the drug detection unit 106 detects the respective regions of the tablets T11 to T13 from the captured image. In step S3, the engraved mark and print extraction unit 108 further processes the respective regions of the tablets T11 to T13 that are detected in step S2, and acquires engraved mark and print extraction images that are extracted images of the respective engraved mark and/or print of the tablets T11 to T13. Here, the engraved mark and print extraction unit 108 further generates composite extraction images by composing the images of the regions of the tablets T11 to T13 with the respective engraved mark and print extraction images with their luminance being inverted.

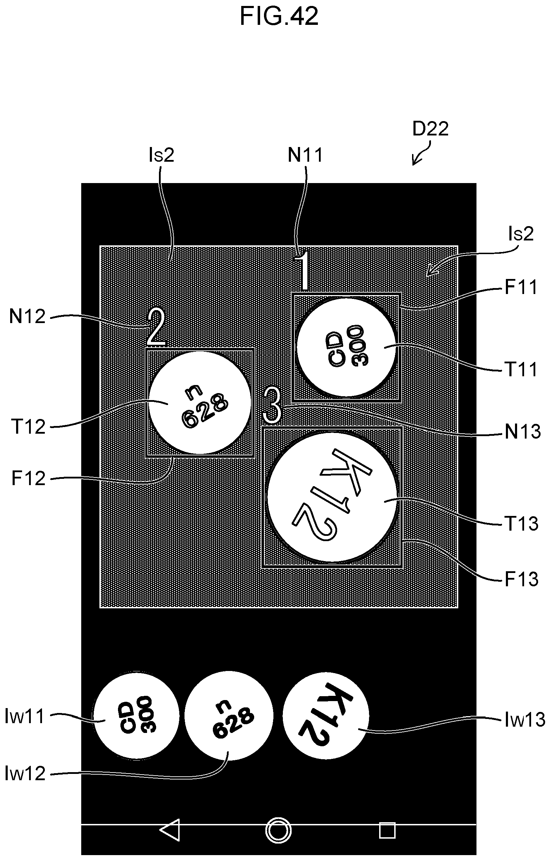

Figure 42 shows the screen display D22 in step S2. The screen display D22 displays the standardized image Is2 of the captured image that is generated by actual shooting in step S 1. The standardized image I_{S}2 is an image generated by standardizing the captured image and then cutting out a region enclosed with a straight line that connects the markers M11 to M14. Here, frames F11, F12, and F13 that enclose the regions of the tablets T11, T12, and T13, respectively, and numbers N11, N12, and N13 corresponding to the frames F11, F12, and F13, are superimposed on the standardized image Is2 and displayed. Here the number N11 is "1", the number N12 is "2", and the number N13 is "3".

Furthermore, below the standardized image Is2 on the screen display D22, composite extraction images I_{W}11, I_{W}12, and I_{W}13 corresponding to the tablets T11, T12 and T13 are displayed, respectively. The composite extraction image I_{W}11, which corresponds to the image of the drug to be identified with the engraved mark and/or print being emphasized, is an image generated by inverting the luminance of the engraved mark and print extraction image of the tablet T11 and superimposing the inverted image on the region image of the tablet T11. The engraved mark and print extraction image is an image generated by performing engraved mark and print extraction processing on the region image. The engraved mark and print extraction processing is processing for expressing the engraved mark portion or the print portion with relatively higher in luminance than portions other than the engraved mark portion and the print portion.

Similarly, the composite extraction image I_{W}12 is an image generated by inverting the luminance of the engraved mark and print extraction image of the tablet T12 and superimposing the inverted image on the region image of the tablet T12, and the composite extraction image I_{W}13 is an image generated by inverting the luminance of the engraved mark and print extraction image of the tablet T13 and superimposing the inverted image on the region image of the tablet T13. The composite extraction images Iw11, I_{W}12, and I_{W}13 are arranged and displayed left to light in the order of the values indicated by the numbers N11, N12, and N13.

In the screen display D22, in a case where any one of the regions of the tablets T11 to T13 (any of the regions enclosed with the frames F11 to F13) in the standardized image I_{S}2, or any one of the composite extraction images I_{W}11, I_{W}12, and I_{W}13, is selected by tapping on the touch panel display 14 by the user, the processing shifts to step S4.

In step S4, the first drug type recognition unit 110 acquires candidates of the drug type of the drug, from the engraved mark and print extraction image of the drug selected in step S3.

Next, in step S31 that is the search character information acquisition step, the search character information acquisition unit 302 acquires the character information of the most promising candidate, out of the candidates of the drug type of the drug to be identified that are acquired in step S4.

Figure 43 shows the screen display D23 in step S31. Shown here is the case where the region of the tablet T13 (the region enclosed with the frame F13) on the standardized image I_{S}2 is selected in the screen display D22.

In the upper part of the screen display D23, the region image I_{R}13 of the tablet T13, the engraved mark and print extraction image I_{E}13 of the region image I_{R}13 and the composite extraction image I_{W}13 of the tablet T13 are displayed side by side in the lateral direction. Note that at least one of the region image I_{R}13, the engraved mark and print extraction image I_{E}13, and the composite extraction image I_{W}13 may be displayed.

Here, the candidate output unit 112 uses the fifth trained model 112A to make the direction of the characters in the engraved mark and/or print upright, in the region image I_{R}13, the engraved mark and print extraction image I_{E}13, and the composite extraction image I_{W}13, respectively (an example of the display control step). As a result, in the screen display D23, the region image I_{R}13, the engraved mark and print extraction image I_{E}13, and the composite extraction image I_{W}13 are displayed with the direction of the characters in the respective engraved mark and/or print being made upright.

The candidate output unit 112 may make each image upright by collation of the rotation direction between each image to be made upright and each corresponding master image. The direction of the characters in the respective engraved mark and/or print in the region image I_{R}13, the engraved mark and print extraction image I_{E}13, and the composite extraction image I_{W}13 may be changeable by rotating the touch panel display 14 by the user in the screen display D23.

In addition, the screen display D23 displays the text box B_{B}, the search button B_{S}3, a clear button B_{S}9, and a software keyboard KB.

The text box B_{B} is a search window that allows the user to input a character string and is also an interface for specifying the drug type of the tablet T13 by the input character string. Here, the character string is not limited to a string constituted of characters. The character string may include only one character.

The user can input a character string into the text box B_{B} and modify the character string using the software keyboard KB or by voice input with the microphone 18 (see Figure 1). The user can obtain candidates of the drug type corresponding to the input character string, by inputting, as text, the identification symbol of the engraved mark and/or print of the tablet T13 while referring to the region image I_{R}13, the engraved mark and print extraction image I_{E}13, and the composite extraction image Iw13 of the tablet T13 in the screen display D23, and tapping the search button B S3. The user can also delete the character string input into the text box B_{B} by tapping the clear button Bs9.

Here, character information of the drug type of the most promising candidate acquired in step S31 is automatically input into the text box B_{B} in the search character information acquisition unit 302. In the example shown in Figure 43, character string "k12: #" is automatically input into the text box B_{B}. In the character string "k12: #", a section before ":" represents the engraved mark and/or print on the front-surface, that is, characters of "k12" here. In the character string "k12:#", a section after ":" represents the engraved mark and/or print on the rear-surface, and "#" indicates a symbol here. The character string that is automatically input can be modified using the software keyboard KB or the microphone 18. The character information on the drug type of the most promising candidate is input in advance, and therefore even if AI erroneously presents a drug similar in a making character as the most promising candidate to the user, the user can slightly correct the character string to input character information on the correct drug, so that high usability can be achieved while minimizing the labor of the user. The character string input into the text box B_{B} may also be deleted by the clear button B_{S}9.

The screen display D23 further includes a candidate drug display region Ac5. In the candidate drug display region A_{C}5, candidates of the drug type of the drug that is the tablet T13 are selectably displayed in the order of higher scores from top to bottom, the scores being calculated based on a matching degree between the input character string and the engraved mark character string on an engraved mark character database.

In a case where the user taps the search button B_{S}3 in the screen display D23, the processing shifts to step S5.

In step S5, the candidate output unit 112 acquires and outputs the candidates of the drug type having engraved mark and/or print, based on the character string input into the text box B_{B} when the search button Bs3 is tapped. The search method for acquiring drug type candidates based on an input character string may be any of the methods including full match search, prefix search, suffix search, partial match search, ambiguous search, and a search method that scores the matching degree of character strings according to a specific algorithm. The user may also designate a desired search method to apply, out of the choices including these search methods.

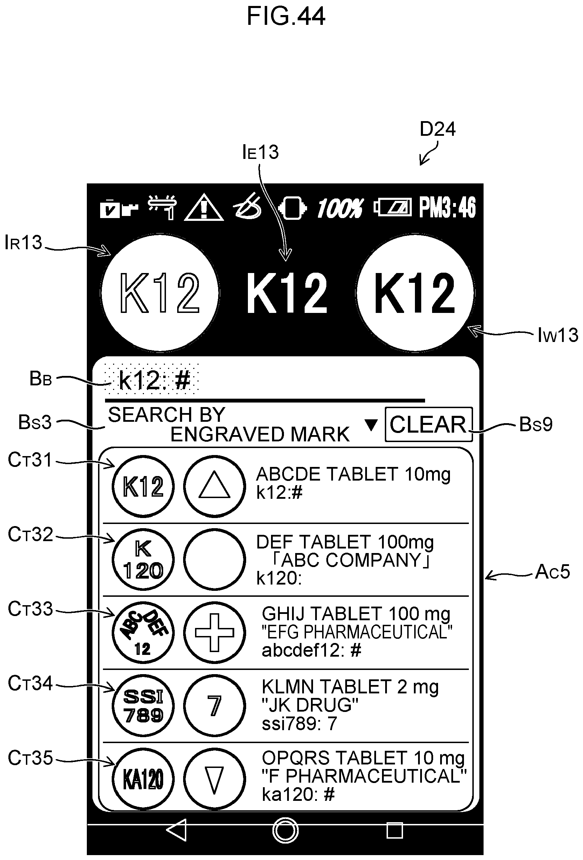

Figure 44 shows the screen display D24 in step S5. Described here is the case where the search button B_{S}3 is tapped while a character string "k12: #" is input into the text box B_{B} in the screen display D23.

In the upper part of the screen display D24, the region image I_{R}13 of the tablet T13, the engraved mark and print extraction image I_{E}13 of the region image I_{R}13, and the composite extraction image I_{W}13 of the tablet T13 are displayed side by side in the lateral direction, successively from the screen display D23.

In the screen display D24, the software keyboard KB is not shown, and in the candidate drug display region A_{C}5, candidate drugs having a character string of the engraved mark and/or print that is close to the character string "k12: #" are selectably displayed in the order of higher scores from top to bottom, the scores indicating the matching degree of the engraved mark character string. Here, the candidate drug display region Ac5 displays front-surface and rear-surface master images of the respective candidate drugs C_{T}11 to C_{T}15 of the tablet T13, and drug information thereof. The drug information includes the name of the drug and the character information on the engraved mark and/or print. In the candidate drug display region Ac5, the candidate drugs with lower scores are displayed in response to swiping from the lower side toward the upper side of the screen by the user.

Thus, the screen display D24 displays the region image I_{R}13, the engraved mark and print extraction image I_{E}13, and the composite extraction image Iw13 of the tablet T13, together with the front-surface image and rear-surface master image of the respective candidate drugs C_{T}31 to C_{T}35 of the tablet T13. Therefore, it becomes easier for the user to compare these images and select the correct drug for the tablet T13.

In the screen display D24, in a case where the user selects one drug out of the candidate drugs displayed in the candidate drug display region Ac5, the processing shifts to step S6.

In step S6, the confirmation unit 114 confirms the drug selected by the user as the correct drug.

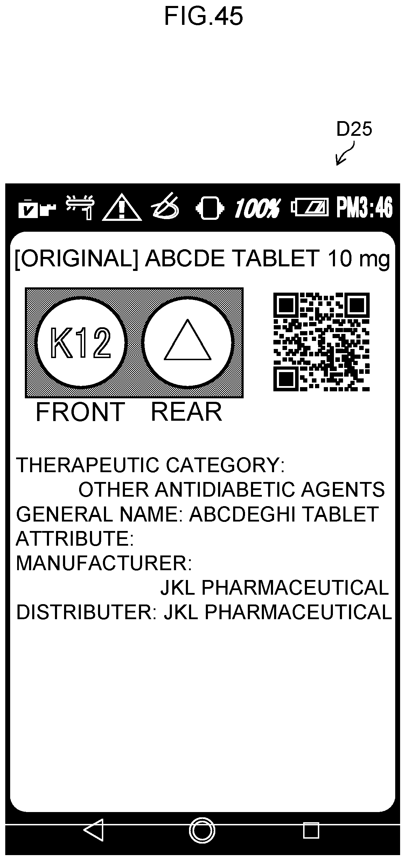

Figure 45 shows the screen display D25 in step S6. Here, the case where the candidate drug C_{T}31 is selected in the screen display D24 is shown.

The screen display D25 displays, the name of the candidate drug C_{T}31, distinction of original/generic, the front-surface master image and the rear-surface master image, a two-dimensional barcode representing identification code, a therapeutic category, a generic name, attribute, a manufacturer, and a vendor information as detailed information about the candidate drug C_{T}31. The user may check these information on the selected drug.

According to the fourth embodiment, drug search with high usability can be implemented as follow.

In a case where the user visually reads the identification symbol in the engraved mark and/or print of a drug and makes a search by text input or voice input, the character information extracted from the captured image is automatically input into the text box, so that the correct drug for the drug to be identified can be swiftly determined.

The drug search task can be completed in a single mobile terminal, so that large eye movement and hand changes or the like become unnecessary. It is also possible to provide the drug image that is displayed as an enlarged image, the engraved mark emphasized image provided by the engraved mark emphasis AI, and the overlap image of both the images, so that the user can recognize the identification characters and the identification symbols with improved visibility.

In addition, because the drugs to be searched can be imaged at once, it is possible to reduce the user's the time and effort for imaging (shooting).

### <Fifth Embodiment>

In the case of identifying a drug (brought-in drug) or drugs brought in by a patient who is admitted to a hospital or identifying drugs remaining at home, it is important not to confuse the identification of a target patient and association between the patient and the drugs to be discriminated. For this reason, in the flow of a sequence of identification tasks, it is important to specify an individual, identify drugs owned by the individual, correctly associate the patient with the drugs to be discriminated, and then ensure data transmission to a management system or the like.

For example, in a case where a patient carrying his/her own brought-in drugs is admitted to a hospital, patient information is input into a management system such as an electronic medical chart system of the hospital, a brought-in drug discrimination request sheet including a patient name and patient identification(ID) or the like is issued, and a set of the brought-in drug discrimination request sheet and the brought-in drugs is handed over to a discriminator, such as a pharmacist. The discriminator discriminates the brought-in drugs, and inputs a discrimination result to the management system such as an electronic medical chart. Then, the brought-in drug discrimination request sheet is signed (or stamped or sealed) to inform that the discriminator has confirmed, and the brought-in drug discrimination request sheet and the discriminated drugs are returned to a requester, in the processing flow.

In a fifth embodiment, there are provided a brought-in drug discrimination request sheet and a drug identification system for identifying a patient and reliably discriminating a brought-in drug along this flow.

### [Configuration of Drug Identification System]

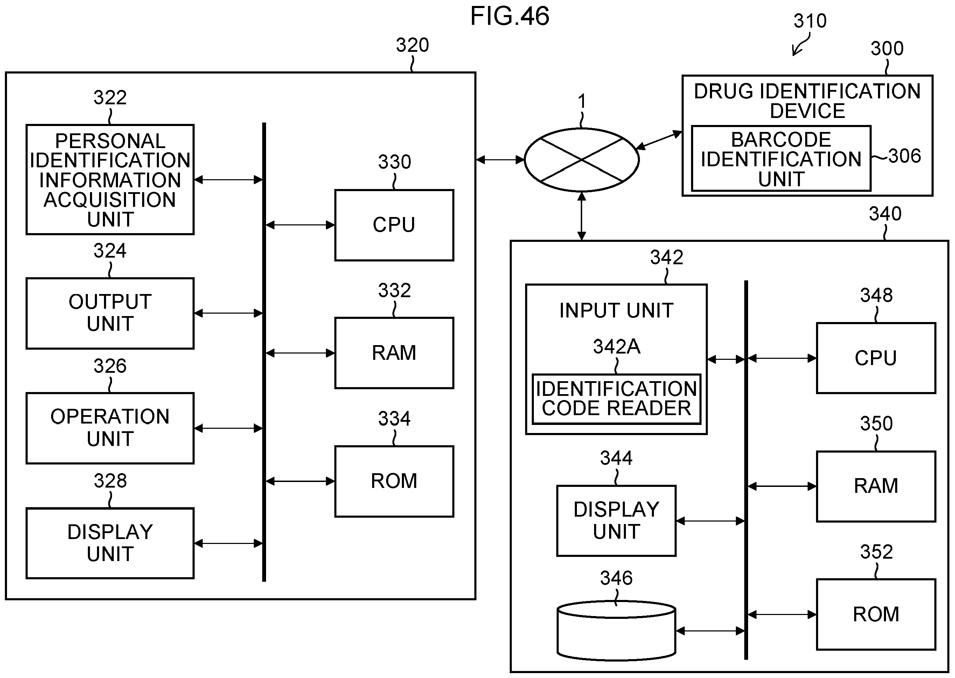

Figure 46 is a block diagram showing a configuration of a drug identification system 310 according to the fifth embodiment. As shown in Figure 46, the drug identification system 310 includes the drug identification device 300, a discrimination request sheet generation unit 320, and an electronic medical chart system 340.

The drug identification device 300 is implemented by, for example, the smartphone 10 as described in the fourth embodiment. The drug identification device 300 according to the fifth embodiment includes a barcode identification unit 306. The barcode identification unit 306 identifies a barcode included in an image. Here, the barcode identification unit 306 identifies the barcode included in the image captured by the in-camera 20 (see Figure 1) or the out-camera 22 (see Figure 2) of the smartphone 10.

The discrimination request sheet generation unit 320 is a device for generating a brought-in drug discrimination request sheet to request discrimination of a brought-in drug or drugs of a patient to a discriminator. As the discrimination request sheet generation unit 320, a personal computer or a workstation may be used.

As shown in Figure 46, the discrimination request sheet generation unit 320 includes a personal identification information acquisition unit 322, an output unit 324, an operation unit 326, a display unit 328, a CPU 330, a RAM 332, and a ROM 334.

The personal identification information acquisition unit 322 is an input interface for obtaining information for identifying an individual who is the owner of the brought-in drugs to be discriminated, and includes wired and wireless communication interfaces.

The output unit 324 is a device that outputs the brought-in drug discrimination request sheet. For example, the output unit 324 is a print device for printing the brought-in drug discrimination request sheet on a printed medium. The output unit 324 may be a print device provided separately from the discrimination request sheet generation unit 320, or may be a display device such as an electronic paper that displays the generated brought-in drug discrimination request sheet, or an organic electro luminescence (EL) film display.

The operation unit 326 is a user interface for the user to control the discrimination request sheet generation unit 320, and includes a keyboard and a pointing device.

The display unit 328 is an output interface that visually displays the state of the discrimination request sheet generation unit 320, and includes a display panel.

The hardware structure of the CPU 330, the RAM 332 and the ROM 334 is similar to the CPU 150, the RAM 152, and the ROM 154.

The electronic medical chart system 340 is also a system that collectively manages medical care information on patients as electronic information. As the electronic medical chart system 340, a personal computer or a workstation may be used.

As shown in Figure 46, the electronic medical chart system 340 includes an input unit 342, a display unit 344, a database 346, a CPU 348, a RAM 350, and a ROM 352.

The input unit 342 is a user interface for the user to control the electronic medical chart system 340, and includes a keyboard and a pointing device. The input unit 342 includes an identification code reader 342A that reads an identification code represented by a GS1 code or a two-dimensional code.

The display unit 344 is an output interface that visually displays the state of the electronic medical chart system 340, and includes a display panel.

The database 346 is a storage device that stores electronic medical charts of patients. For example, a hard disk drive is used as the database 346.

The hardware structure of the CPU 348, the RAM 350 and the ROM 352 is similar to the CPU 150, the RAM 152, and the ROM 154.

The drug identification device 300, the discrimination request sheet generation unit 320, and the electronic medical chart system 340 are connected via the network 1 so as to allow data communication between them. Here, the discrimination request sheet generation unit 320 may be included in the electronic medical chart system 340.

### [Drug Identification Method]

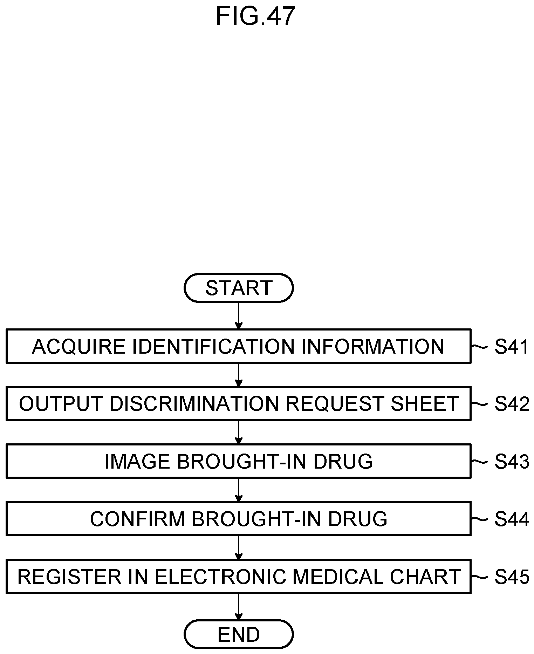

Figure 47 is a flowchart showing the steps of a drug identification method using the drug identification system 310. Described here is an example in which when a patient having brought-in drugs is admitted to a hospital, the information on the patient and information on the brought-in drugs are associated with each other and registered in an electronic medical chart.

In step S41, the personal identification information acquisition unit 322 of the discrimination request sheet generation unit 320 acquires information that identifies the patient. Here, a discrimination requester, who is a staff member of the hospital, inputs the information that identifies the patient into the personal identification information acquisition unit 322 by using the operation unit 326. The information that identifies the patient is, for example, a patient ID. The patient ID is, for example, an identifier assigned to each patient to identify the patient. In addition to the information to identify the patient, the patient ID may be imparted with annexed-information related to discrimination, such as drug administration time information and a serial number of the same individual, issued by the electronic medical chart system 340. The personal identification information acquisition unit 322 acquires patient information from the electronic medical chart system 340 based on the acquired patient ID. The patient information includes patient name, gender, date of birth, ward, room, department, and information on a physician in charge.

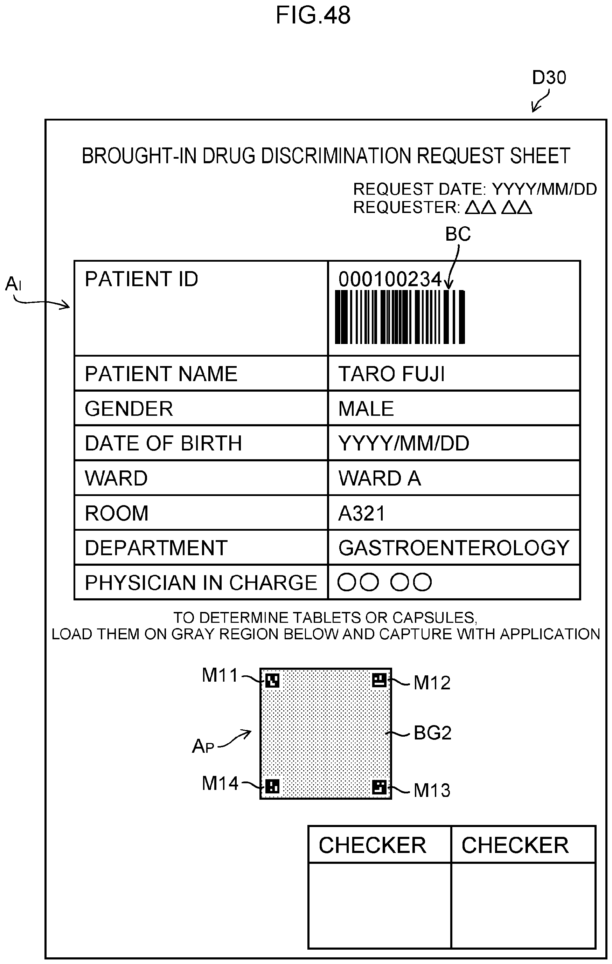

In step S42, the output unit 324 prints a brought-in drug discrimination request sheet (an example of an output object) on a printed medium by a print device. On the brought-in drug discrimination request sheet, a personal information display region and a loading region are arranged. The printing medium is, for example, A4-size (297 mm long x 210 mm wide) fine quality paper. Figure 48 shows an example of the brought-in drug discrimination request sheet D30 to be printed by the output unit 324. As shown in Figure 48, the brought-in drug discrimination request sheet D30 includes request date, requester, personal information display region A_{I}, loading region A_{P}, and stamp column (seal colum) for a checker.

The personal information display region A_{I} is a region for displaying the patient information acquired in step S41. Here, the patient information A_{I} includes patient ID, patient name, gender, date of birth, ward, room, department, and information on a physician in charge. The patient ID also includes a barcode BC indicating the patient ID.

The loading region A_{P} is a region where brought-in drugs are to be loaded when imaging the brought-in drugs, which are drugs to be identified, of the patient. The loading region A_{P} includes a background BG that is a reference-gray colored region, and four markers M11, M12, M13, and M14 arranged at four corners of the background BG. Instead of being printed by the print device, the loading region A_{P} may be a drug loading table that is separately generated and placed on the brought-in drug discrimination request sheet, the drug loading table being made of paper, fiber, rubber, glass or plastic.

The brought-in drug discrimination request sheet D30 printed in step S42 is handed over from the discrimination requester to the discriminator.

In step S43, the discriminator images (photographs) the barcode BC indicating the patient ID in the personal information display region A_{I} on the brought-in drug discrimination request sheet D30 with the in-camera 20 or the out-camera 22 of the drug identification device 300 (the smartphone 10). The barcode identification unit 306 identifies the barcode BC included in the captured image and specifies the patient ID.

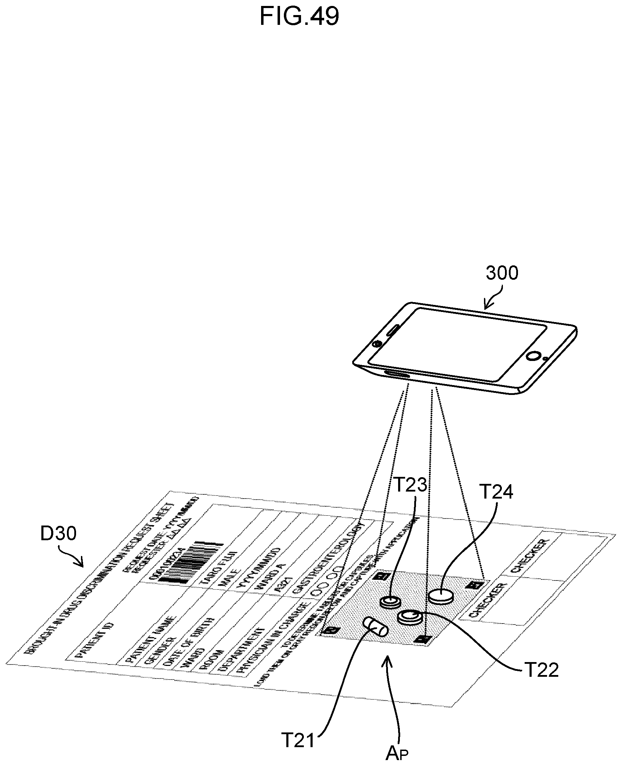

Further, in step S44, the discriminator loads the brought-in drugs of the patient in the loading region A_{P} on the brought-in drug discrimination request sheet D30, and images (photographs) the brought-in drugs with an unshown camera of the drug identification device 300. Figure 49 shows the case where one dose of the brought-in drugs of the patient is loaded onto the loading region A_{P} on the brought-in drug discrimination request sheet D30 and imaged. Here, four tablets T21, T22, T23, and T24 are loaded onto the loading region A_{P}. A unit of shooting (unit of photographing) needs only to correspond to a unit expected by the electronic medical chart system 340 as an input. The unit of shooting may be a single dose at each time of administration or a daily dose, or may be other units of shooting. In a case where there are a large amount of drugs for one dose, the drugs to be administrated at the same time may be divided and photographed (shoot) over two or more brought-in drug discrimination request sheets D30. Or, the drugs to be administrated at the same time are loaded two or more loading regions AP arranged on one brought-in drug discrimination request sheet D30, and are shoot (photographed) two or more times using the one brough-in drug discrimination request sheet D30.

In step S45, the drug identification device 300 confirms the drug types of four tablets T21, T22, T23 and T24 based on the images captured (photographed) in step S43 as in the case of the fourth embodiment.

Other than one-dose packaging drugs, as to drugs (such as PTP sheets) which are imparted with GTIN (a global trade identification number that is a GS1-standard barcode) so that the barcode can be read, the drugs may be identified by collation between the captured images and the master images. Or, as to drugs whose drug names are known, the drugs may be identified by text search.

In step S46, the drug identification device 300 inputs the patient ID identified in step S43 in association with the four tablets T21, T22, T23, and T24 that are identified in step S45 into the electronic medical chart system 340, as the discrimination result of the brought-in drugs. The electronic medical chart system 340 registers the patient ID and the drug types of the tablets T21, T22, T23, and T24 in association with each other.

The drug identification device 300 may compile the discrimination results in an electronic file format, and transmit them to the electronic medical chart system 340 via the network 1. The drug identification device 300 may display the text information on the drug name as a barcode in the vicinity of the read drug name. Then, the barcode may be read by the identification code reader 342A of the electronic medical chart system 340 so that the text information are input into the electronic medical chart system 340.

The discriminator then stamps (seals) on the brought-in drug discrimination request sheet D30, and returns the brought-in drug discrimination request sheet D30 and the discriminated tablets T21 to T24 to the discrimination requester, and ends the processing of this flowchart.

As described above, according to the fifth embodiment, it is possible to associate the patient and the drugs to be discriminated, and reliably discriminate the brought-in drugs. Note that in the drug identification method in the fifth embodiment, steps S41 and S42 constitute a production method of an output object.

### [Other Aspects of Reference Markers]

In order to enhance the detection accuracy of the reference markers, deep learning (an example of "machine learning") may be used. However, the ArUco markers with a logical structure have disadvantage that compatibility between the ArUco markers and deep learning is not very good.

In a case where deep learning is used to detect the region of the drug to be identified from the captured image, typically (center point coordinates, width, height), (center point coordinates, width, height, rotation angle), or mask images that paints an object shape or the like, are used as the format of teacher data for detection. Note that "height" here means the length in the direction perpendicular to "width".

Among them, (center point coordinates, width, height and rotation angle) may be most preferentially used due to the amount of information being relatively light and the compatibility with the detection of oval drugs. In the case where the rotation angle is not used, if oval drugs line up diagonally, drugs other than the drug to be detected may be included in a bounding box of the drug to be detected.

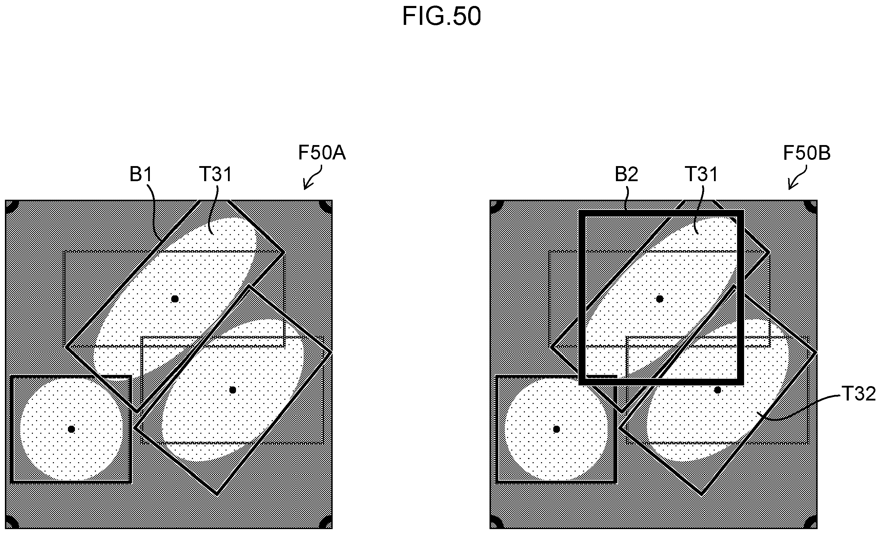

Figure 50 shows drugs and ground truth data of bounding boxes for the drugs, in a captured image. F50A shown in Figure 50 shows the ground truth data with (center point coordinates, width, height, rotation angle). The bounding box B1 shown in F50A is a rectangle that rotates in the captured image by a rotation angle corresponding to an arrangement direction of the oval tablet T31 to be detected and circumscribes the region of the tablet T31

F50B shown in Figure 50 shows the ground truth data with (center point coordinates, width, height). The bounding box B2 shown in F50B is a rectangle shape that circumscribes the region of the tablet T31, without rotation. In the example shown in F50B, the bounding box B2 includes the region of the tablet T31 as well as the region of the tablet T32.

Note that for circular drugs, their infinite symmetry makes it possible to define infinite number of ground truths of (center point coordinates, width, height, and rotation angle). However, when the teacher data is generated with the rotation angle set to be constantly zero degree, it is possible to make the deep leaning which uniquely performs inference.

The reference markers arranged on the loading surface of the drug loading table 74 may be detected using deep learning as in the case of drugs. In this case, it is necessary to generate teacher data for captured images (pre-standardization images) that are not standardized with respect to the imaging distance and the imaging viewpoint. Therefore, it is necessary to consider how the reference markers are shown in the pre-standardization image and how they are detected.

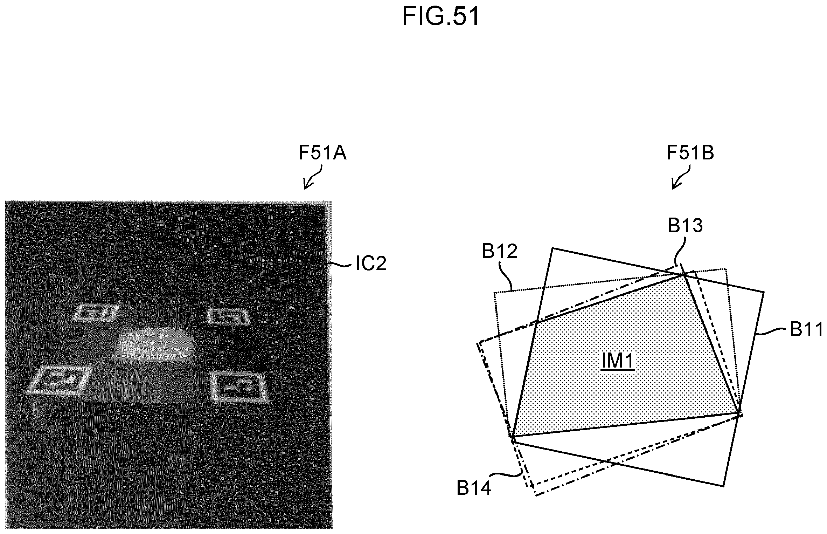

Figure 51 illustrates detection of reference markers when the reference markers are quadrangular. F51A shown in Figure 51 shows a captured image IC2 that is a pre-standardization image including quadrangular reference markers. F51B shown in Figure 51 shows a marker region IM1 that is a region of the reference markers acquired from the captured image IC2, and bounding boxes B11, B12, B13, and B14 corresponding to the marker region IM1.

As shown in F51A, in a case where the reference markers are quadrangular, there may be cases where none of the four sides of the quadrangle are parallel to each other in a marker region corresponding to one reference marker in the captured image IC2. When ground truth data for (center point coordinates, width, height, rotation angle) is defined for such a quadrilateral marker region IM1, many types of bounding boxes may be defined as shown in F51B. Such an arbitrariness has disadvantages of: possible occurrence of conflicts in learning; reduction in the overall detection accuracy; and difficulty in generating teacher data. Thus, that arbitrariness is undesirable.

On the other hand, in a case where circular reference markers are used, the circular shape is distorted into an oval shape with perspective in the pre-standardization image. However, the rotation angle may constantly be set to zero degree so that (center point coordinates, width, height, and rotation angle) may be given as teacher data.

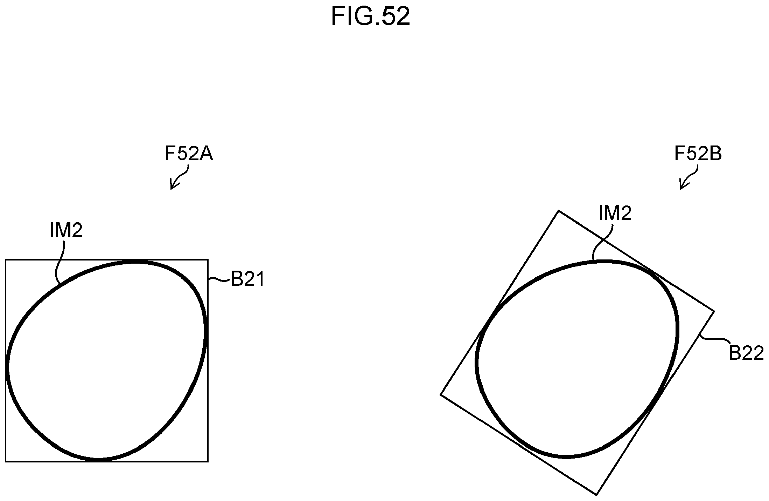

Figure 52 illustrates detection of reference markers in a case where the markers are circular. F52A shown in Figure 52 shows a marker region IM2 that is acquired from the pre-standardization image, and a bounding box B21 corresponding to the marker region IM2. The bounding box B21 has a rectangle shape that circumscribes the marker region IM2. The rotation angle of the bounding box B21 is zero degree. F52B shown in Figure 52 shows the marker region IM2, and a bounding box B22 corresponding to the marker region IM2. The bounding box B22 has a rectangle shape that circumscribes the marker region IM2. The bounding box B22 is rotated in the captured image by a rotation angle corresponding to the arrangement direction of the marker region IM2.

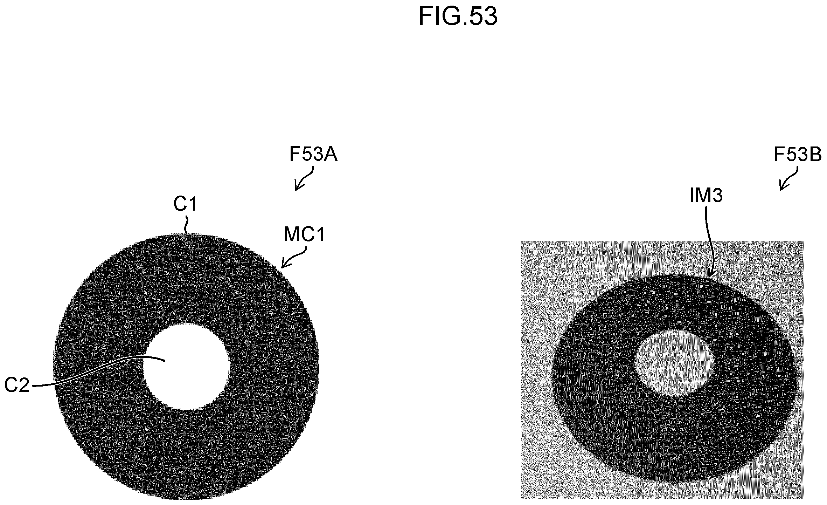

Figure 53 shows specific examples of the reference markers with a circular outer shape. F53A in Figure 53 shows a circular marker MC1. The circular marker MC1 includes: an outer true circle C1 that is relatively large in diameter; and an inner true circle C2 that is arranged concentrically with the true circle C1 and relatively smaller in diameter than the true circle C1. In other words, the true circles C1 and C2 are circles which are different in radius, and arranged so as to have the same center. In the circular marker MC1, the inside of true circle C2 is white color, and regions inside the true circle C1 and outside the true circle C2 are filled with black color.

The diameter of the true circle C1 is preferably 3 mm to 20 mm. The diameter of the true circle C2 is preferably 0.5 mm to 5 mm. A diameter ratio of the true circle C1 to the true circle C2 (diameter of true circle C1/ diameter of true circle C2) is preferably 2 to 10. For more accurate estimation of the center coordinates, an unshown black circle (for example, a true circle) relatively smaller in diameter than the true circle C2 may be concentrically arranged inside the true circle C2.

F53B in Figure 53 shows a marker region IM3 that is the region of the circular marker MC1 acquired from the pre-standardization image which includes the circular marker MC1.

There is a possibility that deviation may occur between the coordinates of the center point of a true object in the pre-standardization image and the coordinates of the center point estimated by machine learning. When the coordinates of the center point of the inner true circle C2 is provided as teacher data, as in the case of the circular marker MC1, it is possible to enable machine learning to accurately and easily estimate the center coordinates of true objects. In addition, with the effect of a relatively large true circle C1 existing on the outer side, the possibility of false detection due to dust or the like adhering to the circular marker MC1 can considerably be reduced.

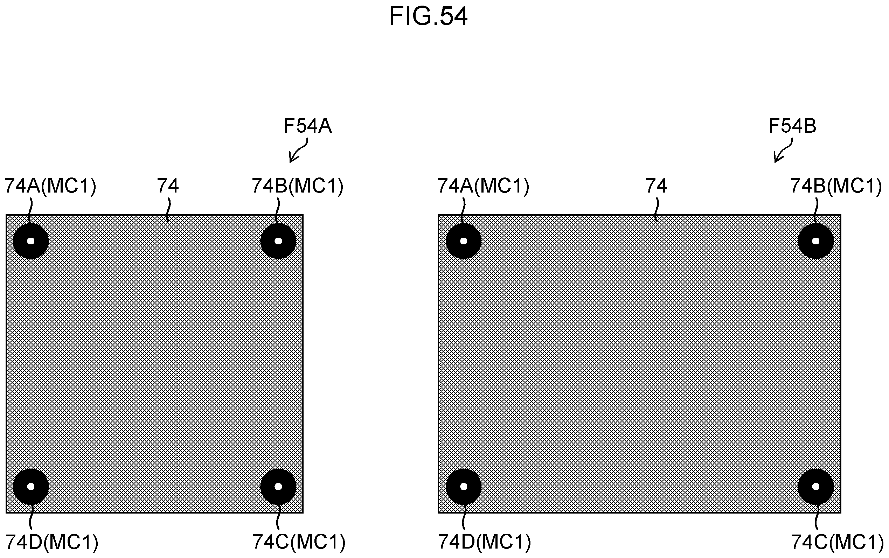

Figure 54 shows top views of the drug loading table using the circular markers MC1 each having a circular shape. As shown in Figure 54, circular markers MC1 in a circular shape are arranged at four corners of the drug loading table 74 as reference markers 74A, 74B, 74C, and 74D. F54A in Figure 54 shows an example where the centers of the reference markers 74A, 74B, 74C, and 74D constitute four vertexes of a square shape. F54B in Figure 54 shows an example where the centers of the reference markers 74A, 74B, 74C, and 74D constitute four vertexes of a rectangle shape. The reason for arranging four reference markers is that the coordinates of the four points are necessary for determining a perspective transformation matrix for standardization.

Here, while the four reference markers 74A, 74B, 74C and 74D are identical in size and color, they may be different in size and color. Note that when the size is different, the centers of adjacent reference markers 74A, 74B, 74C, and 74D may preferably be arranged to constitute four vertexes of a square or a rectangle. By using the reference markers different in size or color, it is easier to specify the imaging direction.

Moreover, at least four circular markers MC1 may be arranged, and five or more circular markers MC1 may also be arranged. If five or more circular markers MC1 are arranged, it is preferable that the centers of the four circular markers MC1 constitute four vertexes of a square or a rectangle, and the center of the additional circular marker MC1 is arranged on a side of the square or the rectangle. Arranging five or more reference markers makes it easier to specify the imaging direction. Moreover, arranging five or more reference markers has such advantages that even if the detection of any one of the reference markers fails, the probability of simultaneous detection of at least four points, which is necessary for calculating the perspective transformation matrix for standardization, can be increased. Therefore, the time and effort for re-take (re-shooting) can be reduced.

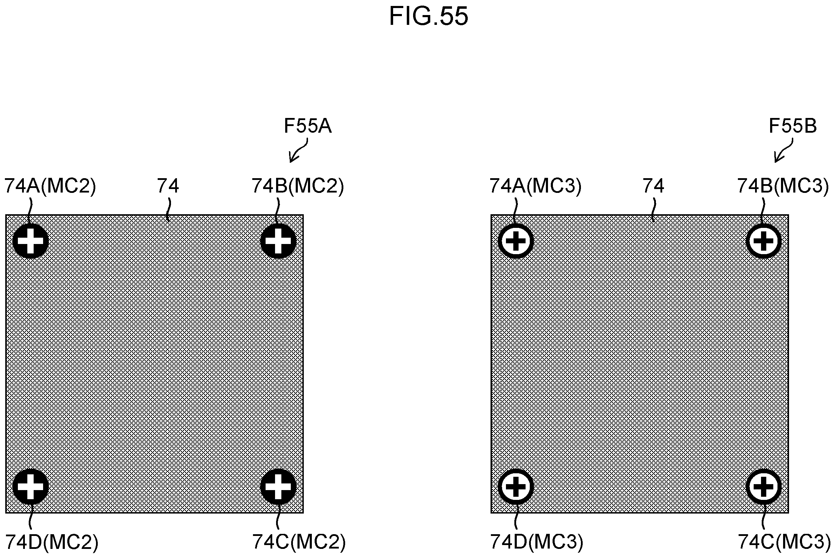

Figure 55 shows top views of the drug loading table 74 using circular markers according to a modification. F55A shown in Figure 55 shows an example where circular markers MC2 are used as the reference markers 74A, 74B, 74C, and 74D. The circular marker MC2 is a true circle which is filled with black color and has a cross-shaped (cruciform) figure inside the circle. The cross-shaped figure is formed with two white lines perpendicular to each other, and is arranged in such a manner that an intersection of the two straight lines is coincident (concentric) with the center of the true circle. The reference markers 74A, 74B, 74C, and 74D, which are the circular markers MC2, are arranged in such a manner that their respective centers constitute four vertexes of the square, and the straight lines of the cross-shaped figure in the circular markers MC2 are arranged parallel to the sides of the square. The reference markers 74A, 74B, 74C and 74D of the circular markers MC2, may be arranged in such a manner that their respective centers constitute four vertexes of a rectangle. The line thickness of the cross-shaped figure in the circular marker MC2 may be determined as appropriate.

Meanwhile, F55B in Figure 55 shows an example where circular markers MC3 are used as the reference markers 74A, 74B, 74C, and 74D. The circular marker MC3 includes two true circles of an inner true circle and an outer true circle that are arranged so as to have the same center and are different in radius. The inside of the inner true circle has white color, while regions of the inside of the outer true circle and the outside of the inner true circle are filled with black color. Furthermore, the circular marker MC3 has a cross-shaped figure formed with two black lines perpendicular to each other. The cross-shaped figure is arranged inside the inner true circle in such a manner that an intersection of the two straight lines is coincident with the center of the true circle. The reference markers 74A, 74B, 74C, and 74D, that are the circular markers MC3, are arranged in such a manner that their respective centers constitute four vertexes of the square, and the straight lines of the cross-shaped figure in the circular markers MC3 are arranged parallel to the sides of the square. The reference markers 74A, 74B, 74C and 74D, which are the circular markers MC3, may be arranged in such a manner that their respective centers constitute four vertexes of a rectangle. The line thickness of the cross-shaped figure in the circular markers MC3 may be determined as appropriate.

The circular markers MC2 and MC3 may enhance estimation accuracy of the center point coordinates. Moreover, since the circular markers MC2 and MC3 look different from drugs, it is easier to recognize the markers.

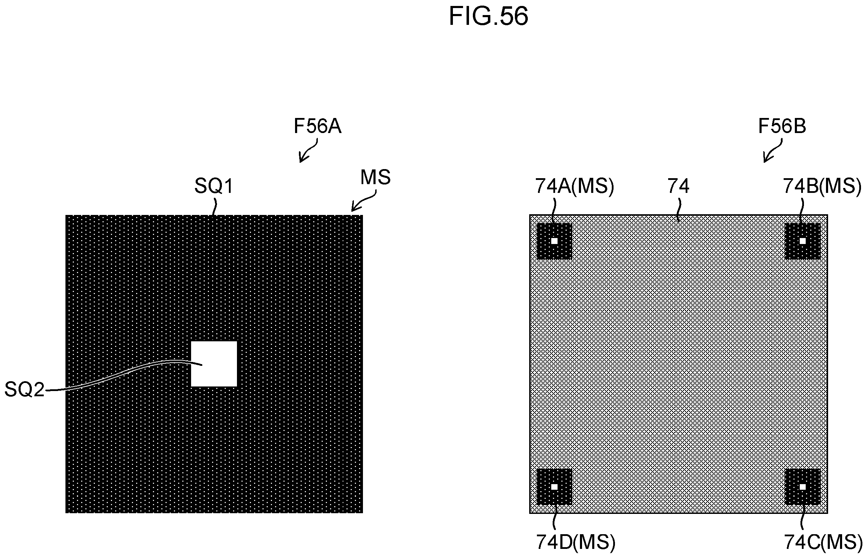

Figure 56 shows specific examples of the reference markers with a quadrangular outer shape. F56A in Figure 56 shows quadrangular markers MS. The quadrangular marker MS includes an outer square SQ1 having a relatively large length of one side, and an inner square SQ2 that is arranged concentrically with the square SQ1 and is relatively smaller in length of one side than the square SQ1. In other words, the squares SQ1 and SQ2 are quadrangles that are arranged on the same center (center of gravity) and are different in side length. In the quadrangular marker MS, the inside of the square SQ2 has white color, and regions inside the square SQ1 and outside the square SQ2 are filled with black color.

The length of one side of the square SQ1 is preferably 3 mm to 20 mm. The length of one side of the square SQ2 is preferably 0.5 mm to 5 mm. A ratio of the length of one side of the square SQ1 to the square SQ2 (length of one side of the square SQ1/ length of one side of the square SQ2) is preferably 2 to 10. For more accurate estimation of the center coordinates, an unshown black rectangle (for example a square) relatively smaller in one side length than the square SQ2 may be concentrically arranged inside the square SQ2.

F56B in Figure 56 shows a top view of the drug loading table 74 using the quadrangular markers MS having a quadrangular shape. As shown in F56B, the quadrangular markers MS having a quadrangular shape, are arranged at four corners of the drug loading table 74 as reference markers 74A, 74B, 74C, and 74D. In the example shown here, a line connecting the centers of the adjacent reference markers 74A, 74B, 74C and 74D constitute a square. However, the line connecting the centers of the adjacent reference markers 74A, 74B, 74C and 74D may constitute a rectangle.

On the drug loading table 74, the circular marker MC and the quadrangular marker MS may be mixedly present. Allowing the mixed presence of the circular marker MC and the quadrangular marker MS makes it easy to specify the imaging direction.

As described in the foregoing, in a case where the detection of the reference markers is based on deep learning, it is preferable to adopt circular markers or quadrangular markers with a simpler structure.

Furthermore, it is preferable to adopt the circular markers rather than the quadrangular markers. This is because when the detection of the reference markers is performed on the mobile terminal device such as a smartphone, following requests (a) to (c) are generated.
(a) It is desirable to use the same trained model for marker detection and drug detection due to capacity restrictions of the mobile terminal devices.
(b) For drug detection, due to the presence of oval tablets, it is desirable to also perform inference of angle. For the request (a), it is necessary to generate teacher data for reasonable rotation angle even also for the marker detection.
(c) In the case of the quadrangular markers, it is difficult to generate reasonable teacher data for the rotation angle in the pre-standardization image that is an input image at the time of marker detection, though it is possible in the case of the circular markers.

In addition, by making the circular markers concentric, the estimation accuracy of the center coordinates of the markers can be enhanced. While a simple circular maker is distorted in the pre-standardization image and error easily occurs in estimation of the center coordinates, the inner circle of the concentric circles has a narrower range so that the trained model can easily specify the center coordinates even in the distorted pre-standardization image. Furthermore, the outer circle of the concentric circles has advantages that: the trained model can be easily found due to a large structure of the outer circle; the outer circle is robust to noise and dust; and so on. The estimation accuracy of the center coordinates of the markers can be also enhanced by making the quadrangular markers concentric.

### <Sixth Embodiment>

In the sixth embodiment, in response to the request (a) stated above, one trained model executes two detection tasks: detection of the reference markers for a pre-standardization image and the detection of drugs in a standardized image.

### [Functional Configuration of Drug Identification Device]

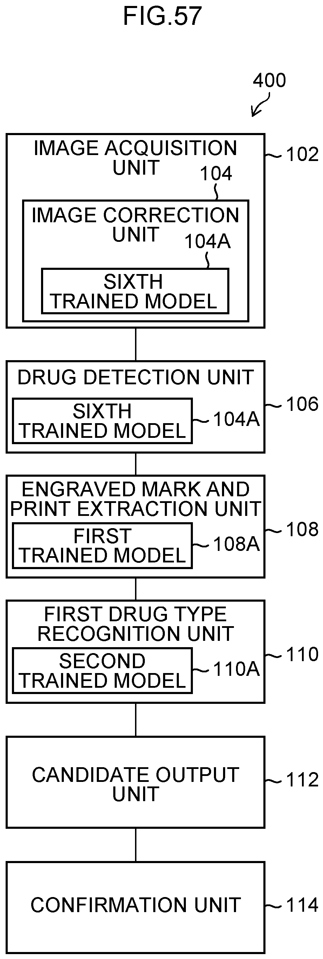

Figure 57 is a block diagram showing a functional configuration of a drug identification device 400 implemented by the smartphone 10 (see Figure 10). Here, component members in common with those of the Figure 5 are designated by identical reference numerals to omit a detailed description thereof. As shown in Figure 57, in the drug identification device 400, the image correction unit 104 and the drug detection unit 106 respectively include sixth trained models 104A. Note that the single sixth trained model 104A stored in the memory 34 (see Figure 3) may be shared by the image correction unit 104 and the drug detection unit 106, and it is not necessary for the image correction unit 104 and the drug detection unit 106 to include their respective sixth trained models 104A.

The sixth trained model 104A is a trained model that outputs, when receiving input of images (a pre-standardization image, a standardized image), rotational rectangular bounding boxes corresponding to the region of the object detected (center coordinates, width, height, rotation angle), class of an object, and probability of object-likeness. As in the case of the first trained model 108A, the CNN may be applied to the sixth trained model 104A. The sixth trained model 104A is not particularly limited as long as rotational rectangular bounding box (center coordinates, width, height, rotation angle) of an object, the class of an object, and the probability of object-likeliness may be estimated.

The class of an object include at least "markers" and "drugs". "Markers" and "drugs" may each be divided into smaller groups. For example, to distinguish the four positions of the markers, markers of different shapes and sizes may be classified as separate classes. Drugs may also be classified into "round tablets," "oval tablets," and "capsules," in view of subsequent drug identification processing.

### [Generation of Training Data Sets]

Description is given of generation of training data sets for learning of the sixth trained model 104A. For learning of the sixth trained model 104A, first training data sets as training data sets for the reference markers, and second training data sets as training data sets for the drugs, are required.

The first training data sets include images that satisfy following conditions.
- Pre-standardization images captured with various imaging distances and imaging viewpoints
- Only circular markers MC or only quadrangular markers MS are shown (no drugs are shown).
- The center point coordinates, width, height, and rotation angle of rotational rectangular bounding boxes indicating the regions of the circular markers MC and the quadrangular markers MS are given as ground truth data.
- Rotational rectangular bounding boxes with a constant rotation angle of zero degree are imparted as ground truth data to the circular markers MC and the quadrangular markers MS. The rotational rectangular bounding boxes in an oblong shape are given so as to circumscribe the circular markers MC or the quadrangular markers MS.
- The class of "marker" is imparted to the circular markers MC and the quadrangular markers MS.

The pre-standardization image in which drugs are not photographed is used because it is difficult to generate teacher data for the rotational rectangular bounding box for drugs in the distorted pre-standardization image.

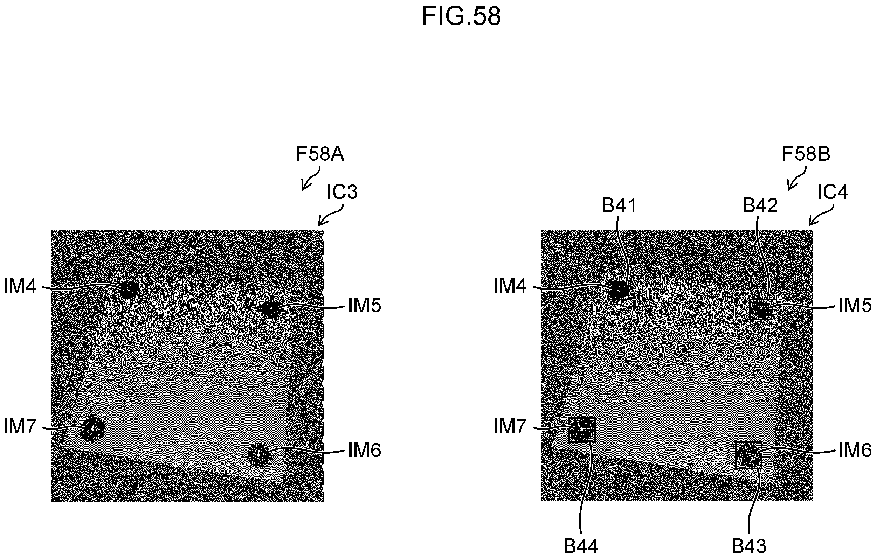

Figure 58 shows examples of images included in the first training data set. F58A in Figure 58 shows a captured image that is a pre-standardization image, and F58B in Figure 58 shows a captured image imparted with the ground truth data. As shown in F58A, the captured image IC3, which is a pre-standardization image, includes four marker regions IM4, IM5, IM6, and IM7. As shown in F58B, in the captured image IC4, which is the captured image IC3 after being imparted with the ground truth data, the four marker regions of IM4, IM5, IM6, and IM7 are imparted with rotational rectangular bounding boxes B41, B42, B43, and B44 that are the ground truth data, respectively.

Meanwhile, the second training data sets include images that satisfy following conditions.
- A standardized image in which the imaging distance and imaging viewpoint are standardized.
- No markers and only drugs are shown.
- The center point coordinates, width, height, and rotation angle of rotational rectangular bounding boxes for drugs are given as ground truth data.

For drugs, a class of "drugs" is given.

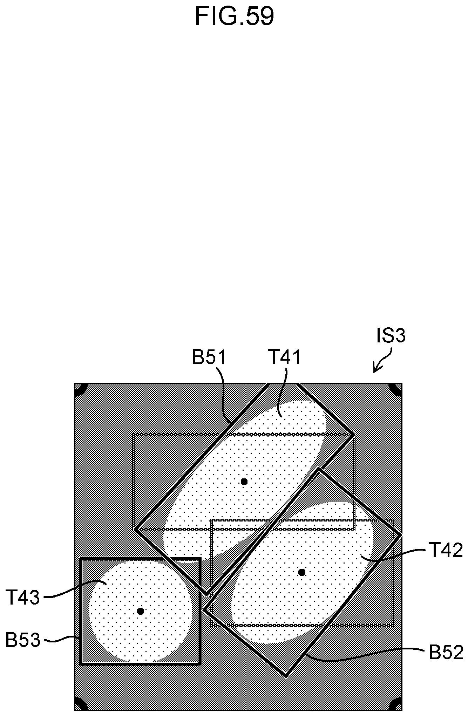

Figure 59 shows an example of an image included in the second training data set. As shown in Figure 59, a standardized image IS3 includes the regions of three tablets T41, T42, and T43. The regions of the three tablets T41 to T43 are imparted with rotational rectangular bounding boxes B51, B52, and B53, respectively, and the center point coordinates, width, height, and rotation angle of the rotational rectangular bounding boxes B51 to B53 are given as ground truth data.

### [Learning Method]

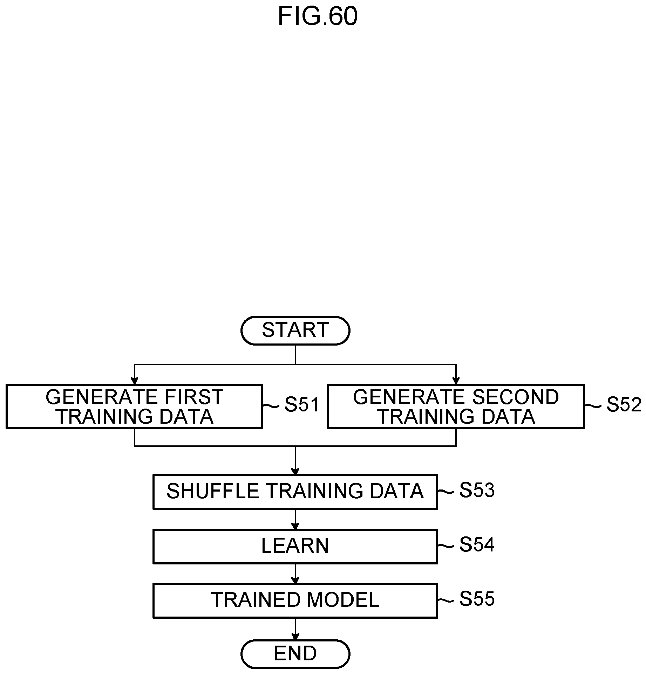

Figure 60 is a flowchart showing an example of a learning method (a learning phase) of the sixth trained model 104A. The learning method is implemented when the CPU 28 reads a drug identification program from the memory 34 and executes the program. The learning program may be provided via the wireless communication unit 30 or the external input/output unit 40 (for each unit, see Figure 3). The learning method may be implemented by a computer that is different from the drug identification device.

In step S51, the CPU 28 generates the first training data sets. In step S52, the CPU 28 generates the second training data sets. The processing of step S51 and step S52 may be done sequentially instead of in parallel.

In step S53, the first training data sets generated in step S51 and the second training data sets generated in step S52 are shuffled randomly. In step S54, with the first training data sets and the second training data sets, learning of the CNN (one example of "learning model") is performed in a shuffled order in step S53.

In step S55, the CNN learned in step S54 is output as the sixth trained model 104A.

### [Inference Method]

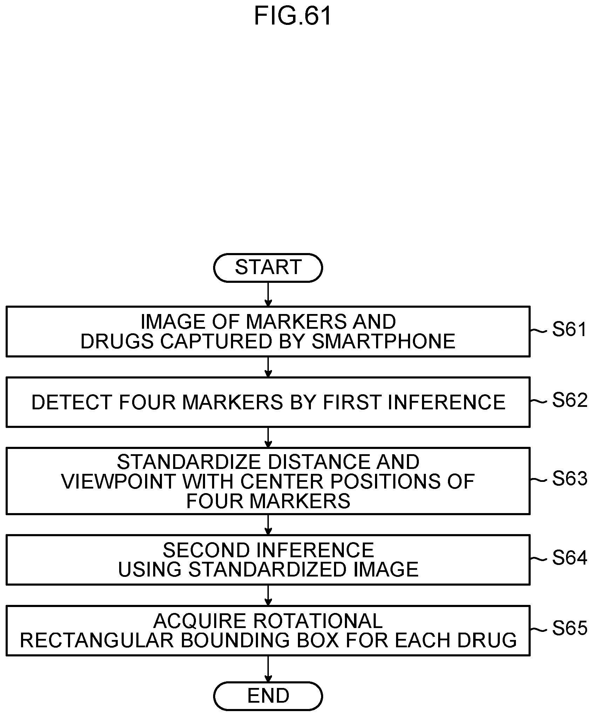

Figure 61 is a flowchart showing an example of an inference method (an inference phase) using the sixth trained model 104A. Each step of the inference method is included in the image acquisition step and the drug detection step in the drug identification method.

In step S61, the image correction unit 104 receives a pre-standardization image in which markers and drugs are photographed, and for which the imaging distance and imaging viewpoint are unknown (see Figure 57). The pre-standardization image is an image captured by the out-camera 22 of the smartphone 10, for example.

In step S62, the image correction unit 104 performs first inference on the pre-standardization image received in step S61, using the sixth trained model 104A in order to detect the markers. The image correction unit 104 extracts only the objects which is determined to have the class of "marker" in the inference result, and extracts four center point coordinates of the markers. Note that the objects inferred to have the class of "drugs" are discarded here.

In step S63, the image correction unit 104 performs perspective transformation based on the four center point coordinates extracted in step S62, and acquires a post-perspective transformation image (standardized image) for which the imaging distance and imaging viewpoint are standardized. In a case where an image is cut out based on the center points of the four markers, a quarter of the marker is cut out at each corner of the standardized image, and therefore, these portions may be masked if necessary.

In step S64, the drug detection unit 106 performs second inference on the standardized image that is acquired in step S63. In step S65, the drug detection unit 106 acquires rotational rectangular bounding boxes for the drugs photographed in the standardized image as a result of the inference in step S64.

The drug detection unit 106 uses the bounding boxes acquired in step S64 to cut out the regions of individual drugs from the standardized image. As a result, the drug identification device 400 can perform the above-mentioned engraved mark and print extraction step, the first drug type recognition step or the like.

The sixth embodiment has following advantages.

The sixth trained model can execute two tasks: detection of the markers in a pre-standardization image; and the detection of drugs in a standardized image. Therefore, the sixth trained model is useful when the trained model is operated on mobile devices that are susceptible to capacity limitations of the trained model.

Since the pre-standardization image is distorted, it is difficult to define the teacher data (especially the rotation angle) of the rotational rectangular bounding boxes for oval tablets and capsules in particular in the pre-standardization image. However, this problem does not occur in this method, since the teacher data in which drugs are photographed is required to be generated only for the standardized image, and only the markers are photographed in the pre-standardization image.

In this method, while the teacher data about the rotation angle of the rotational rectangular bounding boxes also needs to be imparted to the markers in the pre-standardization image, it is simply achieved by imparting the ground truth data with the rotation angle being constantly set to zero degree to the markers. Therefore, generation of the teacher data is simple and easy. Here, it is also possible to impart ground truth data with a rotation angle of zero degree even in the case of using quadrangular markers, the sixth trained model may be confused at the time of inferring because the quadrangular markers have straight parts. In this regard, it is preferable to use circular markers with a smooth structure.

Since the sixth trained model has learned to detect markers and drugs, it is possible to use the pre-standardization image in which both the drugs and the markers are photographed, even during the first inference.

According to the above description, the drug identification device and the trained models described below can be grasped.

### [Supplement 1]

A drug identification device, including:
an image acquisition unit configured to acquire a captured image generated by imaging at least one drug to be identified and a marker;
a marker detection unit configured to detect a region of the marker from the captured image by a sixth trained model configured to receive input of an image of an object and output at least a class of the object and a region of the object;
an image correction unit configured to standardize an imaging distance and an imaging viewpoint of the captured image based on the marker to acquire a standardized image; and
a drug detection unit configured to detect the region of the drug to be identified from the standardized image by the sixth trained model.

### [Supplement 2]

The drug identification device according to supplement 1, in which the image acquisition unit acquires the captured image generated by imaging at least four circular markers, or at least four quadrangular markers.

### [Supplement 3]

The drug identification device according to supplement 2, in which the at least four circular markers or the at least four quadrangular markers are arranged at positions where respective centers constitute four vertexes of a square or a rectangle.

### [Supplement 4]

The drug identification device according to supplement 2 or 3, in which the circular markers include a concentric circle, and the square markers include a concentric square.

### [Supplement 5]

The drug identification device according to any of supplements 1 to 4, in which
the drug to be identified is imparted with engraved mark and/or print, and
the drug identification device includes:
   a drug detection unit configured to detect a region of the drug to be identified from the standardized image;
   an engraved mark and print extraction unit configured to process at least the region of the drug to be identified in the standardized image to acquire an engraved mark and print extraction image that is an image of the engraved mark and/or print of the drug to be identified extracted from the region of the drug to be identified; and
   a first drug type recognition unit extracted receive input of the engraved mark and print extraction image and infer a drug type of the drug to be identified to acquire at least one candidate of the drug type of the drug to be identified.

### [Supplement 6]

A drug identification device, including at least one processor and at least one memory that stores a command for the at least one processor to execute, in which
the at least one processor is configured to:
acquire a captured image generated by imaging at least one drug to be identified and a marker;
detect a region of the marker from the captured image by a sixth trained model that receives input of an image of an object, and outputs at least a class of the object and a region of the object;
standardize an imaging distance and an imaging viewpoint of the captured image based on the marker to acquire a standardized image; and
detect the region of the drug to be identified from the standardized image by the sixth trained model.

### [Supplement 7]

A drug identification method, including:
an image acquisition step of acquiring a captured image generated by imaging at least one drug to be identified and a marker;
a marker detection step of detecting a region of the marker from the captured image by a sixth trained model that receives input of an image of an object and outputs at least a class of the object and a region of the object;
an image correction step of standardizing an imaging distance and an imaging viewpoint of the captured image based on the marker to acquire a standardized image; and
a drug detection step of detecting the region of the drug to be identified from the standardized image by the sixth trained model.

### [Supplement 8]

A trained model that is machine-learned based on
a first training data set including: a captured image of markers arranged on a loading surface and ground truth data indicating center coordinates, width, height, and rotation angle of the markers, and a class representing the markers, as a set, and
a second training data set including: a standardized image generated by standardizing an imaging distance and an imaging viewpoint of a captured image of a drug; and ground truth data indicating center coordinates, width, height, and rotation angle of the drug, and a class representing the drugs, as a set.

### [Supplement 9]

A generation method of a trained model, including:
a step of performing learning of a learning model based on a first training data set including a captured image of markers arranged on a loading surface and ground truth data indicating center coordinates, width, height, and rotation angle of the markers, and a class representing the markers, as a set; and
a step of performing learning of the learning model based on a second training data set including a standardized image generated by standardizing an imaging distance and an imaging viewpoint of a captured image of a drug and ground truth data indicating center coordinates, width, height, and rotation angle of the drug, and a class indicating the drugs, as a set.

### [Other Aspects of Drug Loading table]

The loading surface in the drug loading table of the imaging assistance device may include an indentation structure on which a drug (drugs) is to be loaded. The indentation structure includes indentations, grooves, recesses, and holes.

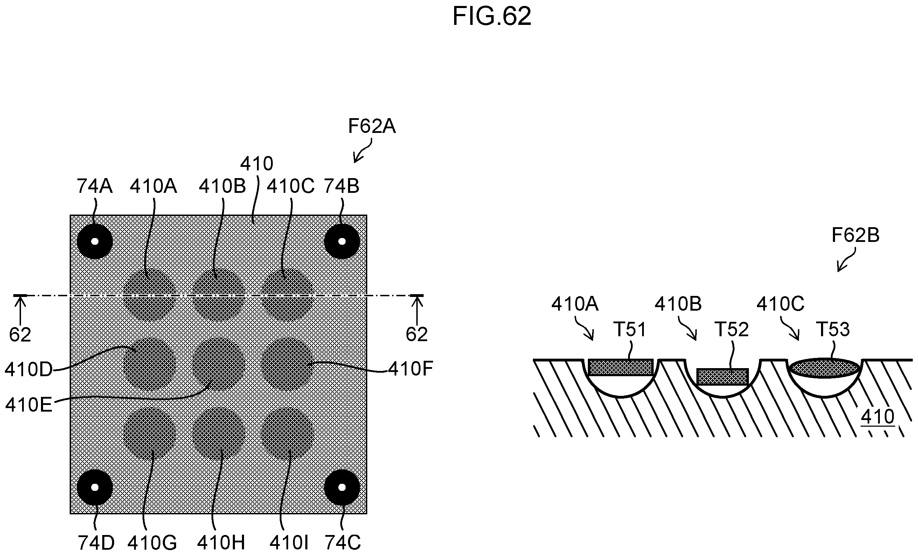

Figure 62 shows a drug loading table 410 that is used in place of the drug loading table 74 (see Figure 23) or in addition to the drug loading table 74. The drug loading table 410 includes an indentation structure. The drug loading table 410 is made of paper, composite resin, fiber, rubber, or glass. F62A in Figure 62 is a top view of the drug loading table 410. As shown in F62A, the loading surface of the drug loading table 410 has a gray color, and reference markers 74A, 74B, 74C and 74D are arranged at the four corners of the loading surface.

Furthermore, on the loading surface of the drug loading table 410, there are nine indentations 410A, 410B, 410C, 410D, 410E, 410F, 410G, 410H and 4101, each with three rows and three columns. The indentations 410A to 410I have circular shapes of the same size in a top view.

F62B in Figure 62 is a cross-sectional view of the drug loading table 410 along 62-62 line. As shown in F62B, the indentations 410A, 410B, and 410C have hemispherical bottom surfaces, each of which has the same depth. This is true for the indentations 410D to 410I.

F62B also shows tablets T51, T52, and T53 loaded on the indentations 410A, 410B and 410C, respectively. The tablets T51 and T52 are each circular in a top view and rectangular in a side view. The tablet T53 is circular in a top view and oval in a side view. In a top view, the tablet T51 and the tablet T53 have the same size, and the tablet T52 is relatively smaller than the tablet T51 and the tablet T53. As shown in F62B, the tablets T51 to T53 are trapped in the indentations 410A to 410C and thereby kept in a stationary state. As long as the tablets T51 to T53 is circular in a top view, the tablets T51 to T53 may be linear in a lateral direction and circular-arc in height direction, in a side view.

In this way, since the drug loading table 410 includes a hemispherical indentation structure on the loading surface, it is possible to prevent movement of circular drugs in a top view and to keep the drugs in a stationary state. Moreover, since the positions of the drugs at the time of imaging can be determined to be the positions of the indentation structure, the regions of the drugs are easily detected.

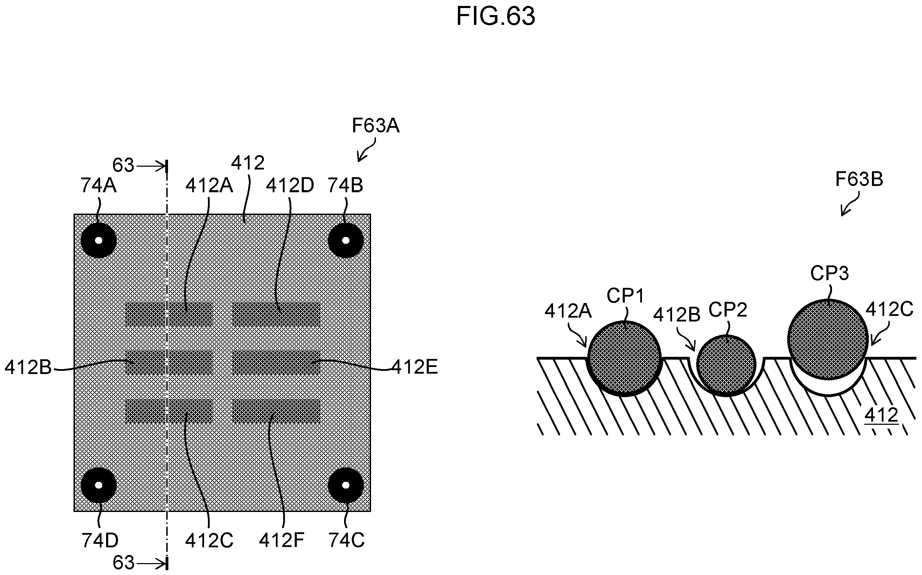

The drug loading table may include an indentation structure for drugs easy to roll, such as capsules. Figure 63 shows a drug loading table 412 having an indentation structure for capsules. Here, component members in common with those of the Figure 62 are designated by identical reference numerals to omit a detailed description thereof.

F63A in Figure 63 is a top view of the drug loading table 412. As shown in F63A, on the loading surface of the drug loading table 412, there are six indentations 412A, 412B, 412C, 412D, 412E and 412F formed on three rows and two columns. The indentations 410A to 412F have rectangular shapes of the same size in a top view.

F63B in Figure 63 is a cross-sectional view of the drug loading table 412 along 63-63 line. As shown in F63B, the indentations 412A, 412B, and 412C have semi-cylindrical bottom surfaces, each of which has the same depth. This is true for the indentations 412D to 412F. F63B also shows capsules CP1, CP2, and CP3 loaded on the indentations 412A, 412B and 412C, respectively. The capsules CP1 to CP3 have hemispherical columnar shapes whose both ends (both bottom surfaces) are hemispherical. However, the capsules CP1 to CP3 are different in diameter, from each other. As shown in F63B, the capsules CP1 to CP3 are trapped in the indentations 412A to 412C and are thereby kept in a stationary state.

In this way, since the drug loading table 412 includes a semi-cylindrical indentation structure on the loading surface, it is possible to prevent the columnar capsules from moving or rolling, and to keep them in a stationary state. Moreover, since the positions of the drugs at the time of imaging can be determined to be the positions of the indentation structure, the regions of the drugs are easily detected.

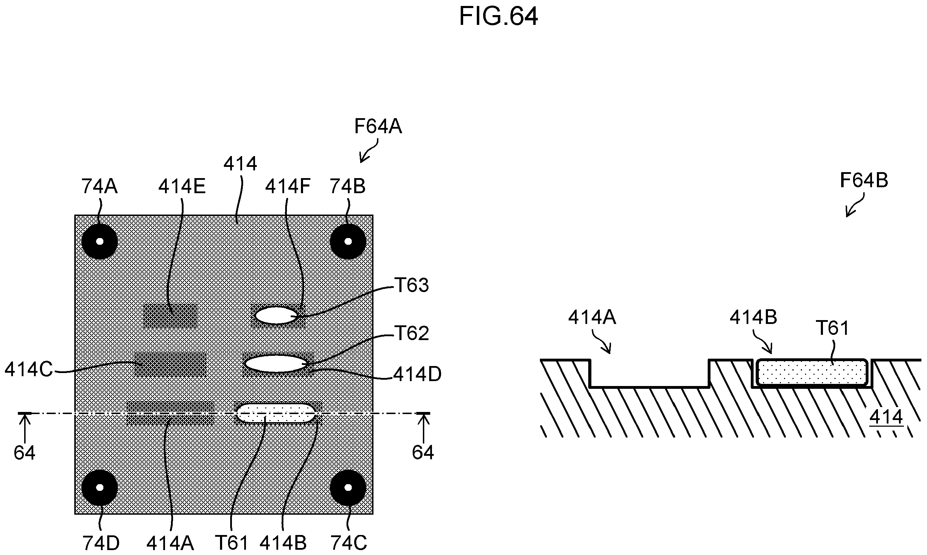

The drug loading table may also have an indentation structure for oval tablets. Figure 64 shows a drug loading table 414 having an indentation structure for oval tablets. Here, component members in common with those of the Figure 62 are designated by identical reference numerals to omit a detailed description thereof.

F64A in Figure 64 is a top view of the drug loading table 414. As shown in F64A, on the loading surface of the drug loading table 414, there are six indentations 414A, 414B, 414C, 414D, 414E and 414F formed on three rows and two columns. The indentations 414A to 414F are each oblong in a top view.

The indentations 414A and 414B are the same in size. The indentations 414C and 414D are the same in size and relatively smaller than the indentations 414A and 414B. The indentations 414E and 414F are in the same size and relatively smaller than the indentations 414C and 414D.

F64A also shows tablets T61, T62, and T63 loaded on the indentations 414B, 414D and 414F, respectively. As shown in F64A, the indentations 414B, 414D and 414F have sizes corresponding to the tablets T61, T62, And T63, respectively.

F64B in Figure 64 is a cross-sectional view of the drug loading table 414 along 64-64 line. As shown in F64B, the indentations 414A and 414B have flat bottom surfaces. As shown in F64B, the tablet T61 is trapped in the indentation 414B and kept in a stationary state. This is also true for the tablets T62 and T63.

In this way, since the drug loading table 414 has a rectangular parallelepiped indentation structure on the loading surface, it is possible to prevent oval tablets from moving and to keep them in a stationary state. Moreover, since the positions of the drugs at the time of imaging can be determined to be the positions of the indentation structure, the regions of the drugs are easily detected.

The shape, number, and arrangement of the indentation structures are not limited to the aspects shown in Figures 62 to 64, and may be combined, enlarged, or reduced as appropriate. The drug loading table 410, the drug loading table 412, or the drug loading table 414 may also be used as the loading region A_{P} (see Figure 48) in the brought-in drug discrimination request sheet D30.

### [Others]

The drug identification programs can also be stored in a non-transitory recording medium, such as a compact disk read only memory (CD-ROM) and be provided.

The technical scope of the present invention is not limited to the scope described in the above embodiments. The configurations and the like in the respective embodiments may appropriately be combined among the respective embodiments within the spirit of the present invention.

### Reference Signs List

- 10: smartphone

- 12: casing
- 14: touch panel display
- 16: speaker
- 18: microphone
- 20: in-camera
- 22: out-camera
- 24: light
- 26: switch
- 30: wireless communication unit
- 32: communication unit
- 34: memory
- 36: internal storage unit
- 38: external storage unit
- 40: external input/output unit
- 42: GPS receiver unit
- 44: power source unit
- 50: photographic lens
- 50F: focus lens
- 50Z: zoom lens
- 54: image element
- 58: A/D converter
- 60: lens drive unit
- 70: imaging assistance device
- 72: casing
- 72A: bottom surface plate
- 72B: side surface plate
- 72C: side surface plate
- 72D: side surface plate
- 72E: side surface plate
- 74: drug loading table
- 74A: reference marker
- 74B: reference marker
- 74C: reference marker
- 74D: reference marker
- 75: main light source
- 76: LED
- 78: auxiliary light source
- 80: imaging assistance device
- 82: casing
- 82A: bottom surface plate
- 82B: side surface plate
- 84: main light source
- 85: LED
- 86: auxiliary light source
- 100: drug identification device
- 102: image acquisition unit
- 104: image correction unit
- 104A: sixth trained model
- 106: drug detection unit
- 108: engraved mark and print extraction unit
- 108A: first trained model
- 110: first drug type recognition unit
- 110A: second trained model
- 112: candidate output unit
- 114: confirmation unit
- 120: drug identification device
- 122: second drug type recognition unit
- 122A: third trained model
- 123: drug-annexed information storage unit
- 124: drug-annexed information acquisition unit
- 126: master image storage unit
- 127: master image acquisition unit
- 128: template matching unit
- 130: drug identification device
- 140: image processing device
- 142: image input unit
- 144: database
- 146: operation unit
- 148: display unit
- 150: CPU
- 152: RAM
- 154: ROM
- 160: learning device
- 162: recognizer
- 162A: input layer
- 162B: intermediate layer
- 162C: output layer
- 164: loss value calculation unit
- 166: parameter control unit
- 170: learning device
- 172: recognizer
- 172A: input layer
- 172B: intermediate layer
- 172C: output layer
- 174: error calculation unit
- 176: parameter control unit
- 200: drug identification system
- 210: server
- 212: communicating unit
- 214: CPU
- 216: memory
- 300: drug identification device
- 302: search character information acquisition unit
- 304: search unit
- 306: barcode identification unit
- 310: drug identification system
- 320: discrimination request sheet generation unit
- 322: personal identification information acquisition unit
- 324: output unit
- 326: operation unit
- 328: display unit
- 330: CPU
- 332: RAM
- 340: electronic medical chart system
- 342: input unit
- 342A: identification code reader
- 344: display unit
- 346: database
- 348: CPU
- 350: RAM
- 400: drug identification device
- 410: drug loading table
- 410A: indentation
- 410B: indentation
- 410C: indentation
- 410D: indentation
- 410E: indentation
- 410F: indentation
- 410G: indentation
- 410H: indentation
- 410I: indentation
- 412: drug loading table
- 412A: indentation
- 412B: indentation
- 412C: indentation
- 412D: indentation
- 412E: indentation
- 412F: indentation
- 414: drug loading table
- 414A: indentation
- 414B: indentation
- 414C: indentation
- 414D: indentation
- 414E: indentation
- 414F: indentation
- A_{C}1: candidate drug display region
- A_{C}2: candidate drug display region
- A_{C}3: candidate drug display region
- A_{C}4: candidate drug display region
- A_{C}5: candidate drug display region
- A_{I}: personal information display region
- A_{P}: loading region
- A_{S}: selected drug display region
- B1: bounding box
- B2: bounding box
- B11: bounding box
- B12: bounding box
- B13: bounding box
- B14: bounding box
- B21: bounding box
- B22: bounding box
- B31: bounding box
- B41: bounding box
- B42: bounding box
- B43: bounding box
- B44: bounding box
- B51: rotational rectangle bounding box
- B52: rotational rectangle bounding box
- B53: rotational rectangle bounding box
- B_{B}: text box
- BC: barcode
- BG: background
- B_{S}1: shooting button
- B_{S}2: re-take button
- B_{S}3: search button
- B_{S}4: button
- B_{S}5: upper shift button
- B_{S}6: lower shift button
- B_{S}7: confirmation button
- B_{S}8: button
- B_{S}9: clear button
- B_{SL}: slider bar for correction
- C1: true circle
- C2: true circle
- CP1: capsule
- CP2: capsule
- CP3: capsule
- C_{T}1: candidate drug
- C_{T}2: candidate drug
- C_{T}3: candidate drug
- C_{T}4: candidate drug
- C_{T}5: candidate drug
- C_{T}11: candidate drug
- C_{T}12: candidate drug
- C_{T}13: candidate drug
- C_{T}14: candidate drug
- C_{T}15: candidate drug
- C_{T}21: candidate drug
- C_{T}22: candidate drug
- C_{T}23: candidate drug
- C_{T}24: candidate drug
- C_{T}25: candidate drug
- C_{T}26: candidate drug
- C_{T}27: candidate drug
- C_{T}28: candidate drug
- C_{T}29: candidate drug
- C_{T}30: candidate drug
- C_{T}31: candidate drug
- C_{T}32: candidate drug
- D1: screen display
- D2: screen display
- D3: screen display
- D4: screen display
- D5: screen display
- D11: screen display
- D12: screen display
- D13: screen display
- D14: screen display
- D21: screen display
- D22: screen display
- D23: screen display
- D24: screen display
- D25: screen display
- D30: brought-in drug discrimination request sheet
- D_{C}1: drug type
- D_{C}2: drug type
- D_{C}3: drug type
- F1: frame
- F2: frame
- F3: frame
- F11: frame
- F12: frame
- F13: frame
- I_{C}1: captured image
- IC2: captured image
- IC3: captured image
- IC4: captured image
- I_{E}: engraved mark and print extraction image
- I_{E}1: engraved mark and print extraction image
- I_{E}A: engraved mark and print extraction image
- I_{E}B: engraved mark and print extraction image
- I_{E}13: engraved mark and print extraction image
- I_{I}01: region image
- I_{I}02: region image
- I_{I}: region image
- I_{I}11: engraved mark and print extraction image
- I_{I}12: engraved mark and print extraction image
- I_{I}13: engraved mark and print extraction image
- I_{L}V: live view image
- I_{LV}2: live view image
- IM1: marker region
- IM2: marker region
- IM3: marker region
- IM41: marker region
- IM42: marker region
- IM43: marker region
- IM44: marker region
- IM51: marker region
- IM52: marker region
- IM53: marker region
- INF: drug-annexed information
- I_{R}: region image
- I_{R}13: region image
- I_{R}1: region image
- I_{R}A: region image
- I_{R}B: region image
- I_{S}: standardized image
- I_{S}1: standardized image
- I_{S}2: standardized image
- IS3: standardized image
- I_{W}11: composite extraction image
- I_{W}12: composite extraction image
- I_{W}13: composite extraction image
- IwA: composite extraction image
- IwB: composite extraction image
- KB: software keyboard
- M1: marker
- M2: marker
- M3: marker
- M4: marker
- M11: marker
- M12: marker
- M13: marker
- M14: marker
- MC1: circular marker
- MC2: circular marker
- MC3: circular marker
- MS: quadrangular marker
- M_{T}: tap position marker
- N11: number
- N12: number
- N13: number
- P1 to P6: each process of drug identification method
- R_{D}1: candidate
- R_{D}2: candidate
- R_{D}3: candidate
- S1 to S25, S31, S41 to S46: each step of drug identification method
- S51 to S55: each step of learning method of sixth trained model
- S61 to S65: each step of inference method of sixth trained model
- SL: slider
- SQ1: square
- SQ2: square
- ST1: GPS satellite
- ST2: GPS satellite
- T1...: tablet
- T2...: tablet
- T3...: tablet
- T11: tablet
- T12: tablet
- T13: tablet
- T21: tablet
- T22: tablet
- T23: tablet
- T24: tablet
- T31: tablet
- T32: tablet
- T41: tablet
- T42: tablet
- T43: tablet
- T51: tablet
- T52: tablet
- T53: tablet
- T61: tablet
- T62: tablet
- T63: tablet
- V1: vertex
- V2: vertex
- V3: vertex
- V4: vertex

## Claims

1. A drug identification device, comprising:
an image acquisition unit configured to acquire a captured image generated by imaging at least one drug to be identified that is imparted with engraved mark and/or print;
a drug detection unit configured to detect a region of the drug to be identified from the captured image;
an engraved mark and print extraction unit configured to process at least the region of the drug to be identified in the captured image, to acquire an engraved mark and print extraction image that is an image of the engraved mark and/or print of the drug to be identified extracted from the region of the drug to be identified; and
a first drug type recognition unit configured to receive input of the engraved mark and print extraction image, and infer a drug type of the drug to be identified to acquire at least one candidate of the drug type of the drug to be identified.

2. The drug identification device according to claim 1, wherein
the engraved mark and print extraction unit includes a first trained model configured to receive input of a first image of a drug that is imparted with an engraved mark and/or print and output a second image that is an image of the engraved mark and/or print of the drug extracted from the first image, and
the first drug type recognition unit includes a second trained model configured to receive input of the second image and output a drug type of a drug corresponding to the engraved mark and/or print.

3. The drug identification device according to claim 2, comprising
a second drug type recognition unit configured to receive input of the at least the region of the drug to be identified in the captured image and infer the drug type of the drug to be identified, wherein
the first drug type recognition unit integrates an inference result of the first drug type recognition unit with an inference result of the second drug type recognition unit to acquire the at least one candidate of the drug type of the drug to be identified, and
the second drug type recognition unit includes a third trained model configured to receive input of the first image and output the drug type of the drug.

4. The drug identification device according to any one of claims 1 to 3, comprising
a drug-annexed information acquisition unit configured to acquire drug-annexed information including at least one of shape, size, and color of a plurality of drugs, wherein
the first drug type recognition unit integrates an inference result of the first drug type recognition unit with the drug-annexed information to acquire the at least one candidate of the drug type of the drug to be identified.

5. The drug identification device according to any one of claims 1 to 4, wherein
the image acquisition unit acquires the captured image generated by imaging the drug to be identified and at least one marker, and includes an image correction unit configured to standardize an imaging distance and an imaging viewpoint of the captured image based on the marker to acquire a standardized image, and
the drug detection unit detects the region of the drug to be identified from the standardized image.

6. The drug identification device according to claim 5, wherein the image acquisition unit acquires the captured image generated by imaging a plurality of ArUco markers, a plurality of circular markers, or a plurality of quadrangular markers.

7. The drug identification device according to claim 6, wherein
the circular markers include a concentric circle, and the quadrangular markers include a concentric quadrangle.

8. The drug identification device according to any one of claims 5 to 7, wherein
the image acquisition unit acquires the captured image generated by imaging the drug to be identified and a reference gray color, and
the image correction unit performs color tone correction on the captured image based on the reference gray color.

9. A drug identification system, comprising a mobile terminal and a server which are configured to be able to communicate with each other, wherein
the mobile terminal includes an image acquisition unit configured to acquire a captured image generated by imaging at least one drug to be identified that is imparted with engraved mark and/or print,
the mobile terminal or the server includes a drug detection unit configured to detect a region of the drug to be identified from the captured image,
the server includes
an engraved mark and print extraction unit configured to process at least the region of the drug to be identified in the captured image to acquire an engraved mark and print extraction image that is an image of the engraved mark and/or print of the drug to be identified extracted from the region of the drug to be identified, and
a first drug type recognition unit configured to receive input of the engraved mark and print extraction image and infer a drug type of the drug to be identified, to acquire at least one candidate of the drug type of the drug to be identified, and
the mobile terminal further includes a display control unit configured to display the at least one candidate of the drug type of the drug to be identified.

10. The drug identification system according to claim 9, wherein
the engraved mark and print extraction unit includes a first trained model configured to receive input of a first image of a drug that is imparted with the engraved mark and/or print and output a second image that is an image of the engraved mark and/or print of the drug extracted from the first image, and
the first drug type recognition unit includes a second trained model configured to receive input of the second image and output the drug type of a drug corresponding to the engraved mark and/or print.

11. The drug identification system according to claim 10, wherein
the server includes a second drug type recognition unit configured to receive input of at least the region of the drug to be identified in the captured image and infer the drug type of the drug to be identified,
the first drug type recognition unit integrates an inference result of the first drug type recognition unit with an inference result of the second drug type recognition unit to acquire the at least one candidate of the drug type of the drug to be identified, and
the second drug type recognition unit includes a third trained model configured to receive input of the first image and output the drug type of the drug.

12. The drug identification system according to any one of claims 9 to 11, wherein
the server includes a drug-annexed information acquisition unit configured to acquire drug-annexed information including at least one of shape, size, and color of a plurality of drugs, and
the first drug type recognition unit integrates an inference result of the first drug type recognition unit with the drug-annexed information to acquire the at least one candidate of the drug type of the drug to be identified.

13. The drug identification system according to any one of claims 9 to 12, wherein
the mobile terminal includes a camera, and a display,
the image acquisition unit acquires the captured image generated by imaging the drug to be identified and at least one marker by the camera,
the mobile terminal or the server includes an image correction unit configured to standardize an imaging distance and an imaging viewpoint of the captured image based on the marker to acquire a standardized image, and
the drug detection unit detects the region of the drug to be identified from the standardized image.

14. The drug identification system according to claim 13, wherein
the drug to be identified is loaded on a loading surface having a gray color, and
the mobile terminal includes an exposure correction unit configured to perform exposure correction of the camera based on the gray color.

15. The drug identification device according to any one of claims 1 to 8, or the drug identification system according to any one of claims 9 to 14, wherein
the image acquisition unit acquires the captured image that is imaged with a standard imaging distance and imaging viewpoint.

16. The drug identification device according to any one of claims 1 to 8, or the drug identification system according to any one of claims 9 to 15, wherein
the image acquisition unit acquires a captured image including a plurality of drugs to be identified,
the drug detection unit detects respective regions of the plurality of drugs to be identified,
the engraved mark and print extraction unit acquires a plurality of engraved mark and print extraction images respectively corresponding to the plurality of drugs to be identified, and
the first drug type recognition unit acquires the candidates of the drug types respectively corresponding to the plurality of drugs to be identified.

17. The drug identification device according to any one of claims 1 to 8 or 16, or the drug identification system according to any one of claims 9 to 16, wherein
the first drug type recognition unit:
acquires a plurality of candidates of the drug type of the at least one drug to be identified;
acquires master images of the respective candidates of the drug type; and
performs template matching between the engraved mark and print extraction image, and the master images.

18. The drug identification device according to any one of claims 1 to 8 or 16 or 17, or the drug identification system according to any one of claims 9 to 17, comprising
a display control unit configured to display, on a display, at least one of: an image of at least the region of the drug to be identified in the captured image; the engraved mark and print extraction image; and an image of the drug to be identified with the engraved mark and/or print being emphasized, wherein
the display control unit further selectably displays, on the display, the at least one master image of the at least one candidate of the drug type of the at least one drug to be identified.

19. The drug identification device according to claim 18, or the drug identification system according to claim 18, wherein
the display control unit displays at least one of: the image of at least the region of the drug to be identified in the captured image; the engraved mark and print extraction image; and an image of the drug to be identified with the engraved mark and/or print being emphasized, and the at least one master image of the at least one candidate of the drug type of the at least one drug to be identified, in a state where directions of the engraved mark and/or print are arranged in an identical direction.

20. The drug identification device according to claim 18 or 19, or the drug identification system according to claim 18 or 19, wherein
the display control unit displays on the display a search window into which a character string can be input, and
the first drug type recognition unit specifies the drug type of the drug to be identified based on the character string input into the search window.

21. A drug loading table for use in capturing the captured image in the drug identification device according to any one of claims 1 to 8 or 16 to 20, or the drug identification system according to claims 9 to 20, the drug loading table comprising
a loading surface on which the at least one drug to be identified is loaded, wherein the loading surface has a gray color, and
a plurality of markers is arranged on the loading surface.

22. The drug loading table according to claim 21, wherein the plurality of markers are, respectively, circular markers or quadrangular markers.

23. The drug loading table according to claim 22, wherein
the circular markers include a concentric circle, and
the quadrangular markers include a concentric quadrangle.

24. The drug loading table according to any one of claims 21 to 23, wherein the loading surface includes an indentation structure provided for loading the at least one drug to be identified.

25. An illumination device for use in capturing the captured image in the drug identification device according to any one of claims 1 to 8 or 16 to 20, or the drug identification system according to claims 9 to 20, the illumination device comprising
a plurality of light sources configured to irradiate the at least one drug to be identified with illumination light from directions different from each other.

26. An imaging assistance device for use in capturing the captured image in the drug identification device according to any one of claims 1 to 8 or 16 to 20, or the drug identification system according to claims 9 to 20, the imaging assistance device comprising:
a drug loading table including a loading surface on which the at least one drug to be identified is loaded; and
an illumination device configured to irradiate the at least one drug to be identified that is loaded on the loading surface, with illumination light, wherein
the loading surface has a gray color,
a plurality of markers is arranged on the loading surface, and
the illumination device includes a plurality of light sources that emit illumination lights from directions different from each other, toward the drug to be identified.

27. A drug identification method, comprising:
an image acquisition step of acquiring a captured image generated by imaging at least one drug to be identified that is imparted with engraved mark and/or print;
a drug detection step of detecting a region of the drug to be identified from the captured image;
an engraved mark and print extraction step of processing at least the region of the drug to be identified in the captured image to acquire an engraved mark and print extraction image that is an image of the engraved mark and/or print of the drug to be identified extracted from the region of the drug to be identified; and
a first drug type recognition step of receiving input of the engraved mark and print extraction image, and inferring a drug type of the drug to be identified to acquire at least one candidate of the drug type of the drug to be identified.

28. The drug identification method according to claim 27, wherein
in the engraved mark and print extraction step, the engraved mark and print extraction image is acquired by using a first trained model that receives input of one drug that is imparted with the engraved mark and/or print and outputs a second image that is an extracted image of the engraved mark and/or print of the drug, and
in the first drug type recognition step, a drug type of the drug to be identified is inferred by using a second trained model that receives input of the second image and outputs a drug type of a drug corresponding to the engraved mark and/or print.

29. A program for causing a computer to execute the drug identification method according to claim 27 or 28.

30. A non-transitory, computer-readable recording medium that records thereon the program according to claim 29.

31. A trained model that is machine-learned using a training data set including a second image that is an extracted image of engraved mark and/or print of a drug that is imparted with the engraved mark and/or print, and a drug type of a drug corresponding to the engraved mark and/or print, as a set.

32. The trained model according to claim 31, wherein noise is added to the second image.

33. A learning device, comprising:
a training data collection unit configured to collect a retraining data set including an engraved mark and print extraction image that is an extracted image of engraved mark and/or print of a drug to be identified, and information on a correct drug type of the drug to be identified, as a set; and
a relearning unit configured to perform relearning of a second trained model by using the collected retraining data set, wherein
the second trained model receives input of a second image that is an extracted image of the engraved mark and/or print of the drug and outputs a drug type of a drug corresponding to the engraved mark and/or print.

34. A learning device, comprising:
a training data collection unit configured to collect a retraining data set including: a captured image generated by imaging at least one drug to be identified that is imparted with engraved mark and/or print; and information on a drug type of the drug to be identified that is acquired by the drug identification device according to claim 3 or the drug identification system according to claim 11, as a set; and
a relearning unit configured to perform relearning of the third trained model by using the collected retraining data set.

35. A learning device, comprising:
a training data collection unit configured to collect a retraining data set including: a captured image generated by imaging at least one drug to be identified that is imparted with engraved mark and/or print; and information on a drug type of the drug to be identified that is acquired by the drug identification device according to any one of claims 1 to 8, or 16 to 20, or the drug identification system according to any one of claims 9 to 20, as a set; and
a learning unit configured to perform learning of a fourth trained model by using the collected retraining data set, wherein
the fourth trained model receives input of a first image of a drug that is imparted with engraved mark and/or print and outputs a drug type of the drug.

36. A drug identification device, comprising:
an image acquisition unit configured to acquire a captured image generated by imaging at least one drug to be identified that is imparted with engraved mark and/or print;
a drug detection unit configured to detect a region of the drug to be identified from the captured image;
an engraved mark and print extraction unit configured to process at least the region of the drug to be identified in the captured image to acquire an engraved mark and print extraction image that is an image of the engraved mark and/or print of the drug to be identified extracted from the region of the drug to be identified; and
a display control unit configured to display, on a display, at least one of: an image of at least the region of the drug to be identified in the captured image; the engraved mark and print extraction image; and an image of the drug to be identified with the engraved mark and/or print being emphasized, in a state where a direction of the engraved mark and/or print is made upright.

37. The drug identification device according to claim 36, wherein the engraved mark and print extraction unit includes a first trained model configured to receive input of a first image of the drug that is imparted with the engraved mark and/or print, and output a second image that is an image of the engraved mark and/or print of the drug extracted from the first image.

38. The drug identification device according to claim 36 or 37, wherein
the display control unit acquires a master image of the drug to be identified in which the direction of the engraved mark and/or print is made upright, and
collates a rotation direction by template matching between at least one of: the image of at least the region of the drug to be identified in the captured image; the engraved mark and print extraction image; and an image of the drug to be identified with the engraved mark and/or print being emphasized, and the master image.

39. The drug identification device according to any one of claims 36 to 38, wherein
the display control unit include a fifth trained model configured to receive input of a first image of a drug that is imparted with the engraved mark and/or print, and output a third image in which a direction of the engraved mark and/or print is made upright.

40. The drug identification device according to any one of claims 36 to 39, wherein
the display control unit displays on the display a search window into which character information can be input, and
the drug identification device comprises:
a search unit configured to retrieve at least one candidate of a drug type of the drug to be identified based on the character information input into the search window; and
a first drug type recognition unit configured to receive input of the engraved mark and print extraction image, and infer the drug type of the drug to be identified to acquire the at least one candidate of the drug type of the drug to be identified, and
the display control unit automatically inputs into the search window, the character information indicating a most promising candidate of the drug type of the drug to be identified that is acquired in the first drug type recognition unit.

41. The drug identification device according to claim 40, wherein the display control unit displays the at least one candidate of the drug type of the drug to be identified that is retrieved by the search unit.

42. A drug identification system, comprising a mobile terminal and a server which are configured to be able to communicate with each other, wherein
the mobile terminal includes an image acquisition unit configured to acquire a captured image generated by imaging at least one drug to be identified that is imparted with engraved mark and/or print,
the mobile terminal or the server includes a drug detection unit configured to detect a region of the drug to be identified from the captured image,
the server includes an engraved mark and print extraction unit configured to processes at least the region of the drug to be identified in the captured image to acquire an engraved mark and print extraction image that is an image of the engraved mark and/or print of the drug to be identified extracted from the region of the drug to be identified, and
the mobile terminal further includes a display control unit configured to display, on a display, at least one of: an image of at least the region of the drug to be identified in the captured image; the engraved mark and print extraction image; and an image of the drug to be identified with the engraved mark and/or print being emphasized, in a state where a direction of the engraved mark and/or print is made upright.

43. A drug identification method, comprising:
an image acquisition step of acquiring a captured image generated by imaging at least one drug to be identified that is imparted with engraved mark and/or print;
a drug detection step of detecting a region of the drug to be identified from the captured image;
an engraved mark and print extraction step of processing at least the region of the drug to be identified in the captured image to acquire an engraved mark and print extraction image that is an image of the engraved mark and/or print of the drug to be identified extracted from the region of the drug to be identified; and
a display control step of displaying, on a display, at least one of: an image of at least the region of the drug to be identified in the captured image; the engraved mark and print extraction image; and an image of the drug to be identified with the engraved mark and/or print being emphasized, in a state where a direction of the engraved mark and/or print is made upright.

44. An output object for use in capturing the captured image in the drug identification device according to any one of claims 1 to 8 or 16 to 20 or 36 to 41, or the drug identification system according to any one of claims 9 to 20 or 42, the output object comprising:
a personal information display region on which information that identifies an individual is displayed; and
a loading region on which at least one drug to be identified of the individual is loaded.

45. The output object according to claim 44, wherein the information that identifies the individual includes a barcode.

46. The output object according to claim 44 or 45, wherein the loading region has a gray color, and
a plurality of markers is arranged in the loading region.

47. A production method of the output object according to any one of claims 44 to 46, comprising:
a step of acquiring the information that identifies the individual; and
a step of arranging and printing the personal information display region and the loading region on a printing medium.
